# EUROPEAN PATENT SPECIFICATION

(11) **EP 1 409 529 B1**
(45) Date of publication and mention of the grant of the patent: **29.08.2007**
(21) Application number: 01990010.9
(22) Date of filing: 06.11.2001
(51) Int. Cl.: C07K 14/33

(54) **RECOMBINANT LIGHT CHAINS OF BOTULINUM NEUROTOXINS AND LIGHT CHAIN FUSION PROTEINS FOR USE IN RESEARCH AND CLINICAL THERAPY**
REKOMBINANTE LEICHTE KETTE DES BOTOLINUM NEUROTOXINS AND FUSIONSPROTEINE DAVON ZUR VERWENDUNG IN FORSCHUNG UND KLINISCHER THERAPIE
CHAINES LEGERES RECOMBINEES DE NEUROTOXINES DE BOTULINUM ET PROTEINES DE FUSION A CHAINES LEGERES A UTILISER DANS LA RECHERCHE ET LA THERAPIE CLINIQUE

(30) Priority: 06.11.2000 US 246774 P; 20.07.2001 US 910186; 09.08.2001 US 311966 P
(43) Date of publication of application: 21.04.2004
(73) Proprietor: United States Army Medical Research and Material Command, Fort Detrick, MD 21702-5102 (US)
(72) Inventor: SMITH, Leonard, A., Clarksburg, MD 20871 (US); JENSEN, Melody, Frederick, MD 21702 (US)
(74) Representative: Lucas, Brian Ronald
(86) International application number: PCT/US2001/047230
(87) International publication number: WO 2002/036758

(56) References cited:
- WO-A-95/32738
- WO-A-98/07864
- WO-A-98/08540
- WO-A-99/20306
- US-A- 5 766 605
- ZHOU L ET AL: "EXPRESSION AND PURIFICATION OF THE LIGHT CHAIN OF BOTULINUM NEUROTOXIN A: A SINGLE MUTATION ABOLISHES ITS CLEAVAGE OF SNAP-25 AND NEUROTOXICITY AFTER RECONSTRUCTION WITH THE HEAVY CHAIN" BIOCHEMISTRY, AMERICAN CHEMICAL SOCIETY. EASTON, PA, US, vol. 34, no. 46, 1 November 1995 (1995-11-01), pages 15175-15181, XP002015939 ISSN: 0006-2960
- CLAYTON M A ET AL: "Protective vaccination with a recombinant fragment of Clostridium botulinum neurotoxin serotype A expressed from a synthetic gene in E. coli" INFECTION AND IMMUNITY, AMERICAN SOCIETY FOR MICROBIOLOGY. WASHINGTON, US, vol. 63, no. 7, July 1995 (1995-07), pages 2738-2742, XP002110803 ISSN: 0019-9567
- LI LI ET ATL.,: "high-level expression, purification, and characterization of recombinant type A botulinum neurotoxin light chain" PROTEIN EXPRESSION AND PURIFICATION, vol. 17, - 1999 pages 339-344, XP002222982
- ASHRAF AHMED S. ET AL.,: "light chain of botulinum a neurotoxin expressed as an inclusion body from a synthetic gene is catalytically and functionally active" J. PROTEIN CHEMISTRY, vol. 19, no. 6, - August 2000 (2000-08) pages 475-487, XP009002086 cited in the application
- AGUADO F. ET AL., : "regulated secretion is impaired in AtT-20 endocrine cells stably transfected with botulinum neurotoxin type a light chain" J. BIOLOGICAL CHEMISTRY, vol. 272, no. 41, - 10 October 1997 (1997-10-10) pages 26005-26008, XP002231890
- YAMASAKI SHINJI ET AL: "Synaptobrevin/vesicle-associated membrane protein (VAMP) of Aplysia californica: Structure and proteolysis by tetanus toxin and botulinal neurotoxins type D and F" PROCEEDINGS OF THE NATIONAL ACADEMY OF SCIENCES OF USA, NATIONAL ACADEMY OF SCIENCE. WASHINGTON, US, vol. 91, no. 11, 1994, pages 4688-4692, XP002203164 ISSN: 0027-8424
- SYUTO B ET AL: "SEPARATION AND CHARACTERIZATION OF HEAVY AND LIGHT CHAINS FROM CLOSTRIDIUM BOTULINUM TYPE C TOXIN AND THEIR RECONSTITUTION" JOURNAL OF BIOLOGICAL CHEMISTRY. (MICROFILMS), AMERICAN SOCIETY OF BIOLOGICAL CHEMISTS, BALTIMORE, MD, US, vol. 256, no. 8, 1981, pages 3712-3717, XP009002064

### SEQUENCE LISTING

<110> Smith, Leonard
<120> RECOMBINANT LIGHT CHAINS OF BOTULINUM NEUROTOXINS AND LIGHT CHAIN FUSION PROTEINS FOR USE IN RESEARCH AND CLINICAL THERAPY
<130> A33778 067252.0106
<150> 60/246,744
   <151> 2000-11-06
<150> 60/311,966
   <151> 2001-08-09
<160> 47
<170> FastSEQ for Windows Version 4.0
<210> 1
   <211> 16
   <212> PRT
   <213> Clostridium botulinum
<220>
   <221> PEPTIDE
   <222> (0)...(0)
   <223> N-terminal residues of mature, wild-type botulinum neurotoxin
<400> 1
   Pro Phe Val Asn Lys Gln Phe Asn Tyr Lys Asp Pro Val Asn Gly Val
    1               5                   10                  15
<210> 2
   <211> 17
   <212> PRT
   <213> Human
<220>
   <221> PEPTIDE
   <222> (0)...(0)
   <223> Residues 187-203 of SNAP-25
<400> 2
   Ser Asn Lys Thr Arg Ile Asp Glu Ala Asn Gln Arg Ala Thr Lys Met
    1               5                   10                  15
   Leu
<210> 3
   <211> 14
   <212> PRT
   <213> Artificial Sequence
<220>
   <223> Synthetic peptide; control for phosphorylation experiments
<400> 3
   Lys Val Glu Lys Ile Gly Glu Gly Thr Gly Val Val Tyr Lys
    1               5                   10
<210> 4
   <211> 1403
   <212> DNA
   <213> Artificial Sequence
<220>
   <223> Synthetic botulinum neurotoxin light chain of serotype A based on wild-type Clostridium botulinum sequence
<400> 4
   gaattcccat ggttcagttc gttaacaaac agttcaacta caaagacccg gttaacggtg 60
   ttgacatcgc ttacatcaaa atcccgaacg ttggtcagat gcagccggtt aaagcattca 120
   aaatccacaa caaaatctgg gttatcccgg aacgtgacac tttcactaac ccggaagaag 180
   gtgacctgaa cccgccgccg gaagctaaac aggttccggt ttcttactac gactctactt 240
   acctgtctac tgacaacgaa aaggacaact acctgaaagg tgttactaaa ctgtttgaac 300
   gtatctactc tactgacctg ggtcgcatgc tgctcacttc tatcgttcgt ggtatcccgt 360
   tctggggtgg ttctactatc gacactgaac tgaaagttat cgacactaac tgcatcaacg 420
   ttatccagcc ggacggttct taccgttctg aagaactgaa cctggttatc atcggtccgt 480
   ctgctgacat catccagttt gaatgcaaat ctttcggtca cgaagttctg aacctgactc 540
   gtaacggtta cggttctact cagtacatcc gtttctctcc ggacttcact ttcggtttcg 600
   aagaatctct ggaagttgac actaacccgc tgctgggtgc tggtaaattc gctactgacc 660
   cggctgttac tctggctcac gaactgatcc acgctggtca ccgtctgtac ggtatcgcta 720
   tcaacccgaa ccgtgttttc aaagttaaca ctaacgctta ctacgaaatg tctggtctgg 780
   aagtttcttt tgaagaactg cgtactttcg gtggtcacga cgctaaattc atcgactctc 840
   tgcaggaaaa cgagttccgt ctgtactact acaacaaatt caaagacatc gcttctactc 900
   tgaacaaagc taaatctatc gttggtacca ctgcttctct gcagtacatg aagaacgttt 960
   tcaaagaaaa gtacctgctg tctgaagaca cttctggtaa attctctgtt gacaaactga 1020
   aattcgacaa actgtacaaa atgctgactg aaatctacac tgaagacaac ttcgttaaat 1080
   tcttcaaagt tctgaaccgt aaaacttacc tgaacttcga caaagctgtt ttcaaaatca 1140
   acatcgttcc gaaagttaac tacactatct acgacggttt caacctgcgt aacactaacc 1200
   tggctgctaa cttcaacggt cagaacactg aaatcaacaa catgaacttc actaaactga 1260
   agaacttcac tggtctgttt gagttctaca aactgctgtg cgttcgtggt atcatcactt 1320
   ctaaaactaa atctctggac aaaggttaca acaaactggt tccgcgtggt tctcatcatc 1380
   atcatcatca ttaatgagaa tcc                                         1403
<210> 5
   <211> 461
   <212> PRT
   <213> Artificial Sequence
<220>
   <223> Synthetic botulinum neurotoxin light chain of serotype A based on wild-type Clostridium botulinum sequence
<400> 5
   Met Val Gln Phe Val Asn Lys Gln Phe Asn Tyr Lys Asp Pro Val Asn
    1               5                  10                  15
   Gly Val Asp Ile Ala Tyr Ile Lys Ile Pro Asn Val Gly Gln Met Gln
               20                  25                  30
   Pro Val Lys Ala Phe Lys Ile His Asn Lys Ile Trp Val Ile Pro Glu
           35                  40                  45
   Arg Asp Thr Phe Thr Asn Pro Glu Glu Gly Asp Leu Asn Pro Pro Pro
       50                  55                  60
   Glu Ala Lys Gln Val Pro Val Ser Tyr Tyr Asp Ser Thr Tyr Leu Ser
   65                  70                  75                  80
   Thr Asp Asn Glu Lys Asp Asn Tyr Leu Lys Gly Val Thr Lys Leu Phe
                   85                  90                  95
   Glu Arg Ile Tyr Ser Thr Asp Leu Gly Arg Met Leu Leu Thr Ser Ile
               100                 105                 110
   Val Arg Gly Ile Pro Phe Trp Gly Gly Ser Thr Ile Asp Thr Glu Leu
           115                 120                 125
   Lys Val Ile Asp Thr Asn Cys Ile Asn Val Ile Gln Pro Asp Gly Ser
       130                 135                 140
   Tyr Arg Ser Glu Glu Leu Asn Leu Val Ile Ile Gly Pro Ser Ala Asp
   145                 150                 155                 160
   Ile Ile Gln Phe Glu Cys Lys Ser Phe Gly His Glu Val Leu Asn Leu
                   165                 170                 175
   Thr Arg Asn Gly Tyr Gly Ser Thr Gln Tyr Ile Arg Phe Ser Pro Asp
               180                 185                 190
   Phe Thr Phe Gly Phe Glu Glu Ser Leu Glu Val Asp Thr Asn Pro Leu
           195                 200                 205
   Leu Gly Ala Gly Lys Phe Ala Thr Asp Pro Ala Val Thr Leu Ala His
       210                 215                 220
   Glu Leu Ile His Ala Gly His Arg Leu Tyr Gly Ile Ala Ile Asn Pro
   225                 230                 235                 240
   Asn Arg Val Phe Lys Val Asn Thr Asn Ala Tyr Tyr Glu Met Ser Gly
                   245                 250                 255
   Leu Glu Val Ser Phe Glu Glu Leu Arg Thr Phe Gly Gly His Asp Ala
               260                 265                 270
   Lys Phe Ile Asp Ser Leu Gln Glu Asn Glu Phe Arg Leu Tyr Tyr Tyr
           275                 280                 285
   Asn Lys Phe Lys Asp Ile Ala Ser Thr Leu Asn Lys Ala Lys Ser Ile
       290                 295                 300
   Val Gly Thr Thr Ala Ser Leu Gln Tyr Met Lys Asn Val Phe Lys Glu
   305                 310                 315                 320
   Lys Tyr Leu Leu Ser Glu Asp Thr Ser Gly Lys Phe Ser Val Asp Lys
                   325                 330                 335
   Leu Lys Phe Asp Lys Leu Tyr Lys Met Leu Thr Glu Ile Tyr Thr Glu
               340                 345                 350
   Asp Asn Phe Val Lys Phe Phe Lys Val Leu Asn Arg Lys Thr Tyr Leu
           355                 360                 365
   Asn Phe Asp Lys Ala Val Phe Lys Ile Asn Ile Val Pro Lys Val Asn
       370                 375                 380
   Tyr Thr Ile Tyr Asp Gly Phe Asn Leu Arg Asn Thr Asn Leu Ala Ala
   385                 390                 395                 400
   Asn Phe Asn Gly Gln Asn Thr Glu Ile Asn Asn Met Asn Phe Thr Lys
                   405                 410                 415
   Leu Lys Asn Phe Thr Gly Leu Phe Glu Phe Tyr Lys Leu Leu Cys Val
               420                     425                     430
   Arg Gly Ile Ile Thr Ser Lys Thr Lys Ser Leu Asp Lys Gly Tyr Asn
           435                     440                     445
   Lys Leu Val Pro Arg Gly Ser His His His His His His
       450                     455                     460
<210> 6
   <211> 1323
   <212> DNA
   <213> Artificial Sequence
<220>
   <223> Synthetic botulinum neurotoxin light chain of serotype B based on wild-type Clostridium botulinum sequence
<400> 6
   atgccagtta ctattaacaa cttcaactac aacgacccaa ttgacaacaa caacattatt 60
   atgatggagc caccattcgc tagaggtact ggtagatact acaaggcttt caagattact 120
   gacagaattt ggattattcc agagagatac actttcggtt acaagccaga ggacttcaac 180
   aagtcttctg gtattttcaa cagagacgtt tgtgagtact acgacccaga ctacttgaac 240
   actaacgaca agaagaacat tttcttgcaa actatgatta agttgttcaa cagaattaag 300
   tctaagccat tgggtgagaa gttgttggag atgattatta acggtattcc atacttgggt 360
   gacagaagag ttccattgga ggagttcaac actaacattg cttctgttac tgttaacaag 420
   ttgatttcta acccaggtga ggttgagaga aagaagggta ttttcgctaa cttgattatt 480
   ttcggtccag gtccagtttt gaacgagaac gagactattg acattggtat tcaaaaccac 540
   ttcgcttcta gagagggttt cggtggtatt atgcaaatga agttctgtcc agagtacgtt 600
   tctgttttca acaacgttca agagaacaag ggtgcttcta ttttcaacag aagaggttac 660
   ttctctgacc cagctttgat tttgatgcac gagttgattc acgttttgca cggtttgtac 720
   ggtattaagg ttgacgactt gccaattgtt ccaaacgaga agaagttctt catgcaatct 780
   actgacgcta ttcaagctga ggagttgtac actttcggtg gtcaagaccc atctattatt 840
   actccatcta ctgacaagtc tatttacgac aaggttttgc aaaacttcag aggtattgtt 900
   gacagattga acaaggtttt ggtttgtatt tctgacccaa acattaacat taacatttac 960
   aagaacaagt tcaaggacaa gtacaagttc gttgaggact ctgagggtaa gtactctatt 1020
   gacgttgagt ctttcgacaa gttgtacaag tctttgatgt tcggtttcac tgagactaac 1080
   attgctgaga actacaagat taagactaga gcttcttact tctctgactc tttgccacca 1140
   gttaagatta agaacttgtt ggacaacgag atttacacta ttgaggaggg tttcaacatt 1200
   tctgacaagg acatggagaa ggagtacaga ggtcaaaaca aggctattaa caagcaagct 1260
   tacgaggaga tttctaagga gcacttggct gtttacaaga ttcaaatgtg taagtctgtt 1320
   aag                                                               1323
<210> 7
   <211> 441
   <212> PRT
   <213> Artificial Sequence
<220>
   <223> Synthetic botulinum neurotoxin light chain of serotype B based on wild-type Clostridium botulinum sequence
<400> 7
   Met Pro Val Thr Ile Asn Asn Phe Asn Tyr Asn Asp Pro Ile Asp Asn
   1               5                   10                  15
   Asn Asn Ile Ile Met Met Glu Pro Pro Phe Ala Arg Gly Thr Gly Arg
                       20                      25                      30
   Tyr Tyr Lys Ala Phe Lys Ile Thr Asp Arg Ile Trp Ile Ile Pro Glu
               35                      40                      45
   Arg Tyr Thr Phe Gly Tyr Lys Pro Glu Asp Phe Asn Lys Ser Ser Gly
           50                      55                      60
   Ile Phe Asn Arg Asp Val Cys Glu Tyr Tyr Asp Pro Asp Tyr Leu Asn
   65                  70                      75                      80
   Thr Asn Asp Lys Lys Asn Ile Phe Leu Gln Thr Met Ile Lys Leu Phe
                   85                      90                      95
   Asn Arg Ile Lys Ser Lys Pro Leu Gly Glu Lys Leu Leu Glu Met Ile
               100                     105                     110
   Ile Asn Gly Ile Pro Tyr Leu Gly Asp Arg Arg Val Pro Leu Glu Glu
           115                     120                     125
   Phe Asn Thr Asn Ile Ala Ser Val Thr Val Asn Lys Leu Ile Ser Asn
       130                     135                     140
   Pro Gly Glu Val Glu Arg Lys Lys Gly Ile Phe Ala Asn Leu Ile Ile
   145                 150                     155                     160
   Phe Gly Pro Gly Pro Val Leu Asn Glu Asn Glu Thr Ile Asp Ile Gly
                   165                     170                     175
   Ile Gln Asn His Phe Ala Ser Arg Glu Gly Phe Gly Gly Ile Met Gln
               180                     185                     190
   Met Lys Phe Cys Pro Glu Tyr Val Ser Val Phe Asn Asn Val Gln Glu
           195                     200                     205
   Asn Lys Gly Ala Ser Ile Phe Asn Arg Arg Gly Tyr Phe Ser Asp Pro
       210                     215                     220
   Ala Leu Ile Leu Met His Glu Leu Ile His Val Leu His Gly Leu Tyr
   225                 230                     235                     240
   Gly Ile Lys Val Asp Asp Leu Pro Ile Val Pro Asn Glu Lys Lys Phe
                   245                     250                     255
   Phe Met Gln Ser Thr Asp Ala Ile Gln Ala Glu Glu Leu Tyr Thr Phe
               260                     265                     270
   Gly Gly Gln Asp Pro Ser Ile Ile Thr Pro Ser Thr Asp Lys Ser Ile
           275                     280                     285
   Tyr Asp Lys Val Leu Gln Asn Phe Arg Gly Ile Val Asp Arg Leu Asn
       290                     295                     300
   Lys Val Leu Val Cys Ile Ser Asp Pro Asn Ile Asn Ile Asn Ile Tyr
   305                 310                     315                     320
   Lys Asn Lys Phe Lys Asp Lys Tyr Lys Phe Val Glu Asp Ser Glu Gly
                   325                     330                     335
   Lys Tyr Ser Ile Asp Val Glu Ser Phe Asp Lys Leu Tyr Lys Ser Leu
               340                     345                     350
   Met Phe Gly Phe Thr Glu Thr Asn Ile Ala Glu Asn Tyr Lys Ile Lys
           355                     360                     365
   Thr Arg Ala Ser Tyr Phe Ser Asp Ser Leu Pro Pro Val Lys Ile Lys
       370                     375                     380
   Asn Leu Leu Asp Asn Glu Ile Tyr Thr Ile Glu Glu Gly Phe Asn Ile
   385                 390                     395                     400
   Ser Asp Lys Asp Met Glu Lys Glu Tyr Arg Gly Gln Asn Lys Ala Ile
                   405                     410                     415
   Asn Lys Gln Ala Tyr Glu Glu Ile Ser Lys Glu His Leu Ala Val Tyr
               420                     425                     430
   Lys Ile Gln Met Cys Lys Ser Val Lys
           435                     440
<210> 8
   <211> 1332
   <212> DNA
   <213> Artificial Sequence
<220>
   <223> Synthetic botulinum neurotoxin light chain of serotype C1 based on wild-type Clostridium botulinum sequence
<400> 8
   atgccaatca ccatcaacaa cttcaactac tcagaccctg tcgacaacaa gaacattctg 60
   tacctggaca ctcacctgaa caccctagct aacgagcctg agaaggcctt tcggatcacc 120
   ggaaacatct gggtcatccc tgatcgtttc tcccgtaact ccaaccccaa cctgaacaag 180
   cctcctcggg tcaccagccc taagagtggt tactacgacc ctaactacct gagtaccgac 240
   tctgacaagg acaccttcct gaaggagatc atcaagctgt tcaagcgtat caactcccgt 300
   gagatcggag aggagctcat ctacagactt tcgaccgata tccccttccc tggtaacaac 360
   aatactccaa tcaacacctt cgacttcgac gtcgacttca actccgtcga cgtcaagact 420
   cggcagggta acaactgggt taagactggt agcatcaacc cttccgtcat catcactgga 480
   cctcgtgaga acatcatcga cccagagact tccacgttca agctgactaa caacaccttc 540
   gcggctcaag aaggattcgg tgctctgtca atcatctcca tctcacctcg tttcatgctg 600
   acctactcga acgcaaccaa cgacgtcgga gagggtaggt tctctaagtc tgagttctgc 660
   atggacccaa tcctgatcct gatgcatgag ctgaaccatg caatgcacaa cctgtacgga 720
   atcgctatcc caaacgacca gaccatctcc tccgtgacct ccaacatctt ctactcccag 780
   tacaacgtga agctggagta cgcagagatc tacgctttcg gaggtccaac tatcgacctt 840
   atccctaagt ccgctaggaa gtacttcgag gagaaggctt tggattacta cagatccatc 900
   gctaagagac tgaacagtat caccaccgca aacccttcca gcttcaacaa gtacatcggt 960
   gagtacaagc agaagctgat cagaaagtac cgtttcgtcg tcgagtcttc aggtgaggtc 1020
   acagtaaacc gtaacaagtt cgtcgagctg tacaacgagc ttacccagat cttcacagag 1080
   ttcaactacg ctaagatcta caacgtccag aacaggaaga tctacctgtc caacgtgtac 1140
   actccggtga cggcgaacat cctggacgac aacgtctacg acatccagaa cggattcaac 1200
   atccctaagt ccaacctgaa cgtactattc atgggtcaaa acctgtctcg aaacccagca 1260
   ctgcgtaagg tcaaccctga gaacatgctg tacctgttca ccaagttctg ccacaaggca 1320
   atcgacggta ga                                                    1332
<210> 9
   <211> 444
   <212> PRT
   <213> Artificial Sequence
<220>
   <223> Synthetic botulinum neurotoxin light chain of serotype C1 based on wild-type Clostridium botulinum sequence
<400> 9
   Met Pro Ile Thr Ile Asn Asn Phe Asn Tyr Ser Asp Pro Val Asp Asn
   1               5                   10                  15
   Lys Asn Ile Leu Tyr Leu Asp Thr His Leu Asn Thr Leu Ala Asn Glu
               20                  25                  30
   Pro Glu Lys Ala Phe Arg Ile Thr Gly Asn Ile Trp Val Ile Pro Asp
           35                  40                  45
   Arg Phe Ser Arg Asn Ser Asn Pro Asn Leu Asn Lys Pro Pro Arg Val
       50                  55                  60
   Thr Ser Pro Lys Ser Gly Tyr Tyr Asp Pro Asn Tyr Leu Ser Thr Asp
   65                  70                  75                  80
   Ser Asp Lys Asp Thr Phe Leu Lys Glu Ile Ile Lys Leu Phe Lys Arg
                           85                  90                  95
   Ile Asn Ser Arg Glu Ile Gly Glu Glu Leu Ile Tyr Arg Leu Ser Thr
                   100                 105                 110
   Asp Ile Pro Phe Pro Gly Asn Asn Asn Thr Pro Ile Asn Thr Phe Asp
               115                 120                 125
   Phe Asp Val Asp Phe Asn Ser Val Asp Val Lys Thr Arg Gln Gly Asn
       130                 135                 140
   Asn Trp Val Lys Thr Gly Ser Ile Asn Pro Ser Val Ile Ile Thr Gly
   145                 150                 155                 160
   Pro Arg Glu Asn Ile Ile Asp Pro Glu Thr Ser Thr Phe Lys Leu Thr
                   165                 170                 175
   Asn Asn Thr Phe Ala Ala Gln Glu Gly Phe Gly Ala Leu Ser Ile Ile
               180                 185                 190
   Ser Ile Ser Pro Arg Phe Met Leu Thr Tyr Ser Asn Ala Thr Asn Asp
           195                 200                 205
   Val Gly Glu Gly Arg Phe Ser Lys Ser Glu Phe Cys Met Asp Pro Ile
       210                 215                 220
   Leu Ile Leu Met His Glu Leu Asn His Ala Met His Asn Leu Tyr Gly
   225                 230                 235                 240
   Ile Ala Ile Pro Asn Asp Gln Thr Ile Ser Ser Val Thr Ser Asn Ile
                   245                 250                 255
   Phe Tyr Ser Gln Tyr Asn Val Lys Leu Glu Tyr Ala Glu Ile Tyr Ala
               260                 265                 270
   Phe Gly Gly Pro Thr Ile Asp Leu Ile Pro Lys Ser Ala Arg Lys Tyr
           275                 280                 285
   Phe Glu Glu Lys Ala Leu Asp Tyr Tyr Arg Ser Ile Ala Lys Arg Leu
       290                 295                 300
   Asn Ser Ile Thr Thr Ala Asn Pro Ser Ser Phe Asn Lys Tyr Ile Gly
   305                 310                 315                 320
   Glu Tyr Lys Gln Lys Leu Ile Arg Lys Tyr Arg Phe Val Val Glu Ser
                   325                 330                 335
   Ser Gly Glu Val Thr Val Asn Arg Asn Lys Phe Val Glu Leu Tyr Asn
               340                 345                 350
   Glu Leu Thr Gln Ile Phe Thr Glu Phe Asn Tyr Ala Lys Ile Tyr Asn
           355                 360                 365
   Val Gln Asn Arg Lys Ile Tyr Leu Ser Asn Val Tyr Thr Pro Val Thr
       370                 375                 380
   Ala Asn Ile Leu Asp Asp Asn Val Tyr Asp Ile Gln Asn Gly Phe Asn
   385                 390                 395                 400
   Ile Pro Lys Ser Asn Leu Asn Val Leu Phe Met Gly Gln Asn Leu Ser
                   405                 410                 415
   Arg Asn Pro Ala Leu Arg Lys Val Asn Pro Glu Asn Met Leu Tyr Leu
               420                 425                 430
   Phe Thr Lys Phe Cys His Lys Ala Ile Asp Gly Arg
           435                 440
<210> 10
   <211> 1323
   <212> DNA
   <213> Artificial Sequence
<220>
   <223> Synthetic botulinum neurotoxin light chain of serotype D based on wild-type Clostridium botulinum sequence
<210> 11
   <211> 441
   <212> PRT
   <213> Artificial Sequence
<220>
   <233> Synthetic botulinum neurotoxin light chain of serotype D based on wild-type Clostridium botulinum sequence
<400> 11
   Met Thr Trp Pro Val Lys Asp Phe Asn Tyr Ser Asp Pro Val Asn Asp
   1               5                   10                  15
   Asn Asp Ile Leu Tyr Leu Arg Ile Pro Gln Asn Lys Leu Ile Thr Thr
               20                  25                  30
   Pro Val Lys Ala Phe Met Ile Thr Gln Asn Thr Trp Val Ile Pro Glu
           35                  40                  45
   Arg Phe Ser Ser Asp Thr Asn Pro Ser Leu Ser Lys Pro Pro Arg Pro
       50                  55                  60
   Thr Ser Lys Tyr Gln Ser Tyr Tyr Asp Pro Ser Tyr Leu Ser Thr Asp
   65                  70                  75                  80
   Glu Gln Lys Asp Thr Phe Leu Lys Gly Ile Ile Lys Leu Phe Lys Arg
                   85                  90                  95
   Ile Asn Glu Arg Asp Ile Gly Lys Lys Leu Ile Asn Tyr Leu Val Val
               100                 105                 110
   Gly Ser Pro Phe Met Gly Asp Ser Ser Thr Pro Glu Asp Thr Phe Asp
           115                 120                 125
   Phe Thr Arg His Thr Thr Asn Ile Ala Val Glu Lys Phe Glu Asn Gly
       130                 135                 140
   Ser Trp Lys Val Thr Asn Ile Ile Thr Pro Ser Val Leu Ile Phe Gly
   145                 150                 155                 160
   Pro Leu Pro Asn Ile Leu Asp Tyr Thr Ala Ser Leu Thr Leu Gln Gly
                   165                 170                 175
   Gln Gln Ser Asn Pro Ser Phe Glu Gly Phe Gly Thr Leu Ser Ile Leu
               180                 185                 190
   Lys Val Ala Pro Glu Phe Leu Leu Thr Phe Ser Asp Val Thr Ser Asn
           195                 200                 205
   Gln Ser Ser Ala Val Leu Gly Lys Ser Ile Phe Cys Met Asp Pro Val
       210                 215                 220
   Ile Ala Leu Met His Glu Leu Thr His Ser Leu His Gln Leu Tyr Gly
   225                 230                 235                 240
   Ile Asn Ile Pro Ser Asp Lys Arg Ile Arg Pro Gln Val Ser Glu Gly
                   245                 250                 255
   Phe Phe Ser Gln Asp Gly Pro Asn Val Gln Phe Glu Glu Leu Tyr Thr
               260                 265                 270
   Phe Gly Gly Leu Asp Val Glu Ile Ile Gln Ile Glu Arg Ser Gln Leu
           275                 280                 285
   Arg Glu Lys Ala Leu Gly His Tyr Lys Asp Ile Ala Lys Arg Leu Asn
       290                 295                 300
   Asn Ile Asn Lys Thr Ile Pro Ser Ser Trp Ile Ser Asn Ile Asp Lys
   305                 310                 315                 320
   Tyr Lys Lys Ile Phe Ser Glu Lys Tyr Asn Phe Asp Lys Asp Asn Thr
                   325                 330                 335
   Gly Asn Phe Val Val Asn Ile Asp Lys Phe Asn Ser Leu Tyr Ser Asp
               340                 345                 350
   Leu Thr Asn Val Met Ser Glu Val Val Tyr Ser Ser Gln Tyr Asn Val
           355                 360                 365
   Lys Asn Arg Thr His Tyr Phe Ser Arg His Tyr Leu Pro Val Phe Ala
       370                 375                 380
   Asn Ile Leu Asp Asp Asn Ile Tyr Thr Ile Arg Asp Gly Phe Asn Leu
   385                 390                 395                 400
   Thr Asn Lys Gly Phe Asn Ile Glu Asn Ser Gly Gln Asn Ile Glu Arg
                   405                 410                 415
   Asn Pro Ala Leu Gln Lys Leu Ser Ser Glu Ser Val Val Asp Leu Phe
               420                 425                 430
   Thr Lys Val Cys Leu Arg Leu Thr Lys
           435                 440
<210> 12
   <211> 1266
   <212> DNA
   <213> Artificial Sequence
<220>
   <223> Synthetic botulinum neurotoxin light chain of serotype E based on wild-type Clostridium botulinum sequence
<400> 12
   atgccaaaga ttaactcctt caactacaac gaccctgtca acgacagaac catcttgtac 60
   atcaagccag gcggttgcca ggagttctac aagtccttca acatcatgaa gaacatctgg 120
   atcatccccg agagaaacgt cattggtacc accccccaag acttccaccc ccctacttcc 180
   ttgaagaacg gagactccag ttactacgac cctaactact tgcaaagtga cgaggagaag 240
   gacagattct tgaagatcgt cacaaagatc ttcaacagaa tcaacaacaa cctttcagga 300
   ggcatcttgt tggaggagct gtccaaggct aacccatact tgggcaacga caacactcca 360
   gataaccagt tccacattgg tgacgcatcc gcagttgaga ttaagttctc caacggtagc 420
   caggacatcc tattgcctaa cgttatcatc atgggagcag agcctgactt gtttgagacc 480
   aactcctcca acatctctct acgtaacaac tacatgccaa gcaatcacgg tttcggatcc 540
   atcgctatcg tcaccttctc ccctgaatat tccttcaggt tcaacgacaa cagcatgaac 600
   gagttcattc aggatcctgc tctcacgctg atgcacgaat tgatccactc cttacatgga 660
   ctatatggcg ctaagggcat tactaccaag tacactatca cacagaagca gaacccccta 720
   ataaccaaca tccggggtac caacatcgag gagttcttga ctttcggagg tactgacttg 780
   aacatcatta ctagtgctca gtccaacgac atctacacta accttctggc tgactacaag 840
   aagatcgcgt ctaagcttag caaggtccaa gtctctaacc cactgcttaa cccttacaag 900
   gacgtcttcg aagcaaagta tggattggac aaggatgcta gcggaattta ctcggtcaac 960
   atcaacaagt tcaacgacat cttcaagaag ctctacagct tcacggagtt cgacttggcc 1020
   accaagttcc aggttaagtg taggcagact tacatcggac agtacaagta cttcaagctg 1080
   tccaacctgt tgaacgactc tatctacaac atctcagaag gctacaacat caacaacttg 1140
   aaggtcaact tcagaggaca gaatgcaaac ttgaacccta gaatcattac cccaatcacc 1200
   ggtagaggac tggtcaagaa gatcatccgt ttctgcaaga acattgtctc tgtcaagggc 1260
   atcagg                                                            1266
<210> 13
   <211> 422
   <212> PRT
   <213> Artificial Sequence
<220>
   <223> Synthetic botulinum neurotoxin light chain of serotype E based on wild-type Clostridium botulinum sequence
<400> 13
   Met Pro Lys Ile Asn Ser Phe Asn Tyr Asn Asp Pro Val Asn Asp Arg
   1               5                   10                  15
   Thr Ile Leu Tyr Ile Lys Pro Gly Gly Cys Gln Glu Phe Tyr Lys Ser
               20                  25                  30
   Phe Asn Ile Met Lys Asn Ile Trp Ile Ile Pro Glu Arg Asn Val Ile
           35                  40                  45
   Gly Thr Thr Pro Gln Asp Phe His Pro Pro Thr Ser Leu Lys Asn Gly
       50                  55                  60
   Asp Ser Ser Tyr Tyr Asp Pro Asn Tyr Leu Gln Ser Asp Glu Glu Lys
   65                  70                  75                  80
   Asp Arg Phe Leu Lys Ile Val Thr Lys Ile Phe Asn Arg Ile Asn Asn
                   85                  90                  95
   Asn Leu Ser Gly Gly Ile Leu Leu Glu Glu Leu Ser Lys Ala Asn Pro
               100                 105                 110
   Tyr Leu Gly Asn Asp Asn Thr Pro Asp Asn Gln Phe His Ile Gly Asp
           115                 120                 125
   Ala Ser Ala Val Glu Ile Lys Phe Ser Asn Gly Ser Gln Asp Ile Leu
       130                 135                 140
   Leu Pro Asn Val Ile Ile Met Gly Ala Glu Pro Asp Leu Phe Glu Thr
   145                 150                 155                 160
   Asn Ser Ser Asn Ile Ser Leu Arg Asn Asn Tyr Met Pro Ser Asn His
                   165                 170                 175
   Gly Phe Gly Ser Ile Ala Ile Val Thr Phe Ser Pro Glu Tyr Ser Phe
               180                 185                 190
   Arg Phe Asn Asp Asn Ser Met Asn Glu Phe Ile Gln Asp Pro Ala Leu
           195                 200                 205
   Thr Leu Met His Glu Leu Ile His Ser Leu His Gly Leu Tyr Gly Ala
       210                 215                 220
   Lys Gly Ile Thr Thr Lys Tyr Thr Ile Thr Gln Lys Gln Asn Pro Leu
   225 230 235 240
   Ile Thr Asn Ile Arg Gly Thr Asn Ile Glu Glu Phe Leu Thr Phe Gly
   245 250 255
   Gly Thr Asp Leu Asn Ile Ile Thr Ser Ala Gln Ser Asn Asp Ile Tyr
   260 265 270
   Thr Asn Leu Leu Ala Asp Tyr Lys Lys Ile Ala Ser Lys Leu Ser Lys
   275 280 285
   Val Gln Val Ser Asn Pro Leu Leu Asn Pro Tyr Lys Asp Val Phe Glu
   290 295 300
   Ala Lys Tyr Gly Leu Asp Lys Asp Ala Ser Gly Ile Tyr Ser Val Asn
   305 310 315 320
   Ile Asn Lys Phe Asn Asp Ile Phe Lys Lys Leu Tyr Ser Phe Thr Glu
   325 330 335
   Phe Asp Leu Ala Thr Lys Phe Gln Val Lys Cys Arg Gln Thr Tyr Ile
   340 345 350
   Gly Gln Tyr Lys Tyr Phe Lys Leu Ser Asn Leu Leu Asn Asp Ser Ile
   355 360 365
   Tyr Asn Ile Ser Glu Gly Tyr Asn Ile Asn Asn Leu Lys Val Asn Phe
   370 375 380
   Arg Gly Gln Asn Ala Asn Leu Asn Pro Arg Ile Ile Thr Pro Ile Thr
   385 390 395 400
   Gly Arg Gly Leu Val Lys Lys Ile Ile Arg Phe Cys Lys Asn Ile Val
   405 410 415
   Ser Val Lys Gly Ile Arg
   420
<210> 14
   <211> 1308
   <212> DNA
   <213> Artificial Sequence
<220>
   <223> Synthetic botulinum neurotoxin light chain of serotype F based on wild-type Clostridium botulinum sequence
<400> 14
   atgccagtcg ctatcaactc cttcaactac aacgacccag tcaacgacga caccattttg 60
   tacatgcaga tcccatacga ggagaagtct aagaagtact acaaggcttt cgagatcatg 120
   agaaacgtct ggattatcga gagaaacacc atcggtacca acccatccga cttcgaccca 180
   ccagcctctt tgaagaacgg ttcctccgct tactacgacc caaactactt gaccaccgac 240
   gccgagaagg acagatactt gaagaccacc atcaagttgt tcaagagaat taactctaac 300
   ccagccggta aggtcttgtt gcaagagatc tcctacgcta agccatacct gggtaacgac 360
   cacaccccaa ttgacgagtt ctccccagtc accagaacca cctccgtcaa catcaagtct 420
   accaacgttg agtcctccat gttgttgaac ttgttggttc tgggtgctgg tccagacatt 480
   ttcgagtctt gttgttaccc agtcagaaag ctgatcgacc cagacgttgt ttacgaccca 540
   tctaactacg gtttcggttc cattaacatc gttaccttct ctccagagta cgagtacacc 600
   ttcaacgaca tctccggtgg tcacaactcc tccaccgagt ctttcattgc tgacccagcc 660
   atctccctgg ctcacgagct gattcacgct ttgcacggtt tgtacggtgc tagaggtgtc 720
   acctacgagg agaccattga ggtcaagcaa gccccattga tgatcgccga gaagccaatc 780
   agattggagg agttcttgac cttcggtggt caggacttga acatcatcac ctccgctatg 840
   aaggagaaga tctacaacaa cctgctggcc aactacgaga agattgccac cagattgtcc 900
   gaggtcaact ctgccccacc agagtacgac atcaacgagt acaaggacta cttccaatgg 960
   aagtacggtt tggacaagaa cgccgacggt tcctacaccg tcaacgagaa caagtccaac 1020
   gagatttaca agaagttgta ctctttcacc gagtccgacc tggctaacaa gttcaaggtt 1080
   aagtgtagaa acacctactt catcaagtac gagttcttga aggttccaaa cctgttggac 1140
   gacgacatct acaccgtttc tgagggtttc aacatcggta acttggctgt caacaacaga 1200
   ggtcagtcca ttaagctgaa cccaaagatc attgactccc cagacaaggg tctggttgag 1260
   aagattgtca agttctgtaa gtccgtcatc ccaagaaagg gtaccaag               1308
<210> 15
   <211> 436
   <212> PRT
   <213> Artificial Sequence
<220>
   <223> Synthetic botulinum neurotoxin light chain of serotype F based on wild-type Clostridium botulinum sequence
<400> 15
   Met Pro Val Ala Ile Asn Ser Phe Asn Tyr Asn Asp Pro Val Asn Asp
    1               5                  10                  15
   Asp Thr Ile Leu Tyr Met Gln Ile Pro Tyr Glu Glu Lys Ser Lys Lys
               20                  25                  30
   Tyr Tyr Lys Ala Phe Glu Ile Met Arg Asn Val Trp Ile Ile Glu Arg
           35                  40                  45
   Asn Thr Ile Gly Thr Asn Pro Ser Asp Phe Asp Pro Pro Ala Ser Leu
       50                  55                  60
   Lys Asn Gly Ser Ser Ala Tyr Tyr Asp Pro Asn Tyr Leu Thr Thr Asp
   65                  70                  75                  80
   Ala Glu Lys Asp Arg Tyr Leu Lys Thr Thr Ile Lys Leu Phe Lys Arg
                   85                  90                  95
   Ile Asn Ser Asn Pro Ala Gly Lys Val Leu Leu Gln Glu Ile Ser Tyr
               100                 105                 110
   Ala Lys Pro Tyr Leu Gly Asn Asp His Thr Pro Ile Asp Glu Phe Ser
           115                 120                 125
   Pro Val Thr Arg Thr Thr Ser Val Asn Ile Lys Ser Thr Asn Val Glu
       130                 135                 140
   Ser Ser Met Leu Leu Asn Leu Leu Val Leu Gly Ala Gly Pro Asp Ile
   145                 150                 155                 160
   Phe Glu Ser Cys Cys Tyr Pro Val Arg Lys Leu Ile Asp Pro Asp Val
                   165                 170                 175
   Val Tyr Asp Pro Ser Asn Tyr Gly Phe Gly Ser Ile Asn Ile Val Thr
               180                 185                 190
   Phe Ser Pro Glu Tyr Glu Tyr Thr Phe Asn Asp Ile Ser Gly Gly His
           195                 200                 205
   Asn Ser Ser Thr Glu Ser Phe Ile Ala Asp Pro Ala Ile Ser Leu Ala
       210                 215                 220
   His Glu Leu Ile His Ala Leu His Gly Leu Tyr Gly Ala Arg Gly Val
   225                 230                 235                 240
   Thr Tyr Glu Glu Thr Ile Glu Val Lys Gln Ala Pro Leu Met Ile Ala
                   245                 250                 255
   Glu Lys Pro Ile Arg Leu Glu Glu Phe Leu Thr Phe Gly Gly Gln Asp
               260                 265                 270
   Leu Asn Ile Ile Thr Ser Ala Met Lys Glu Lys Ile Tyr Asn Asn Leu
           275                 280                 285
   Leu Ala Asn Tyr Glu Lys Ile Ala Thr Arg Leu Ser Glu Val Asn Ser
       290                 295                 300
   Ala Pro Pro Glu Tyr Asp Ile Asn Glu Tyr Lys Asp Tyr Phe Gln Trp
   305                 310                 315                 320
   Lys Tyr Gly Leu Asp Lys Asn Ala Asp Gly Ser Tyr Thr Val Asn Glu
                   325                 330                 335
   Asn Lys Ser Asn Glu Ile Tyr Lys Lys Leu Tyr Ser Phe Thr Glu Ser
               340                 345                 350
   Asp Leu Ala Asn Lys Phe Lys Val Lys Cys Arg Asn Thr Tyr Phe Ile
           355                 360                 365
   Lys Tyr Glu Phe Leu Lys Val Pro Asn Leu Leu Asp Asp Asp Ile Tyr
       370                 375                 380
   Thr Val Ser Glu Gly Phe Asn Ile Gly Asn Leu Ala Val Asn Asn Arg
   385                 390                 395                 400
   Gly Gln Ser Ile Lys Leu Asn Pro Lys Ile Ile Asp Ser Pro Asp Lys
                   405                 410                 415
   Gly Leu Val Glu Lys Ile Val Lys Phe Cys Lys Ser Val Ile Pro Arg
               420                 425                 430
   Lys Gly Thr Lys
           435
<210> 16
   <211> 1317
   <212> DNA
   <213> Artificial Sequence
<220>
   <223> Synthetic botulinum neurotoxin light chain of serotype G based on wild-type Clostridium botulinum sequence
<400> 16
   atgccagtca acatcaagaa cttcaactac aacgacccaa ttaacaacga cgacatcatg 60
   atggagccat tcaacgaccc aggtccaggt acctactaca aggctttcag aatcattgac 120
   agaatttgga tcgttccaga gagattcacc tacggtttcc aaccagacca gttcaacgcc 180
   tccaccggtg tcttctctaa ggacgtctac gagtactacg acccaaccta cttgaagacc 240
   gacgctgaga aggacaagtt cttgaagacc atgatcaagt tgttcaacag aattaactct 300
   aagccatccg gtcaaagatt gttggacatg attgttgacg ctattccata cttgggtaac 360
   gcctccaccc caccagacaa gttcgctgcc aacgtcgcta acgtttctat caacaagaag 420
   attatccaac caggtgctga ggaccagatc aagggtttga tgaccaactt gattattttc 480
   ggtccaggtc cagtcttgtc cgacaacttc accgactcta tgatcatgaa cggtcactcc 540
   ccaatttccg agggtttcgg tgctagaatg atgatcagat tctgtccatc ctgtttgaac 600
   gttttcaaca acgtccaaga gaacaaggac acctctatct tctctagaag agcttacttc 660
   gctgacccag ctctgaccct gatgcacgag ttgatccacg tcttgcacgg tctgtacggt 720
   attaagatct ccaacctgcc aattacccca aacaccaagg agttcttcat gcaacactcc 780
   gacccagttc aagccgagga gctgtacacc ttcggtggtc acgacccatc tgtttcccca 840
   tctaccgaca tgaacattta caacaaggct ctgcagaact tccaagacat tgctaacaga 900
   ctgaacatcg tctcctctgc ccaaggttct ggtatcgaca tttccttgta caagcaaatc 960
   tacaagaaca agtacgactt cgtcgaggac ccaaacggta agtactctgt tgacaaggac 1020
   aagttcgaca agctgtacaa ggctttgatg ttcggtttca ccgagaccaa cttggccggt 1080
   gagtacggta ttaagaccag atactcttac ttctctgagt acctgccacc aatcaagacc 1140
   gagaagttgt tggacaacac catctacacc cagaacgagg gtttcaacat tgcttccaag 1200
   aacttgaaga acgagttcaa cggtcagaac aaggccgtca acaaggaggc ctacgaggag 1260
   atttccctgg agcacttggt catctacaga atcgctatgt gtaagccagt catgtac    1317
<210> 17
   <211> 439
   <212> PRT
   <213> Artificial Sequence
<220>
   <323> Synthetic botulinum neurotoxin light chain of serotype G based on wild-type Clostridium botulinum sequence
<400> 17
   Met Pro Val Asn Ile Lys Asn Phe Asn Tyr Asn Asp Pro Ile Asn Asn
    1               5                  10                  15
   Asp Asp Ile Met Met Glu Pro Phe Asn Asp Pro Gly Pro Gly Thr Tyr
               20                  25                  30
   Tyr Lys Ala Phe Arg Ile Ile Asp Arg Ile Trp Ile Val Pro Glu Arg
           35                  40                  45
   Phe Thr Tyr Gly Phe Gln Pro Asp Gln Phe Asn Ala Ser Thr Gly Val
       50                  55                  60
   Phe Ser Lys Asp Val Tyr Glu Tyr Tyr Asp Pro Thr Tyr Leu Lys Thr
   65                  70                  75                  80
   Asp Ala Glu Lys Asp Lys Phe Leu Lys Thr Met Ile Lys Leu Phe Asn
                   85                  90                  95
   Arg Ile Asn Ser Lys Pro Ser Gly Gln Arg Leu Leu Asp Met Ile Val
               100                 105                 110
   Asp Ala Ile Pro Tyr Leu Gly Asn Ala Ser Thr Pro Pro Asp Lys Phe
           115                 120                 125
   Ala Ala Asn Val Ala Asn Val Ser Ile Asn Lys Lys Ile Ile Gln Pro
       130                 135                 140
   Gly Ala Glu Asp Gln Ile Lys Gly Leu Met Thr Asn Leu Ile Ile Phe
   145                 150                 155                 160
   Gly Pro Gly Pro Val Leu Ser Asp Asn Phe Thr Asp Ser Met Ile Met
                   165                 170                 175
   Asn Gly His Ser Pro Ile Ser Glu Gly Phe Gly Ala Arg Met Met Ile
               180                 185                 190
   Arg Phe Cys Pro Ser Cys Leu Asn Val Phe Asn Asn Val Gln Glu Asn
           195                 200                 205
   Lys Asp Thr Ser Ile Phe Ser Arg Arg Ala Tyr Phe Ala Asp Pro Ala
       210                 215                 220
   Leu Thr Leu Met His Glu Leu Ile His Val Leu His Gly Leu Tyr Gly
   225                 230                 235                 240
   Ile Lys Ile Ser Asn Leu Pro Ile Thr Pro Asn Thr Lys Glu Phe Phe
                   245                 250                 255
   Met Gln His Ser Asp Pro Val Gln Ala Glu Glu Leu Tyr Thr Phe Gly
               260                 265                 270
   Gly His Asp Pro Ser Val Ser Pro Ser Thr Asp Met Asn Ile Tyr Asn
           275                 280                 285
   Lys Ala Leu Gln Asn Phe Gln Asp Ile Ala Asn Arg Leu Asn Ile Val
       290                 295                 300
   Ser Ser Ala Gln Gly Ser Gly Ile Asp Ile Ser Leu Tyr Lys Gln Ile
   305                 310                 315                 320
   Tyr Lys Asn Lys Tyr Asp Phe Val Glu Asp Pro Asn Gly Lys Tyr Ser
                   325                 330                 335
   Val Asp Lys Asp Lys Phe Asp Lys Leu Tyr Lys Ala Leu Met Phe Gly
               340                 345                 350
   Phe Thr Glu Thr Asn Leu Ala Gly Glu Tyr Gly Ile Lys Thr Arg Tyr
           355                 360                 365
   Ser Tyr Phe Ser Glu Tyr Leu Pro Pro Ile Lys Thr Glu Lys Leu Leu
       370                 375                 380
   Asp Asn Thr Ile Tyr Thr Gln Asn Glu Gly Phe Asn Ile Ala Ser Lys
   385 390 395 400
   Asn Leu Lys Asn Glu Phe Asn Gly Gln Asn Lys Ala Val Asn Lys Glu
   405 410 415
   Ala Tyr Glu Glu Ile Ser Leu Glu His Leu Val Ile Tyr Arg Ile Ala
   420 425 430
   Met Cys Lys Pro Val Met Tyr
   435
<210> 18
   <211> 1239
   <212> DNA
   <213> Artificial Sequence
<220>
   <223> Synthetic N-terminal region of the heavy chain of botulinum neurotoxin serotype A based on wild-type Clostridium botulinum sequence
<400> 18
   atggctctga acgacctgtg catcaaagtt aacaactggg acctgttctt ctccccgtct 60
   gaagacaact tcactaacga cctgaacaaa ggcgaagaaa tcacctccga cactaacatc 120
   gaagctgctg aagaaaacat ctctctggac ctgatccagc agtactacct gactttcaac 180
   ttcgacaacg aaccggaaaa catctccatc gaaaacctgt cttccgacat catcggtcag 240
   ctggaactga tgccgaacat cgaacgcttc ccgaacggca agaaatacga actggacaaa 300
   tacaccatgt tccactacct gcgtgctcag gaattcgaac acggtaaatc tcgtatcgct 360
   ctgactaact ccgttaacga agctctgctg aacccgtctc gcgtttacac cttcttctct 420
   tccgactacg ttaagaaagt taacaaagct actgaagctg ctatgttcct gggttgggtt 480
   gaacagctgg tttacgactt caccgacgaa acttctgaag tttccaccac tgacaaaatc 540
   gctgacatca ctatcatcat cccgtacatc ggcccggctc tgaacatcgg taacatgctg 600
   tacaaagacg acttcgttgg tgctctgatc ttctctggcg ctgttatcct gctggaattc 660
   atcccggaaa tcgctatccc ggttctgggt accttcgctc tggtttccta catcgctaac 720
   aaagttctga ctgttcagac catcgacaac gctctgtcta aacgtaacga aaaatgggac 780
   gaagtttaca aatacatcgt tactaactgg ctggctaaag ttaacactca gatcgacctg 840
   atccgtaaga agatgaaaga agctctggaa aaccaggctg aagctactaa agctatcatc 900
   aactaccagt acaaccagta caccgaagaa gaaaagaaca acatcaactt caacatcgat 960
   gacctgtcct ctaaactgaa cgaatccatc aacaaagcta tgatcaacat caacaaattc 1020
   ctgaaccagt gctctgtttc ctacctgatg aactctatga tcccgtacgg cgttaaacgc 1080
   ctggaagact tcgacgcttc cctgaaagac gctctgctga aatacatccg tgacaactac 1140
   ggtactctga tcggccaggt tgaccgtctg aaagacaagg ttaacaacac cctgtctact 1200
   gacatcccgt tccagctgtc caaatacgtt gacaaccag 1239
<210> 19
   <211> 413
   <212> PRT
   <213> Artificial Sequence
<220>
   <223> Synthetic N-terminal region of the heavy chain of botulinum neurotoxin serotype A based on wild-type Clostridium botulinum sequence
<400> 19
   Met Ala Leu Asn Asp Leu Cys Ile Lys Val Asn Asn Trp Asp Leu Phe
   1               5                   10                  15
   Phe Ser Pro Ser Glu Asp Asn Phe Thr Asn Asp Leu Asn Lys Gly Glu
               20                  25                  30
   Glu Ile Thr Ser Asp Thr Asn Ile Glu Ala Ala Glu Glu Asn Ile Ser
           35                  40                  45
   Leu Asp Leu Ile Gln Gln Tyr Tyr Leu Thr Phe Asn Phe Asp Asn Glu
       50                  55                  60
   Pro Glu Asn Ile Ser Ile Glu Asn Leu Ser Ser Asp Ile Ile Gly Gln
   65                  70                  75                  80
   Leu Glu Leu Met Pro Asn Ile Glu Arg Phe Pro Asn Gly Lys Lys Tyr
                   85                  90                  95
   Glu Leu Asp Lys Tyr Thr Met Phe His Tyr Leu Arg Ala Gln Glu Phe
               100                 105                 110
   Glu His Gly Lys Ser Arg Ile Ala Leu Thr Asn Ser Val Asn Glu Ala
           115                 120                 125
   Leu Leu Asn Pro Ser Arg Val Tyr Thr Phe Phe Ser Ser Asp Tyr Val
       130                 135                 140
   Lys Lys Val Asn Lys Ala Thr Glu Ala Ala Met Phe Leu Gly Trp Val
   145                 150                 155                 160
   Glu Gln Leu Val Tyr Asp Phe Thr Asp Glu Thr Ser Glu Val Ser Thr
                   165                 170                 175
   Thr Asp Lys Ile Ala Asp Ile Thr Ile Ile Ile Pro Tyr Ile Gly Pro
               180                 185                 190
   Ala Leu Asn Ile Gly Asn Met Leu Tyr Lys Asp Asp Phe Val Gly Ala
           195                 200                 205
   Leu Ile Phe Ser Gly Ala Val Ile Leu Leu Glu Phe Ile Pro Glu Ile
       210                 215                 220
   Ala Ile Pro Val Leu Gly Thr Phe Ala Leu Val Ser Tyr Ile Ala Asn
   225                 230                 235                 240
   Lys Val Leu Thr Val Gln Thr Ile Asp Asn Ala Leu Ser Lys Arg Asn
                   245                 250                 255
   Glu Lys Trp Asp Glu Val Tyr Lys Tyr Ile Val Thr Asn Trp Leu Ala
               260                 265                 270
   Lys Val Asn Thr Gln Ile Asp Leu Ile Arg Lys Lys Met Lys Glu Ala
           275                 280                 285
   Leu Glu Asn Gln Ala Glu Ala Thr Lys Ala Ile Ile Asn Tyr Gln Tyr
       290                 295                 300
   Asn Gln Tyr Thr Glu Glu Glu Lys Asn Asn Ile Asn Phe Asn Ile Asp
   305                 310                 315                 320
   Asp Leu Ser Ser Lys Leu Asn Glu Ser Ile Asn Lys Ala Met Ile Asn
                   325                 330                 335
   Ile Asn Lys Phe Leu Asn Gln Cys Ser Val Ser Tyr Leu Met Asn Ser
               340                 345                 350
   Met Ile Pro Tyr Gly Val Lys Arg Leu Glu Asp Phe Asp Ala Ser Leu
           355                 360                 365
   Lys Asp Ala Leu Leu Lys Tyr Ile Arg Asp Asn Tyr Gly Thr Leu Ile
       370                 375                 380
   Gly Gln Val Asp Arg Leu Lys Asp Lys Val Asn Asn Thr Leu Ser Thr
   385                 390                 395                 400
   Asp Ile Pro Phe Gln Leu Ser Lys Tyr Val Asp Asn Gln
                   405                 410
<210> 20
   <211> 2583
   <212> DNA
   <213> Artificial Sequence
<220>
   <223> Synthetic polynucleotide sequence for the light chain with Hn of C. botulinum Type A.
<400> 20
   atggttcagt tcgttaacaa acagttcaac tacaaagacc cggttaacgg tgttgacatc 60
   gcttacatca aaatcccgaa cgttggtcag atgcagccgg ttaaagcatt caaaatccac 120
   aacaaaatct gggttatccc ggaacgtgac actttcacta acccggaaga aggtgacctg 180
   aacccgccgc cggaagctaa acaggttccg gtttcttact acgactctac ttacctgtct 240
   actgacaacg aaaaggacaa ctacctgaaa ggtgttacta aactgtttga acgtatctac 300
   tctactgacc tgggtcgcat gctgctcact tctatcgttc gtggtatccc gttctggggt 360
   ggttctacta tcgacactga actgaaagtt atcgacacta actgcatcaa cgttatccag 420
   ccggacggtt cttaccgttc tgaagaactg aacctggtta tcatcggtcc gtctgctgac 480
   atcatccagt ttgaatgcaa atctttcggt cacgaagttc tgaacctgac tcgtaacggt 540
   tacggttcta ctcagtacat ccgtttctct ccggacttca ctttcggttt cgaagaatct 600
   ctggaagttg acactaaccc gctgctgggt gctggtaaat tcgctactga cccggctgtt 660
   actctggctc acgaactgat ccacgctggt caccgtctgt acggtatcgc tatcaacccg 720
   aaccgtgttt tcaaagttaa cactaacgct tactacgaaa tgtctggtct ggaagtttct 780
   tttgaagaac tgcgtacttt cggtggtcac gacgctaaat tcatcgactc tctgcaggaa 840
   aacgagttcc gtctgtacta ctactacaaa ttcaaagaca tcgcttctac tctgaacaaa 900
   gctaaatcta tcgttggtac cactgcttct ctgcagtaca tgaagaacgt tttcaaagaa 960
   aagtacctgc tgtctgaaga cacttctggt aaattctctg ttgacaaact gaaattcgac 1020
   aaactgtaca aaatgctgac tgaaatctac actgaagaca acttcgttaa attcttcaaa 1080
   gttctgaacc gtaaaactta cctgaacttc gacaaagctg ttttcaaaat caacatcgtt 1140
   ccgaaagtta actacactat ctacgacggt ttcaacctgc gtaacactaa cctggctgct 1200
   aacttcaacg gtcagaacac tgaaatcaac aacatgaact tcactaaact gaagaacttc 1260
   actggtctgt ttgagttcta caaactgctg tgcgttcgtg gtatcatcac ttctaaaact 1320
   aaatctctgg acaaaggtta caacaaagct ctgaacgacc tgtgcatcaa agttaacaac 1380
   tgggacctgt tcttctcccc gtctgaagac aacttcacta acgacctgaa caaaggcgaa 1440
   gaaatcacct ccgacactaa catcgaagct gctgaagaaa acatctctct ggacctgatc 1500
   cagcagtact acctgacttt caacttcgac aacgaaccgg aaaacatctc catcgaaaac 1560
   ctgtcttccg acatcatcgg tcagctggaa ctgatgccga acatcgaacg cttcccgaac 1620
   ggcaagaaat acgaactgga caaatacacc atgttccact acctgcgtgc tcaggaattc 1680
   gaacacggta aatctcgtat cgctctgact aactccgtta acgaagctct gctgaacccg 1740
   tctcgcgttt acaccttctt ctcttccgac tacgttaaga aagttaacaa agctactgaa 1800
   gctgctatgt tcctgggttg ggttgaacag ctggtttacg acttcaccga cgaaacttct 1860
   gaagtttcca ccactgacaa aatcgctgac atcactatca tcatcccgta catcggcccg 1920
   gctctgaaca tcggtaacat gctgtacaaa gacgacttcg ttggtgctct gatcttctct 1980
   ggcgctgtta tcctgctgga attcatcccg gaaatcgcta tcccggttct gggtaccttc 2040
   gctctggttt cctacatcgc taacaaagtt ctgactgttc agaccatcga caacgctctg 2100
   tctaaacgta acgaaaaatg ggacgaagtt tacaaataca tcgttactaa ctggctggct 2160
   aaagttaaca ctcagatcga cctgatccgt aagaagatga aagaagctct ggaaaaccag 2220
   gctgaagcta ctaaagctat catcaactac cagtacaacc agtacaccga agaagaaaag 2280
   aacaacatca acttcaacat cgatgacctg tcctctaaac tgaacgaatc catcaacaaa 2340
   gctatgatca acatcaacaa attcctgaac cagtgctctg tttcctacct gatgaactct 2400
   atgatcccgt acggcgttaa acgcctggaa gacttcgacg cttccctgaa agacgctctg 2460
   ctgaaataca tccgtgacaa ctacggtact ctgatcggcc aggttgaccg tctgaaagac 2520
   aaggttaaca acaccctgtc tactgacatc ccgttccagc tgtccaaata cgttgacaac 2580
   cag                                                               2583
<210> 21
   <211> 861
   <212> PRT
   <213> Artificial Sequence
<220>
   <223> Recombinant protein encoded by SEQ ID NO:20
<400> 21
   Met Val Gln Phe Val Asn Lys Gln Phe Asn Tyr Lys Asp Pro Val Asn
   1               5                   10                  15
   Gly Val Asp Ile Ala Tyr Ile Lys Ile Pro Asn Val Gly Gln Met Gln
               20                  25                  30
   Pro Val Lys Ala Phe Lys Ile His Asn Lys Ile Trp Val Ile Pro Glu
           35                  40                  45
   Arg Asp Thr Phe Thr Asn Pro Glu Glu Gly Asp Leu Asn Pro Pro Pro
       50                  55                  60
   Glu Ala Lys Gln Val Pro Val Ser Tyr Tyr Asp Ser Thr Tyr Leu Ser
   65                  70                  75                  80
   Thr Asp Asn Glu Lys Asp Asn Tyr Leu Lys Gly Val Thr Lys Leu Phe
                   85                  90                  95
   Glu Arg Ile Tyr Ser Thr Asp Leu Gly Arg Met Leu Leu Thr Ser Ile
               100                 105                 110
   Val Arg Gly Ile Pro Phe Trp Gly Gly Ser Thr Ile Asp Thr Glu Leu
           115                 120                 125
   Lys Val Ile Asp Thr Asn Cys Ile Asn Val Ile Gln Pro Asp Gly Ser
       130                 135                 140
   Tyr Arg Ser Glu Glu Leu Asn Leu Val Ile Ile Gly Pro Ser Ala Asp
   145                 150                 155                 160
   Ile Ile Gln Phe Glu Cys Lys Ser Phe Gly His Glu Val Leu Asn Leu
                   165                 170                 175
   Thr Arg Asn Gly Tyr Gly Ser Thr Gln Tyr Ile Arg Phe Ser Pro Asp
               180                 185                 190
   Phe Thr Phe Gly Phe Glu Glu Ser Leu Glu Val Asp Thr Asn Pro Leu
           195                 200                 205
   Leu Gly Ala Gly Lys Phe Ala Thr Asp Pro Ala Val Thr Leu Ala His
       210                 215                 220
   Glu Leu Ile His Ala Gly His Arg Leu Tyr Gly Ile Ala Ile Asn Pro
   225                 230                 235                 240
   Asn Arg Val Phe Lys Val Asn Thr Asn Ala Tyr Tyr Glu Met Ser Gly
                   245                 250                 255
   Leu Glu Val Ser Phe Glu Glu Leu Arg Thr Phe Gly Gly His Asp Ala
               260                 265                 270
   Lys Phe Ile Asp Ser Leu Gln Glu Asn Glu Phe Arg Leu Tyr Tyr Tyr
           275                 280                 285
   Tyr Lys Phe Lys Asp Ile Ala Ser Thr Leu Asn Lys Ala Lys Ser Ile
       290                 295                 300
   Val Gly Thr Thr Ala Ser Leu Gln Tyr Met Lys Asn Val Phe Lys Glu
   305                 310                 315                 320
   Lys Tyr Leu Leu Ser Glu Asp Thr Ser Gly Lys Phe Ser Val Asp Lys
                   325                 330                 335
   Leu Lys Phe Asp Lys Leu Tyr Lys Met Leu Thr Glu Ile Tyr Thr Glu
               340                 345                 350
   Asp Asn Phe Val Lys Phe Phe Lys Val Leu Asn Arg Lys Thr Tyr Leu
           355                 360                 365
   Asn Phe Asp Lys Ala Val Phe Lys Ile Asn Ile Val Pro Lys Val Asn
       370                 375                 380
   Tyr Thr Ile Tyr Asp Gly Phe Asn Leu Arg Asn Thr Asn Leu Ala Ala
   385                 390                 395                 400
   Asn Phe Asn Gly Gln Asn Thr Glu Ile Asn Asn Met Asn Phe Thr Lys
                   405                 410                 415
   Leu Lys Asn Phe Thr Gly Leu Phe Glu Phe Tyr Lys Leu Leu Cys Val
               420                 425                 430
   Arg Gly Ile Ile Thr Ser Lys Thr Lys Ser Leu Asp Lys Gly Tyr Asn
           435                 440                 445
   Lys Ala Leu Asn Asp Leu Cys Ile Lys Val Asn Asn Trp Asp Leu Phe
       450                 455                 460
   Phe Ser Pro Ser Glu Asp Asn Phe Thr Asn Asp Leu Asn Lys Gly Glu
   465                 470                 475                 480
   Glu Ile Thr Ser Asp Thr Asn Ile Glu Ala Ala Glu Glu Asn Ile Ser
                   485                 490                 495
   Leu Asp Leu Ile Gln Gln Tyr Tyr Leu Thr Phe Asn Phe Asp Asn Glu
               500                 505                 510
   Pro Glu Asn Ile Ser Ile Glu Asn Leu Ser Ser Asp Ile Ile Gly Gln
           515                 520                 525
   Leu Glu Leu Met Pro Asn Ile Glu Arg Phe Pro Asn Gly Lys Lys Tyr
       530                 535                 540
   Glu Leu Asp Lys Tyr Thr Met Phe His Tyr Leu Arg Ala Gln Glu Phe
   545                 550                 555                 560
   Glu His Gly Lys Ser Arg Ile Ala Leu Thr Asn Ser Val Asn Glu Ala
                   565                 570                 575
   Leu Leu Asn Pro Ser Arg Val Tyr Thr Phe Phe Ser Ser Asp Tyr Val
               580                 585                 590
   Lys Lys Val Asn Lys Ala Thr Glu Ala Ala Met Phe Leu Gly Trp Val
           595                 600                 605
   Glu Gln Leu Val Tyr Asp Phe Thr Asp Glu Thr Ser Glu Val Ser Thr
       610                 615                 620
   Thr Asp Lys Ile Ala Asp Ile Thr Ile Ile Ile Pro Tyr Ile Gly Pro
   625                 630                 635                 640
   Ala Leu Asn Ile Gly Asn Met Leu Tyr Lys Asp Asp Phe Val Gly Ala
                   645                 650                 655
   Leu Ile Phe Ser Gly Ala Val Ile Leu Leu Glu Phe Ile Pro Glu Ile
               660                 665                 670
   Ala Ile Pro Val Leu Gly Thr Phe Ala Leu Val Ser Tyr Ile Ala Asn
           675                 680                 685
   Lys Val Leu Thr Val Gln Thr Ile Asp Asn Ala Leu Ser Lys Arg Asn
       690                 695                 700
   Glu Lys Trp Asp Glu Val Tyr Lys Tyr Ile Val Thr Asn Trp Leu Ala
   705                 710                 715                 720
   Lys Val Asn Thr Gln Ile Asp Leu Ile Arg Lys Lys Met Lys Glu Ala
                   725                 730                 735
   Leu Glu Asn Gln Ala Glu Ala Thr Lys Ala Ile Ile Asn Tyr Gln Tyr
               740                 745                 750
   Asn Gln Tyr Thr Glu Glu Glu Lys Asn Asn Ile Asn Phe Asn Ile Asp
           755                 760                 765
   Asp Leu Ser Ser Lys Leu Asn Glu Ser Ile Asn Lys Ala Met Ile Asn
       770                 775                 780
   Ile Asn Lys Phe Leu Asn Gln Cys Ser Val Ser Tyr Leu Met Asn Ser
   785                 790                 795                 800
   Met Ile Pro Tyr Gly Val Lys Arg Leu Glu Asp Phe Asp Ala Ser Leu
                   805                 810                 815
   Lys Asp Ala Leu Leu Lys Tyr Ile Arg Asp Asn Tyr Gly Thr Leu Ile
               820                 825                 830
   Gly Gln Val Asp Arg Leu Lys Asp Lys Val Asn Asn Thr Leu Ser Thr
           835                 840                 845
   Asp Ile Pro Phe Gln Leu Ser Lys Tyr Val Asp Asn Gln
       850                 855                 860
<210> 22
   <211> 1329
   <212> DNA
   <213> Artificial Sequence
<220>
   <223> Synthetic polynucleotide sequence for the light chain of C. botulinum Type B, optimized for expression in *E*. *coli.*
<400> 22
   atgccagtta ccatcaacaa cttcaactac aacgacccaa tcgacaacaa caacatcatt 60
   atgatggagc caccattcgc tagaggtacc ggtagatact acaaggcttt caagatcacc 120
   gacagaattt ggattattcc agagagatac actttcggtt acaagccaga ggacttcaac 180
   aagtcttctg gtattttcaa cagagacgtc tgcgagtact acgacccaga ctacctgaac 240
   accaacgaca agaagaacat cttcctgcag accatgatca agctgttcaa cagaatcaag 300
   tccaagccat tgggtgagaa gctgctggag atgatcatta acggtatccc atacctgggt 360
   gacagaagag tcccactgga ggagttcaac accaacatcg cctccgtcac cgtcaacaag 420
   ctgatctcca acccgggtga ggtcgagcgt aagaagggca tcttcgccaa cctgatcatc 480
   ttcggcccag gtccagtctt gaacgagaac gagactattg acattggcat tcaaaaccac 540
   ttcgcctcca gagagggttt cggcggtatc atgcaaatga agttctgtcc agagtacgtc 600
   tccgttttca acaacgtcca agagaacaag ggtgcctcca tcttcaacag aagaggctac 660
   ttctccgacc cagccttgat cttgatgcac gagttgatcc acgtcttgca cggtttgtac 720
   ggtatcaagg tcgacgactt gccaattgtc ccaaacgaga agaagttctt catgcagtcc 780
   accgacgcca tccaggccga ggagctgtac accttcggtg gtcaggaccc atccatcatt 840
   accccatcca ccgacaagtc catctacgac aaggtcttgc agaacttcag aggtatcgtc 900
   gatagactga acaaggtctt ggtctgcatc tccgacccaa acatcaacat caacatttac 960
   aagaacaagt tcaaggacaa gtacaagttc gtcgaggact ccgagggtaa gtactccatc 1020
   gacgtcgagt ccttcgacaa gctgtacaag tccctgatgt tcggtttcac cgagaccaac 1080
   atcgccgaga actacaagat caagaccaga gcctcctact tctccgactc cctgccacca 1140
   gtcaagatca agaacttgtt ggacaacgaa atctacacta ttgaggaggg tttcaacatt 1200
   tccgacaagg acatggagaa ggagtacaga ggtcaaaaca aggctattaa caagcaagct 1260
   tacgaggaga tttctaagga gcacttggct gtttacaaga ttcaaatgtg taagtctgtt 1320
   aagtaatag                                                         1329
<210> 23
   <211> 441
   <212> PRT
   <213> Artificial Sequence
<220>
   <223> Recombinant protein encoded by SEQ ID NO:22
<400> 23
   Met Pro Val Thr Ile Asn Asn Phe Asn Tyr Asn Asp Pro Ile Asp Asn
    1               5                  10                  15
   Asn Asn Ile Ile Met Met Glu Pro Pro Phe Ala Arg Gly Thr Gly Arg
               20                  25                  30
   Tyr Tyr Lys Ala Phe Lys Ile Thr Asp Arg Ile Trp Ile Ile Pro Glu
           35                  40                  45
   Arg Tyr Thr Phe Gly Tyr Lys Pro Glu Asp Phe Asn Lys Ser Ser Gly
       50                  55                  60
   Ile Phe Asn Arg Asp Val Cys Glu Tyr Tyr Asp Pro Asp Tyr Leu Asn
   65                  70                  75                  80
   Thr Asn Asp Lys Lys Asn Ile Phe Leu Gln Thr Met Ile Lys Leu Phe
   85 90 95
   Asn Arg Ile Lys Ser Lys Pro Leu Gly Glu Lys Leu Leu Glu Met Ile
   100 105 110
   Ile Asn Gly Ile Pro Tyr Leu Gly Asp Arg Arg Val Pro Leu Glu Glu
   115 120 125
   Phe Asn Thr Asn Ile Ala Ser Val Thr Val Asn Lys Leu Ile Ser Asn
   130 135 140
   Pro Gly Glu Val Glu Arg Lys Lys Gly Ile Phe Ala Asn Leu Ile Ile
   145 150 155 160
   Phe Gly Pro Gly Pro Val Leu Asn Glu Asn Glu Thr Ile Asp Ile Gly
   165 170 175
   Ile Gln Asn His Phe Ala Ser Arg Glu Gly Phe Gly Gly Ile Met Gln
   180 185 190
   Met Lys Phe Cys Pro Glu Tyr Val Ser Val Phe Asn Asn Val Gln Glu
   195 200 205
   Asn Lys Gly Ala Ser Ile Phe Asn Arg Arg Gly Tyr Phe Ser Asp Pro
   210 215 220
   Ala Leu Ile Leu Met His Glu Leu Ile His Val Leu His Gly Leu Tyr
   225 230 235 240
   Gly Ile Lys Val Asp Asp Leu Pro Ile Val Pro Asn Glu Lys Lys Phe
   245 250 255
   Phe Met Gln Ser Thr Asp Ala Ile Gln Ala Glu Glu Leu Tyr Thr Phe
   260 265 270
   Gly Gly Gln Asp Pro Ser Ile Ile Thr Pro Ser Thr Asp Lys Ser Ile
   275 280 285
   Tyr Asp Lys Val Leu Gln Asn Phe Arg Gly Ile Val Asp Arg Leu Asn
   290 295 300
   Lys Val Leu Val Cys Ile Ser Asp Pro Asn Ile Asn Ile Asn Ile Tyr
   305 310 315 320
   Lys Asn Lys Phe Lys Asp Lys Tyr Lys Phe Val Glu Asp Ser Glu Gly
   325 330 335
   Lys Tyr Ser Ile Asp Val Glu Ser Phe Asp Lys Leu Tyr Lys Ser Leu
   340 345 350
   Met Phe Gly Phe Thr Glu Thr Asn Ile Ala Glu Asn Tyr Lys Ile Lys
   355 360 365
   Thr Arg Ala Ser Tyr Phe Ser Asp Ser Leu Pro Pro Val Lys Ile Lys
   370 375 380
   Asn Leu Leu Asp Asn Glu Ile Tyr Thr Ile Glu Glu Gly Phe Asn Ile
   385 390 395 400
   Ser Asp Lys Asp Met Glu Lys Glu Tyr Arg Gly Gln Asn Lys Ala Ile
   405 410 415
   Asn Lys Gln Ala Tyr Glu Glu Ile Ser Lys Glu His Leu Ala Val Tyr
   420 425 430
   Lys Ile Gln Met Cys Lys Ser Val Lys
   435 440
<210> 24
   <211> 2559
   <212> DNA
   <213> Artificial Sequence
<220>
   <223> Synthetic polynucleotide sequence for the light chain with Hn segment of of C. botulinum Type B, optimized for expression in *E*. *coli.*
<400> 24
   atgccagtta ccatcaacaa cttcaactac aacgacccaa tcgacaacaa caacatcatt 60
   atgatggagc caccattcgc tagaggtacc ggtagatact acaaggcttt caagatcacc 120
   gacagaattt ggattattcc agagagatac actttcggtt acaagccaga ggacttcaac 180
   aagtcttctg gtattttcaa cagagacgtc tgcgagtact acgacccaga ctacctgaac 240
   accaacgaca agaagaacat cttcctgcag accatgatca agctgttcaa cagaatcaag 300
   tccaagccat tgggtgagaa gctgctggag atgatcatta acggtatccc atacctgggt 360
   gacagaagag tcccactgga ggagttcaac accaacatcg cctccgtcac cgtcaacaag 420
   ctgatctcca acccgggtga ggtcgagcgt aagaagggca tcttcgccaa cctgatcatc 480
   ttcggcccag gtccagtctt gaacgagaac gagactattg acattggcat tcaaaaccac 540
   ttcgcctcca gagagggttt cggcggtatc atgcaaatga agttctgtcc agagtacgtc 600
   tccgttttca acaacgtcca agagaacaag ggtgcctcca tcttcaacag aagaggctac 660
   ttctccgacc cagccttgat cttgatgcac gagttgatcc acgtcttgca cggtttgtac 720
   ggtatcaagg tcgacgactt gccaattgtc ccaaacgaga agaagttctt catgcagtcc 780
   accgacgcca tccaggccga ggagctgtac accttcggtg gtcaggaccc atccatcatt 840
   accccatcca ccgacaagtc catctacgac aaggtcttgc agaacttcag aggtatcgtc 900
   gatagactga acaaggtctt ggtctgcatc tccgacccaa acatcaacat caacatttac 960
   aagaacaagt tcaaggacaa gtacaagttc gtcgaggact ccgagggtaa gtactccatc 1020
   gacgtcgagt ccttcgacaa gctgtacaag tccctgatgt tcggtttcac cgagaccaac 1080
   atcgccgaga actacaagat caagaccaga gcctcctact tctccgactc cctgccacca 1140
   gtcaagatca agaacttgtt ggacaacgaa atctacacta ttgaggaggg tttcaacatt 1200
   tccgacaagg acatggagaa ggagtacaga ggtcaaaaca aggctattaa caagcaagct 1260
   tacgaggaga tttctaagga gcacttggct gtttacaaga ttcaaatgtg taagtctgtt 1320
   aaggctccag gaatctgtat cgacgtcgac aacgaggact tgttcttcat cgctgacaag 1380
   aactccttct ccgacgactt gtccaagaac gagagaatcg agtacaacac ccagtccaac 1440
   tacatcgaga acgacttccc aatcaacgag ttgatcttgg acaccgactt gatctccaag 1500
   atcgagttgc catccgagaa caccgagtcc ttgactgact tcaacgtcga cgtcccagtc 1560
   tacgagaagc aaccagctat caagaagatt ttcaccgacg agaacaccat cttccaatac 1620
   ctgtactctc agaccttccc tttggacatc agagacatct ccttgacctc ttccttcgac 1680
   gacgccctgc tgttctccaa caaggtctac tccttcttct ccatggacta catcaagact 1740
   gctaacaagg tcgtcgaggc cggtttgttc gctggttggg tcaagcagat cgtcaacgat 1800
   ttcgtcatcg aggctaacaa gtccaacacc atggacaaga ttgccgacat ctccttgatt 1860
   gtcccataca tcggtttggc cttgaacgtc ggtaacgaga ccgccaaggg taacttcgag 1920
   aacgctttcg agatcgctgg tgcctccatc ttgttggagt tcatcccaga gttgttgatc 1980
   ccagtcgtcg gtgccttctt gttggagtcc tacatcgaca acaagaacaa gatcatcaag 2040
   accatcgaca acgctttgac caagagaaac gagaagtggt ccgacatgta cggtttgatc 2100
   gtcgcccaat ggttgtccac cgtcaacacc caattctaca ccatcaagga gggtatgtac 2160
   aaggccttga actaccaggc ccaagctttg gaggagatca tcaagtacag atacaacatc 2220
   tactccgaga aggagaagtc caacattaac atcgacttca acgacatcaa ctccaagctg 2280
   aacgagggta ttaaccaggc catcgacaac atcaacaact tcatcaacgg ttgttccgtc 2340
   tcctacttga tgaagaagat gattccattg gccgtcgaga agttgttgga cttcgacaac 2400
   accctgaaga agaacttgtt gaactacatc gacgagaaca agttgtactt gatcggttcc 2460
   gctgagtacg agaagtccaa ggtcaacaag tacttgaaga ccatcatgcc attcgacttg 2520
   tccatctaca ccaacgacac catcttgatc gagatgttc                       2559
<210> 25
   <211> 852
   <212> PRT
   <213> Artificial Sequence
<220>
   <223> Recombinant protein encoded by SEQ ID NO:24
<400> 25
   Pro Val Thr Ile Asn Asn Phe Asn Tyr Asn Asp Pro Ile Asp Asn Asn
   1               5                   10                  15
   Asn Ile Ile Met Met Glu Pro Pro Phe Ala Arg Gly Thr Gly Arg Tyr
               20                  25                  30
   Tyr Lys Ala Phe Lys Ile Thr Asp Arg Ile Trp Ile Ile Pro Glu Arg
           35                  40                  45
   Tyr Thr Phe Gly Tyr Lys Pro Glu Asp Phe Asn Lys Ser Ser Gly Ile
       50                  55                  60
   Phe Asn Arg Asp Val Cys Glu Tyr Tyr Asp Pro Asp Tyr Leu Asn Thr
   65                  70                  75                  80
   Asn Asp Lys Lys Asn Ile Phe Leu Gln Thr Met Ile Lys Leu Phe Asn
                   85                  90                  95
   Arg Ile Lys Ser Lys Pro Leu Gly Glu Lys Leu Leu Glu Met Ile Ile
               100                 105                 110
   Asn Gly Ile Pro Tyr Leu Gly Asp Arg Arg Val Pro Leu Glu Glu Phe
           115                 120                 125
   Asn Thr Asn Ile Ala Ser Val Thr Val Asn Lys Leu Ile Ser Asn Pro
       130                 135                 140
   Gly Glu Val Glu Arg Lys Lys Gly Ile Phe Ala Asn Leu Ile Ile Phe
   145                 150                 155                 160
   Gly Pro Gly Pro Val Leu Asn Glu Asn Glu Thr Ile Asp Ile Gly Ile
                   165                 170                 175
   Gln Asn His Phe Ala Ser Arg Glu Gly Phe Gly Gly Ile Met Gln Met
               180                 185                 190
   Lys Phe Cys Pro Glu Tyr Val Ser Val Phe Asn Asn Val Gln Glu Asn
           195                 200                 205
   Lys Gly Ala Ser Ile Phe Asn Arg Arg Gly Tyr Phe Ser Asp Pro Ala
       210                 215                 220
   Leu Ile Leu Met His Glu Leu Ile His Val Leu His Gly Leu Tyr Gly
   225                 230                 235                 240
   Ile Lys Val Asp Asp Leu Pro Ile Val Pro Asn Glu Lys Lys Phe Phe
                   245                 250                 255
   Met Gln Ser Thr Asp Ala Ile Gln Ala Glu Glu Leu Tyr Thr Phe Gly
               260                 265                 270
   Gly Gln Asp Pro Ser Ile Ile Thr Pro Ser Thr Asp Lys Ser Ile Tyr
           275                 280                 285
   Asp Lys Val Leu Gln Asn Phe Arg Gly Ile Val Asp Arg Leu Asn Lys
       290                 295                 300
   Val Leu Val Cys Ile Ser Asp Pro Asn Ile Asn Ile Asn Ile Tyr Lys
   305                 310                 315                 320
   Asn Lys Phe Lys Asp Lys Tyr Lys Phe Val Glu Asp Ser Glu Gly Lys
                   325                 330                 335
   Tyr Ser Ile Asp Val Glu Ser Phe Asp Lys Leu Tyr Lys Ser Leu Met
               340                 345                 350
   Phe Gly Phe Thr Glu Thr Asn Ile Ala Glu Asn Tyr Lys Ile Lys Thr
           355                 360                 365
   Arg Ala Ser Tyr Phe Ser Asp Ser Leu Pro Pro Val Lys Ile Lys Asn
       370                 375                 380
   Leu Leu Asp Asn Glu Ile Tyr Thr Ile Glu Glu Gly Phe Asn Ile Ser
   385                 390                 395                 400
   Asp Lys Asp Met Glu Lys Glu Tyr Arg Gly Gln Asn Lys Ala Ile Asn
                   405                 410                 415
   Lys Gln Ala Tyr Glu Glu Ile Ser Lys Glu His Leu Ala Val Tyr Lys
               420                 425                 430
   Ile Gln Met Cys Lys Ser Val Lys Ala Pro Gly Ile Cys Ile Asp Val
           435                 440                 445
   Asp Asn Glu Asp Leu Phe Phe Ile Ala Asp Lys Asn Ser Phe Ser Asp
       450                 455                 460
   Asp Leu Ser Lys Asn Glu Arg Ile Glu Tyr Asn Thr Gln Ser Asn Tyr
   465                 470                 475                 480
   Ile Glu Asn Asp Phe Pro Ile Asn Glu Leu Ile Leu Asp Thr Asp Leu
                   485                 490                 495
   Ile Ser Lys Ile Glu Leu Pro Ser Glu Asn Thr Glu Ser Leu Thr Asp
               500                 505                 510
   Phe Asn Val Asp Val Pro Val Tyr Glu Lys Gln Pro Ala Ile Lys Lys
           515                 520                 525
   Ile Phe Thr Asp Glu Asn Thr Ile Phe Gln Tyr Leu Tyr Ser Gln Thr
       530                 535                 540
   Phe Pro Leu Asp Ile Arg Asp Ile Ser Leu Thr Ser Ser Phe Asp Asp
   545                 550                 555                 560
   Ala Leu Leu Phe Ser Asn Lys Val Tyr Ser Phe Phe Ser Met Asp Tyr
                   565                 570                 575
   Ile Lys Thr Ala Asn Lys Val Val Glu Ala Gly Leu Phe Ala Gly Trp
               580                 585                 590
   Val Lys Gln Ile Val Asn Asp Phe Val Ile Glu Ala Asn Lys Ser Asn
           595                 600                 605
   Thr Met Asp Lys Ile Ala Asp Ile Ser Leu Ile Val Pro Tyr Ile Gly
       610                 615                 620
   Leu Ala Leu Asn Val Gly Asn Glu Thr Ala Lys Gly Asn Phe Glu Asn
   625                 630                 635                 640
   Ala Phe Glu Ile Ala Gly Ala Ser Ile Leu Leu Glu Phe Ile Pro Glu
                   645                 650                 655
   Leu Leu Ile Pro Val Val Gly Ala Phe Leu Leu Glu Ser Tyr Ile Asp
               660                 665                 670
   Asn Lys Asn Lys Ile Ile Lys Thr Ile Asp Asn Ala Leu Thr Lys Arg
           675                 680                 685
   Asn Glu Lys Trp Ser Asp Met Tyr Gly Leu Ile Val Ala Gln Trp Leu
       690                 695                 700
   Ser Thr Val Asn Thr Gln Phe Tyr Thr Ile Lys Glu Gly Met Tyr Lys
   705                 710                 715                 720
   Ala Leu Asn Tyr Gln Ala Gln Ala Leu Glu Glu Ile Ile Lys Tyr Arg
                   725                 730                 735
   Tyr Asn Ile Tyr Ser Glu Lys Glu Lys Ser Asn Ile Asn Ile Asp Phe
               740                 745                 750
   Asn Asp Ile Asn Ser Lys Leu Asn Glu Gly Ile Asn Gln Ala Ile Asp
           755                 760                 765
   Asn Ile Asn Asn Phe Ile Asn Gly Cys Ser Val Ser Tyr Leu Met Lys
       770                 775                 780
   Lys Met Ile Pro Leu Ala Val Glu Lys Leu Leu Asp Phe Asp Asn Thr
   785                 790                 795                 800
   Leu Lys Lys Asn Leu Leu Asn Tyr Ile Asp Glu Asn Lys Leu Tyr Leu
                   805                 810                 815
   Ile Gly Ser Ala Glu Tyr Glu Lys Ser Lys Val Asn Lys Tyr Leu Lys
               820                 825                 830
   Thr Ile Met Pro Phe Asp Leu Ser Ile Tyr Thr Asn Asp Thr Ile Leu
           835                 840                 845
   Ile Glu Met Phe
       850
<210> 26
   <211> 1311
   <212> DNA
   <213> Artificial Sequence
<220>
   <223> Synthetic polynucleotide sequence for the light chain of of C. botulinum Type C, optimized for expression in *E*. *coli.*
<400> 26
   atgccaatca ccatcaacaa cttcaactac tcagaccctg tcgacaacaa gaacattctg 60
   tacctggaca ctcacctgaa caccctagct aacgagcctg agaaggcctt tcggatcacc 120
   ggaaacatct gggtcatccc tgatcgtttc tcccgtaact ccaaccccaa cctgaacaag 180
   cctcctcggg tcaccagccc taagagtggt tactacgacc ctaactacct gagtaccgac 240
   tctgacaagg acaccttcct gaaggagatc atcaagctgt tcaagcgtat caactcccgt 300
   gagatcggag aggagctcat ctacagactt tcgaccgata tccccttccc tggtaacaac 360
   aatactccaa tcaacacctt cgacttcgac gtcgacttca actccgtcga cgtcaagact 420
   cggcagggta acaactgggt taagactggt agcatcaacc cttccgtcat catcactgga 480
   cctcgtgaga acatcatcga cccagagact tccacgttca agctgactaa caacaccttc 540
   gcggctcaag aaggattcgg tgctctgtca atcatctcca tctcacctcg tttcatgctg 600
   acctactcga acgcaaccaa cgacgtcgga gagggtaggt tctctaagtc tgagttctgc 660
   atggacccaa tcctgatcct gatgcatgag ctgaaccatg caatgcacaa cctgtacgga 720
   atcgctatcc caaacgacca gaccatctcc tccgtgacct ccaacatctt ctactcccag 780
   tacaacgtga agctggagta cgcagagatc tacgctttcg gaggtccaac tatcgacctt 840
   atccctaagt ccgctaggaa gtacttcgag gagaaggctt tggattacta cagatccatc 900
   gctaagagac tgaacagtat caccaccgca aacccttcca gcttcaacaa gtacatcggt 960
   gagtacaagc agaagctgat cagaaagtac cgtttcgtcg tcgagtcttc aggtgaggtc 1020
   acagtaaacc gtaacaagtt cgtcgagctg tacaacgagc ttacccagat cttcacagag 1080
   ttcaactacg ctaagatcta caacgtccag aacaggaaga tctacctgtc caacgtgtac 1140
   actccggtga cggcgaacat cctggacgac aacgtctacg acatccagaa cggattcaac 1200
   atccctaagt ccaacctgaa cgtactattc atgggtcaaa acctgcctcg aaacccagca 1260
   ctgcgtaagg tcaaccctga gaacatgctg tacctgttca ccaagttctg c          1311
<210> 27
   <211> 436
   <212> PRT
   <213> Artificial Sequence
<220>
   <223> Recombinant protein encoded by SEQ ID NO:26
<400> 27
   Pro Ile Thr Ile Asn Asn Phe Asn Tyr Ser Asp Pro Val Asp Asn Lys
    1               5                  10                  15
   Asn Ile Leu Tyr Leu Asp Thr His Leu Asn Thr Leu Ala Asn Glu Pro
               20                  25                  30
   Glu Lys Ala Phe Arg Ile Thr Gly Asn Ile Trp Val Ile Pro Asp Arg
           35                  40                  45
   Phe Ser Arg Asn Ser Asn Pro Asn Leu Asn Lys Pro Pro Arg Val Thr
       50                  55                  60
   Ser Pro Lys Ser Gly Tyr Tyr Asp Pro Asn Tyr Leu Ser Thr Asp Ser
   65                  70                  75                  80
   Asp Lys Asp Thr Phe Leu Lys Glu Ile Ile Lys Leu Phe Lys Arg Ile
                   85                  90                  95
   Asn Ser Arg Glu Ile Gly Glu Glu Leu Ile Tyr Arg Leu Ser Thr Asp
               100                 105                 110
   Ile Pro Phe Pro Gly Asn Asn Asn Thr Pro Ile Asn Thr Phe Asp Phe
           115                 120                 125
   Asp Val Asp Phe Asn Ser Val Asp Val Lys Thr Arg Gln Gly Asn Asn
       130                 135                 140
   Trp Val Lys Thr Gly Ser Ile Asn Pro Ser Val Ile Ile Thr Gly Pro
   145                 150                 155                 160
   Arg Glu Asn Ile Ile Asp Pro Glu Thr Ser Thr Phe Lys Leu Thr Asn
                   165                 170                 175
   Asn Thr Phe Ala Ala Gln Glu Gly Phe Gly Ala Leu Ser Ile Ile Ser
               180                 185                 190
   Ile Ser Pro Arg Phe Met Leu Thr Tyr Ser Asn Ala Thr Asn Asp Val
           195                 200                 205
   Gly Glu Gly Arg Phe Ser Lys Ser Glu Phe Cys Met Asp Pro Ile Leu
       210                 215                 220
   Ile Leu Met His Glu Leu Asn His Ala Met His Asn Leu Tyr Gly Ile
   225                 230                 235                 240
   Ala Ile Pro Asn Asp Gln Thr Ile Ser Ser Val Thr Ser Asn Ile Phe
                   245                 250                 255
   Tyr Ser Gln Tyr Asn Val Lys Leu Glu Tyr Ala Glu Ile Tyr Ala Phe
               260                 265                 270
   Gly Gly Pro Thr Ile Asp Leu Ile Pro Lys Ser Ala Arg Lys Tyr Phe
           275                 280                 285
   Glu Glu Lys Ala Leu Asp Tyr Tyr Arg Ser Ile Ala Lys Arg Leu Asn
       290                 295                 300
   Ser Ile Thr Thr Ala Asn Pro Ser Ser Phe Asn Lys Tyr Ile Gly Glu
   305                 310                 315                 320
   Tyr Lys Gln Lys Leu Ile Arg Lys Tyr Arg Phe Val Val Glu Ser Ser
                   325                 330                 335
   Gly Glu Val Thr Val Asn Arg Asn Lys Phe Val Glu Leu Tyr Asn Glu
               340                 345                 350
   Leu Thr Gln Ile Phe Thr Glu Phe Asn Tyr Ala Lys Ile Tyr Asn Val
           355                 360                 365
   Gln Asn Arg Lys Ile Tyr Leu Ser Asn Val Tyr Thr Pro Val Thr Ala
       370                 375                 380
   Asn Ile Leu Asp Asp Asn Val Tyr Asp Ile Gln Asn Gly Phe Asn Ile
   385                 390                 395                 400
   Pro Lys Ser Asn Leu Asn Val Leu Phe Met Gly Gln Asn Leu Ser Arg
                   405                 410                 415
   Asn Pro Ala Leu Arg Lys Val Asn Pro Glu Asn Met Leu Tyr Leu Phe
               420                 425                 430
   Thr Lys Phe Cys
           435
<210> 28
   <211> 2436
   <212> DNA
   <213> Artificial Sequence
<220>
   <223> Synthetic polynucleotide sequence for the light chain with Hn segment of of C. botulinum Type C, optimized for expression in *E. coli.*
<400> 28
   atgccaatca ccatcaacaa cttcaactac tcagaccctg tcgacaacaa gaacattctg 60
   tacctggaca ctcacctgaa caccctagct aacgagcctg agaaggcctt tcggatcacc 120
   ggaaacatct gggtcatccc tgatcgtttc tcccgtaact ccaaccccaa cctgaacaag 180
   cctcctcggg tcaccagccc taagagtggt tactacgacc ctaactacct gagtaccgac 240
   tctgacaagg acaccttcct gaaggagatc atcaagctgt tcaagcgtat caactcccgt 300
   gagatcggag aggagctcat ctacagactt tcgaccgata tccccttccc tggtaacaac 360
   aatactccaa tcaacacctt cgacttcgac gtcgacttca actccgtcga cgtcaagact 420
   cggcagggta acaactgggt taagactggt agcatcaacc cttccgtcat catcactgga 480
   cctcgtgaga acatcatcga cccagagact tccacgttca agctgactaa caacaccttc 540
   gcggctcaag aaggattcgg tgctctgtca atcatctcca tctcacctcg tttcatgctg 600
   acctactcga acgcaaccaa cgacgtcgga gagggtaggt tctctaagtc tgagttctgc 660
   atggacccaa tcctgatcct gatgcatgag ctgaaccatg caatgcacaa cctgtacgga 720
   atcgctatcc caaacgacca gaccatctcc tccgtgacct ccaacatctt ctactcccag 780
   tacaacgtga agctggagta cgcagagatc tacgctttcg gaggtccaac tatcgacctt 840
   atccctaagt ccgctaggaa gtacttcgag gagaaggctt tggattacta cagatccatc 900
   gctaagagac tgaacagtat caccaccgca aacccttcca gcttcaacaa gtacatcggt 960
   gagtacaagc agaagctgat cagaaagtac cgtttcgtcg tcgagtcttc aggtgaggtc 1020
   acagtaaacc gtaacaagtt cgtcgagctg tacaacgagc ttacccagat cttcacagag 1080
   ttcaactacg ctaagatcta caacgtccag aacaggaaga tctacctgtc caacgtgtac 1140
   actccggtga cggcgaacat cctggacgac aacgtctacg acatccagaa cggattcaac 1200
   atccctaagt ccaacctgaa cgtactattc atgggtcaaa acctgtctcg aaacccagca 1260
   ctgcgtaagg tcaaccctga gaacatgctg tacctgttca ccaagttctg ctccctgtac 1320
   aacaagaccc ttgactgtag agagctgctg gtgaagaaca ctgacctgcc attcatcggt 1380
   gacatcagtg acgtgaagac tgacatcttc ctgcgtaagg acatcaacga ggagactgag 1440
   gtgatctact acccagacaa cgtgtcagta gaccaagtga tcctcagtaa gaacacctcc 1500
   gagcatggac aactagacct gctctaccct agtatcgaca gtgagagtga gatcctgcca 1560
   ggggagaatc aagtcttcta cgacaaccgt acccagaacg tggactacct gaactcctac 1620
   tactacctag agtctcagaa gctgagtgac aacgtggagg acttcacttt cacgcgttca 1680
   atcgaggagg ctctggacaa cagtgcaaag gtgtacactt acttccctac cctggctaac 1740
   aaggtgaatg ccggtgtgca aggtggtctg ttcctgatgt gggcaaacga cgtggttgag 1800
   gacttcacta ccaacatcct gcgtaaggac acactggaca agatctcaga tgtgtcagct 1860
   atcatcccct acatcggacc cgcactgaac atctccaact ctgtgcgtcg tggaaacttc 1920
   actgaggcat tcgcagtcac tggtgtcacc atcctgctgg aggcattccc tgagttcaca 1980
   atccctgctc tgggtgcatt cgtgatctac agtaaggtcc aggagcgaaa cgagatcatc 2040
   aagaccatcg acaactgtct ggagcagagg atcaagagat ggaaggactc ctacgagtgg 2100
   atgatgggaa cgtggttgtc caggatcatc acccagttca acaacatctc ctaccagatg 2160
   tacgactccc tgaactacca ggcaggtgca atcaaggcta agatcgacct ggagtacaag 2220
   aagtactccg gaagcgacaa ggagaacatc aagagccagg ttgagaacct gaagaacagt 2280
   ctggacgtca agatctcgga ggcaatgaac aacatcaaca agttcatccg agagtgctcc 2340
   gtcacctacc tgttcaagaa catgctgcct aaggtcatcg acgagctgaa cgagttcgac 2400
   cgaaacacca aggcaaagct gatcaacctg atcgac                           2436
<210> 29
   <211> 811
   <212> PRT
   <213> Artificial Sequence
<220>
   <223> Recombinant protein encoded by SEQ ID NO:28
<400> 29
   Pro Ile Thr Ile Asn Asn Phe Asn Tyr Ser Asp Pro Val Asp Asn Lys
    1              5                   10                  15
   Asn Ile Leu Tyr Leu Asp Thr His Leu Asn Thr Leu Ala Asn Glu Pro
               20                  25                  30
   Glu Lys Ala Phe Arg Ile Thr Gly Asn Ile Trp Val Ile Pro Asp Arg
           35                  40                  45
   Phe Ser Arg Asn Ser Asn Pro Asn Leu Asn Lys Pro Pro Arg Val Thr
       50                  55                  60
   Ser Pro Lys Ser Gly Tyr Tyr Asp Pro Asn Tyr Leu Ser Thr Asp Ser
   65                  70                  75                  80
   Asp Lys Asp Thr Phe Leu Lys Glu Ile Ile Lys Leu Phe Lys Arg Ile
                   85                  90                  95
   Asn Ser Arg Glu Ile Gly Glu Glu Leu Ile Tyr Arg Leu Ser Thr Asp
               100                 105                 110
   Ile Pro Phe Pro Gly Asn Asn Asn Thr Pro Ile Asn Thr Phe Asp Phe
           115                 120                 125
   Asp Val Asp Phe Asn Ser Val Asp Val Lys Thr Arg Gln Gly Asn Asn
       130                 135                 140
   Trp Val Lys Thr Gly Ser Ile Asn Pro Ser Val Ile Ile Thr Gly Pro
   145                 150                 155                 160
   Arg Glu Asn Ile Ile Asp Pro Glu Thr Ser Thr Phe Lys Leu Thr Asn
                   165                 170                 175
   Asn Thr Phe Ala Ala Gln Glu Gly Phe Gly Ala Leu Ser Ile Ile Ser
               180                 185                 190
   Ile Ser Pro Arg Phe Met Leu Thr Tyr Ser Asn Ala Thr Asn Asp Val
           195                 200                 205
   Gly Glu Gly Arg Phe Ser Lys Ser Glu Phe Cys Met Asp Pro Ile Leu
       210                 215                 220
   Ile Leu Met His Glu Leu Asn His Ala Met His Asn Leu Tyr Gly Ile
   225                 230                 235                 240
   Ala Ile Pro Asn Asp Gln Thr Ile Ser Ser Val Thr Ser Asn Ile Phe
                   245                 250                 255
   Tyr Ser Gln Tyr Asn Val Lys Leu Glu Tyr Ala Glu Ile Tyr Ala Phe
               260                 265                 270
   Gly Gly Pro Thr Ile Asp Leu Ile Pro Lys Ser Ala Arg Lys Tyr Phe
           275                 280                 285
   Glu Glu Lys Ala Leu Asp Tyr Tyr Arg Ser Ile Ala Lys Arg Leu Asn
       290                 295                 300
   Ser Ile Thr Thr Ala Asn Pro Ser Ser Phe Asn Lys Tyr Ile Gly Glu
   305                 310                 315                 320
   Tyr Lys Gln Lys Leu Ile Arg Lys Tyr Arg Phe Val Val Glu Ser Ser
                   325                 330                 335
   Gly Glu Val Thr Val Asn Arg Asn Lys Phe Val Glu Leu Tyr Asn Glu
               340                 345                 350
   Leu Thr Gln Ile Phe Thr Glu Phe Asn Tyr Ala Lys Ile Tyr Asn Val
           355                 360                 365
   Gln Asn Arg Lys Ile Tyr Leu Ser Asn Val Tyr Thr Pro Val Thr Ala
       370                 375                 380
   Asn Ile Leu Asp Asp Asn Val Tyr Asp Ile Gln Asn Gly Phe Asn Ile
   385                 390                 395                 400
   Pro Lys Ser Asn Leu Asn Val Leu Phe Met Gly Gln Asn Leu Ser Arg
                   405                 410                 415
   Asn Pro Ala Leu Arg Lys Val Asn Pro Glu Asn Met Leu Tyr Leu Phe
               420                 425                 430
   Thr Lys Phe Cys Ser Leu Tyr Asn Lys Thr Leu Asp Cys Arg Glu Leu
           435                 440                 445
   Leu Val Lys Asn Thr Asp Leu Pro Phe Ile Gly Asp Ile Ser Asp Val
       450                 455                 460
   Lys Thr Asp Ile Phe Leu Arg Lys Asp Ile Asn Glu Glu Thr Glu Val
   465                 470                 475                 480
   Ile Tyr Tyr Pro Asp Asn Val Ser Val Asp Gln Val Ile Leu Ser Lys
                       485                 490                 495
   Asn Thr Ser Glu His Gly Gln Leu Asp Leu Leu Tyr Pro Ser Ile Asp
                   500                 505                 510
   Ser Glu Ser Glu Ile Leu Pro Gly Glu Asn Gln Val Phe Tyr Asp Asn
               515                 520                 525
   Arg Thr Gln Asn Val Asp Tyr Leu Asn Ser Tyr Tyr Tyr Leu Glu Ser
           530                 535                 540
   Gln Lys Leu Ser Asp Asn Val Glu Asp Phe Thr Phe Thr Arg Ser Ile
   545                 550                 555                 560
   Glu Glu Ala Leu Asp Asn Ser Ala Lys Val Tyr Thr Tyr Phe Pro Thr
                   565                 570                 575
   Leu Ala Asn Lys Val Asn Ala Gly Val Gln Gly Gly Leu Phe Leu Met
               580                 585                 590
   Trp Ala Asn Asp Val Val Glu Asp Phe Thr Thr Asn Ile Leu Arg Lys
           595                 600                 605
   Asp Thr Leu Asp Lys Ile Ser Asp Val Ser Ala Ile Ile Pro Tyr Ile
       610                 615                 620
   Gly Pro Ala Leu Asn Ile Ser Asn Ser Val Arg Arg Gly Asn Phe Thr
   625                 630                 635                 640
   Glu Ala Phe Ala Val Thr Gly Val Thr Ile Leu Leu Glu Ala Phe Pro
                   645                 650                 655
   Glu Phe Thr Ile Pro Ala Leu Gly Ala Phe Val Ile Tyr Ser Lys Val
               660                 665                 670
   Gln Glu Arg Asn Glu Ile Ile Lys Thr Ile Asp Asn Cys Leu Glu Gln
           675                 680                 685
   Arg Ile Lys Arg Trp Lys Asp Ser Tyr Glu Trp Met Met Gly Thr Trp
       690                 695                 700
   Leu Ser Arg Ile Ile Thr Gln Phe Asn Asn Ile Ser Tyr Gln Met Tyr
   705                 710                 715                 720
   Asp Ser Leu Asn Tyr Gln Ala Gly Ala Ile Lys Ala Lys Ile Asp Leu
                   725                 730                 735
   Glu Tyr Lys Lys Tyr Ser Gly Ser Asp Lys Glu Asn Ile Lys Ser Gln
               740                 745                 750
   Val Glu Asn Leu Lys Asn Ser Leu Asp Val Lys Ile Ser Glu Ala Met
           755                 760                 765
   Asn Asn Ile Asn Lys Phe Ile Arg Glu Cys Ser Val Thr Tyr Leu Phe
       770                 775                 780
   Lys Asn Met Leu Pro Lys Val Ile Asp Glu Leu Asn Glu Phe Asp Arg
   785                 790                 795                 800
   Asn Thr Lys Ala Lys Leu Ile Asn Leu Ile Asp
                   805                 810
<210> 30
   <211> 1323
   <212> DNA
   <213> Artificial Sequence
<220>
   <223> Synthetic polynucleotide sequence for the light chain of of C. botulinum Type D, optimized for expression in *E. coli.*
<400> 30
   atgacctggc cagtcaagga cttcaactac tccgacccag tcaacgacaa cgacatcttg 60
   tacttgagaa tcccacaaaa caagttgatc accaccccag tcaaggcttt catgatcacc 120
   cagaacacct gggttatccc agagagattc tcctccgaca ccaacccatc cctgtccaag 180
   ccaccaagac caacctccaa gtaccagtct tactacgacc catcttactt gtctaccgac 240
   gagcaaaagg acaccttctt gaagggtatt atcaagctgt tcaagagaat caacgagaga 300
   gacatcggta agaagttgat caactacttg gtcgttggtt ccccattcat gggtgactcc 360
   tctaccccag aggacacctt cgacttcacc agacacacca ccaacattgc cgtcgagaag 420
   ttcgagaacg gttcctggaa ggtcaccaac atcatcaccc catctgtttt gatcttcggt 480
   ccattgccaa acatcttgga ctacaccgcc tccctgacct tgcaaggtca gcaatccaac 540
   ccatccttcg agggtttcgg taccctgtct attttgaagg tcgctccaga gttcttgttg 600
   accttctccg acgtcacctc caaccaatcc tccgccgtct tgggtaagtc catcttctgt 660
   atggacccag tcatcgcttt gatgcacgag ttgacccact ccctgcacca gttgtacggt 720
   attaacatcc catctgacaa gagaatcaga ccacaggtct ctgagggttt cttctcccaa 780
   gacggtccaa acgttcagtt cgaggagttg tacaccttcg gtggtttgga cgtcgagatt 840
   atccaaattg agagatccca attgagagag aaggctttgg gtcactacaa ggacatcgcc 900
   aagagactga acaacatcaa caagaccatt ccatcttcct ggatctccaa cattgacaag 960
   tacaagaaga ttttctccga gaagtacaac ttcgacaagg acaacaccgg taacttcgtc 1020
   gttaacatcg acaagttcaa ctctttgtac tccgacttga ccaacgttat gtctgaggtt 1080
   gtctactcct cccaatacaa cgtcaagaac agaacccact acttctccag acactacttg 1140
   ccagttttcg ctaacatctt ggacgacaac atttacacca tcagagacgg tttcaacttg 1200
   accaacaagg gtttcaacat cgagaactcc ggtcaaaaca tcgagagaaa cccagccctg 1260
   caaaagctgt cctccgagtc tgtcgtcgac ttgttcacca aggtctgttt gagattgacc 1320
   aag                                                               1323
<210> 31
   <211> 440
   <212> PRT
   <213> Artificial Sequence
<220>
   <223> Recombinant protein encoded by SEQ ID NO:30
<400> 31
   Thr Trp Pro Val Lys Asp Phe Asn Tyr Ser Asp Pro Val Asn Asp Asn
    1               5                  10                  15
   Asp Ile Leu Tyr Leu Arg Ile Pro Gln Asn Lys Leu Ile Thr Thr Pro
               20                  25                  30
   Val Lys Ala Phe Met Ile Thr Gln Asn Thr Trp Val Ile Pro Glu Arg
           35                  40                  45
   Phe Ser Ser Asp Thr Asn Pro Ser Leu Ser Lys Pro Pro Arg Pro Thr
       50                  55                  60
   Ser Lys Tyr Gln Ser Tyr Tyr Asp Pro Ser Tyr Leu Ser Thr Asp Glu
   65                  70                  75                  80
   Gln Lys Asp Thr Phe Leu Lys Gly Ile Ile Lys Leu Phe Lys Arg Ile
                   85                  90                  95
   Asn Glu Arg Asp Ile Gly Lys Lys Leu Ile Asn Tyr Leu Val Val Gly
               100                 105                 110
   Ser Pro Phe Met Gly Asp Ser Ser Thr Pro Glu Asp Thr Phe Asp Phe
           115                 120                 125
   Thr Arg His Thr Thr Asn Ile Ala Val Glu Lys Phe Glu Asn Gly Ser
       130                 135                 140
   Trp Lys Val Thr Asn Ile Ile Thr Pro Ser Val Leu Ile Phe Gly Pro
   145                 150                 155                 160
   Leu Pro Asn Ile Leu Asp Tyr Thr Ala Ser Leu Thr Leu Gln Gly Gln
                   165                 170                 175
   Gln Ser Asn Pro Ser Phe Glu Gly Phe Gly Thr Leu Ser Ile Leu Lys
               180                 185                 190
   Val Ala Pro Glu Phe Leu Leu Thr Phe Ser Asp Val Thr Ser Asn Gln
           195                 200                 205
   Ser Ser Ala Val Leu Gly Lys Ser Ile Phe Cys Met Asp Pro Val Ile
       210                 215                 220
   Ala Leu Met His Glu Leu Thr His Ser Leu His Gln Leu Tyr Gly Ile
   225                 230                 235                 240
   Asn Ile Pro Ser Asp Lys Arg Ile Arg Pro Gln Val Ser Glu Gly Phe
                   245                 250                 255
   Phe Ser Gln Asp Gly Pro Asn Val Gln Phe Glu Glu Leu Tyr Thr Phe
               260                 265                 270
   Gly Gly Leu Asp Val Glu Ile Ile Gln Ile Glu Arg Ser Gln Leu Arg
           275                 280                 285
   Glu Lys Ala Leu Gly His Tyr Lys Asp Ile Ala Lys Arg Leu Asn Asn
       290                 295                 300
   Ile Asn Lys Thr Ile Pro Ser Ser Trp Ile Ser Asn Ile Asp Lys Tyr
   305                 310                 315                 320
   Lys Lys Ile Phe Ser Glu Lys Tyr Asn Phe Asp Lys Asp Asn Thr Gly
                   325                 330                 335
   Asn Phe Val Val Asn Ile Asp Lys Phe Asn Ser Leu Tyr Ser Asp Leu
               340                 345                 350
   Thr Asn Val Met Ser Glu Val Val Tyr Ser Ser Gln Tyr Asn Val Lys
           355                 360                 365
   Asn Arg Thr His Tyr Phe Ser Arg His Tyr Leu Pro Val Phe Ala Asn
       370                 375                 380
   Ile Leu Asp Asp Asn Ile Tyr Thr Ile Arg Asp Gly Phe Asn Leu Thr
   385                 390                 395                 400
   Asn Lys Gly Phe Asn Ile Glu Asn Ser Gly Gln Asn Ile Glu Arg Asn
                   405                 410                 415
   Pro Ala Leu Gln Lys Leu Ser Ser Glu Ser Val Val Asp Leu Phe Thr
               420                 425                 430
   Lys Val Cys Leu Arg Leu Thr Lys
           435                 440
<210> 32
   <211> 2475
   <212> DNA
   <213> Artificial Sequence
<220>
   <223> Synthetic polynucleotide sequence for the light chain with Hn segment of of C. botulinum Type D, optimized for expression in *E*. *coli.*
<400> 32
   atgacctggc cagtcaagga cttcaactac tccgacccag tcaacgacaa cgacatcttg 60
   tacttgagaa tcccacaaaa caagttgatc accaccccag tcaaggcttt catgatcacc 120
   cagaacacct gggttatccc agagagattc tcctccgaca ccaacccatc cctgtccaag 180
   ccaccaagac caacctccaa gtaccagtct tactacgacc catcttactt gtctaccgac 240
   gagcaaaagg acaccttctt gaagggtatt atcaagctgt tcaagagaat caacgagaga 300
   gacatcggta agaagttgat caactacttg gtcgttggtt ccccattcat gggtgactcc 360
   tctaccccag aggacacctt cgacttcacc agacacacca ccaacattgc cgtcgagaag 420
   ttcgagaacg gttcctggaa ggtcaccaac atcatcaccc catctgtttt gatcttcggt 480
   ccattgccaa acatcttgga ctacaccgcc tccctgacct tgcaaggtca gcaatccaac 540
   ccatccttcg agggtttcgg taccctgtct attttgaagg tcgctccaga gttcttgttg 600
   accttctccg acgtcacctc caaccaatcc tccgccgtct tgggtaagtc catcttctgt 660
   atggacccag tcatcgcttt gatgcacgag ttgacccact ccctgcacca gttgtacggt 720
   attaacatcc catctgacaa gagaatcaga ccacaggtct ctgagggttt cttctcccaa 780
   gacggtccaa acgttcagtt cgaggagttg tacaccttcg gtggtttgga cgtcgagatt 840
   atccaaattg agagatccca attgagagag aaggctttgg gtcactacaa ggacatcgcc 900
   aagagactga acaacatcaa caagaccatt ccatcttcct ggatctccaa cattgacaag 960
   tacaagaaga ttttctccga gaagtacaac ttcgacaagg acaacaccgg taacttcgtc 1020
   gttaacatcg acaagttcaa ctctttgtac tccgacttga ccaacgttat gtctgaggtt 1080
   gtctactcct cccaatacaa cgtcaagaac agaacccact acttctccag acactacttg 1140
   ccagttttcg ctaacatctt ggacgacaac atttacacca tcagagacgg tttcaacttg 1200
   accaacaagg gtttcaacat cgagaactcc ggtcaaaaca tcgagagaaa cccagccctg 1260
   caaaagctgt cctccgagtc tgtcgtcgac ttgttcacca aggtctgttt gagattgacc 1320
   aagaactccc gtgacgactc cacctgcatc aaggtcaaga acaacagact gccatacgtt 1380
   gccgacaagg actccatctc ccaggagatc ttcgagaaca agatcatcac cgacgagacc 1440
   aacgttcaaa actactccga caagttctct ttggacgagt ccatcctgga cggtcaggtc 1500
   ccaatcaacc cagagatcgt cgacccactg ttgccaaacg tcaacatgga gccattgaac 1560
   ttgccaggtg aggagatcgt cttctacgac gacatcacca agtacgtcga ctacttgaac 1620
   tcctactact acttggagtc tcaaaagttg tctaacaacg tcgagaacat caccttgacc 1680
   acctccgtcg aggaggcctt gggttactct aacaagatct acaccttcct gccatccttg 1740
   gctgagaagg ttaacaaggg tgttcaagct ggtttgttcc tgaactgggc caacgaggtc 1800
   gtcgaggact tcaccaccaa catcatgaag aaggacaccc tggacaagat ctccgacgtc 1860
   tccgtcatca tcccatacat cggtccagcc ttgaacatcg gtaactccgc cctgagaggt 1920
   aacttcaacc aggccttcgc caccgccggt gtcgccttcc tgctggaggg tttcccagag 1980
   ttcaccatcc cagccctggg tgtcttcacc ttctactcct ccatccagga gagagagaag 2040
   atcatcaaga ccatcgagaa ctgcttggag cagagagtca agagatggaa ggactcctac 2100
   cagtggatgg tttccaactg gctgtccaga atcaccaccc aattcaacca catcaactac 2160
   cagatgtacg actccctgtc ctaccaggcc gacgccatca aggccaagat cgacctggag 2220
   tacaagaagt actccggttc cgacaaggag aacatcaagt cccaggtcga gaacctgaag 2280
   aactccttgg acgtcaagat ctccgaggcc atgaacaaca tcaacaagtt catccgtgag 2340
   tgttccgtca cctacctgtt caagaacatg ctgccaaagg tcatcgacga gctgaacaag 2400
   ttcgacctga gaaccaagac cgagctgatc aacctgatcg actcccacaa catcatcctg 2460
   gttggtgagg ttgac                                                  2475
<210> 33
   <211> 824
   <212> PRT
   <213> Artificial Sequence
<220>
   <223> Recombinant protein encoded by SEQ ID NO:32
<400> 33
   Thr Trp Pro Val Lys Asp Phe Asn Tyr Ser Asp Pro Val Asn Asp Asn
    1               5                  10                  15
   Asp Ile Leu Tyr Leu Arg Ile Pro Gln Asn Lys Leu Ile Thr Thr Pro
               20                  25                  30
   Val Lys Ala Phe Met Ile Thr Gln Asn Thr Trp Val Ile Pro Glu Arg
           35                  40                  45
   Phe Ser Ser Asp Thr Asn Pro Ser Leu Ser Lys Pro Pro Arg Pro Thr
       50                  55                  60
   Ser Lys Tyr Gln Ser Tyr Tyr Asp Pro Ser Tyr Leu Ser Thr Asp Glu
   65                  70                  75                  80
   Gln Lys Asp Thr Phe Leu Lys Gly Ile Ile Lys Leu Phe Lys Arg Ile
                   85                  90                  95
   Asn Glu Arg Asp Ile Gly Lys Lys Leu Ile Asn Tyr Leu Val Val Gly
               100                 105                 110
   Ser Pro Phe Met Gly Asp Ser Ser Thr Pro Glu Asp Thr Phe Asp Phe
           115                 120                 125
   Thr Arg His Thr Thr Asn Ile Ala Val Glu Lys Phe Glu Asn Gly Ser
       130                 135                 140
   Trp Lys Val Thr Asn Ile Ile Thr Pro Ser Val Leu Ile Phe Gly Pro
   145                 150                 155                 160
   Leu Pro Asn Ile Leu Asp Tyr Thr Ala Ser Leu Thr Leu Gln Gly Gln
                   165                 170                 175
   Gln Ser Asn Pro Ser Phe Glu Gly Phe Gly Thr Leu Ser Ile Leu Lys
               180                 185                 190
   Val Ala Pro Glu Phe Leu Leu Thr Phe Ser Asp Val Thr Ser Asn Gln
           195                 200                 205
   Ser Ser Ala Val Leu Gly Lys Ser Ile Phe Cys Met Asp Pro Val Ile
       210                 215                 220
   Ala Leu Met His Glu Leu Thr His Ser Leu His Gln Leu Tyr Gly Ile
   225                 230                 235                 240
   Asn Ile Pro Ser Asp Lys Arg Ile Arg Pro Gln Val Ser Glu Gly Phe
                   245                 250                 255
   Phe Ser Gln Asp Gly Pro Asn Val Gln Phe Glu Glu Leu Tyr Thr Phe
               260                 265                 270
   Gly Gly Leu Asp Val Glu Ile Ile Gln Ile Glu Arg Ser Gln Leu Arg
           275                 280                 285
   Glu Lys Ala Leu Gly His Tyr Lys Asp Ile Ala Lys Arg Leu Asn Asn
       290                 295                 300
   Ile Asn Lys Thr Ile Pro Ser Ser Trp Ile Ser Asn Ile Asp Lys Tyr
   305                 310                 315                 320
   Lys Lys Ile Phe Ser Glu Lys Tyr Asn Phe Asp Lys Asp Asn Thr Gly
                   325                 330                 335
   Asn Phe Val Val Asn Ile Asp Lys Phe Asn Ser Leu Tyr Ser Asp Leu
               340                 345                 350
   Thr Asn Val Met Ser Glu Val Val Tyr Ser Ser Gln Tyr Asn Val Lys
           355                 360                 365
   Asn Arg Thr His Tyr Phe Ser Arg His Tyr Leu Pro Val Phe Ala Asn
       370                 375                 380
   Ile Leu Asp Asp Asn Ile Tyr Thr Ile Arg Asp Gly Phe Asn Leu Thr
   385                 390                 395                 400
   Asn Lys Gly Phe Asn Ile Glu Asn Ser Gly Gln Asn Ile Glu Arg Asn
                   405                 410                 415
   Pro Ala Leu Gln Lys Leu Ser Ser Glu Ser Val Val Asp Leu Phe Thr
               420                 425                 430
   Lys Val Cys Leu Arg Leu Thr Lys Asn Ser Arg Asp Asp Ser Thr Cys
           435                 440                 445
   Ile Lys Val Lys Asn Asn Arg Leu Pro Tyr Val Ala Asp Lys Asp Ser
       450                 455                 460
   Ile Ser Gln Glu Ile Phe Glu Asn Lys Ile Ile Thr Asp Glu Thr Asn
   465                 470                 475                 480
   Val Gln Asn Tyr Ser Asp Lys Phe Ser Leu Asp Glu Ser Ile Leu Asp
                   485                 490                 495
   Gly Gln Val Pro Ile Asn Pro Glu Ile Val Asp Pro Leu Leu Pro Asn
               500                 505                 510
   Val Asn Met Glu Pro Leu Asn Leu Pro Gly Glu Glu Ile Val Phe Tyr
           515                 520                 525
   Asp Asp Ile Thr Lys Tyr Val Asp Tyr Leu Asn Ser Tyr Tyr Tyr Leu
       530                 535                 540
   Glu Ser Gln Lys Leu Ser Asn Asn Val Glu Asn Ile Thr Leu Thr Thr
   545                 550                 555                 560
   Ser Val Glu Glu Ala Leu Gly Tyr Ser Asn Lys Ile Tyr Thr Phe Leu
                   565                 570                 575
   Pro Ser Leu Ala Glu Lys Val Asn Lys Gly Val Gln Ala Gly Leu Phe
               580                 585                 590
   Leu Asn Trp Ala Asn Glu Val Val Glu Asp Phe Thr Thr Asn Ile Met
           595                 600                 605
   Lys Lys Asp Thr Leu Asp Lys Ile Ser Asp Val Ser Val Ile Ile Pro
       610                 615                 620
   Tyr Ile Gly Pro Ala Leu Asn Ile Gly Asn Ser Ala Leu Arg Gly Asn
   625                 630                 635                 640
   Phe Asn Gln Ala Phe Ala Thr Ala Gly Val Ala Phe Leu Leu Glu Gly
                   645                 650                 655
   Phe Pro Glu Phe Thr Ile Pro Ala Leu Gly Val Phe Thr Phe Tyr Ser
               660                 665                 670
   Ser Ile Gln Glu Arg Glu Lys Ile Ile Lys Thr Ile Glu Asn Cys Leu
           675                 680                 685
   Glu Gln Arg Val Lys Arg Trp Lys Asp Ser Tyr Gln Trp Met Val Ser
       690                 695                 700
   Asn Trp Leu Ser Arg Ile Thr Thr Gln Phe Asn His Ile Asn Tyr Gln
   705                 710                 715                 720
   Met Tyr Asp Ser Leu Ser Tyr Gln Ala Asp Ala Ile Lys Ala Lys Ile
                   725                 730                 735
   Asp Leu Glu Tyr Lys Lys Tyr Ser Gly Ser Asp Lys Glu Asn Ile Lys
               740                 745                 750
   Ser Gln Val Glu Asn Leu Lys Asn Ser Leu Asp Val Lys Ile Ser Glu
           755                 760                 765
   Ala Met Asn Asn Ile Asn Lys Phe Ile Arg Glu Cys Ser Val Thr Tyr
       770                 775                 780
   Leu Phe Lys Asn Met Leu Pro Lys Val Ile Asp Glu Leu Asn Lys Phe
   785                 790                 795                 800
   Asp Leu Arg Thr Lys Thr Glu Leu Ile Asn Leu Ile Asp Ser His Asn
                   805                 810                 815
   Ile Ile Leu Val Gly Glu Val Asp
               820
<210> 34
   <211> 1283
   <212> DNA
   <213> Artificial Sequence
<220>
   <223> Synthetic polynucleotide sequence for the light chain of of C. botulinum Type E, optimized for expression in *E*. *coli.*
<400> 34
   catatgccga aaatcaactc gttcaactac aacgacccgg tgaatgaccg cacaatcctg 60
   tacattaagc cgggcggttg ccaggagttc tacaagagct ttaacattat gaagaacatc 120
   tggatcatcc ctgaacgcaa tgtgatcggg acaacgccac aagatttcca ccctccgact 180
   tcgctcaaaa acggggactc ctcctactac gacccaaatt acttgcaaag cgatgaggag 240
   aaagatcggt tcctgaagat tgtgacaaag atcttcaacc gtattaacaa caatctctcg 300
   gggggcatcc tcctggagga attatccaag gcgaaccctt acctgggcaa cgacaacact 360
   ccagacaacc agttccacat tggcgacgcc tccgcggtgg agatcaagtt ctcgaatggc 420
   agtcaggaca tccttctccc taatgtcatt attatgggcg ccgagccgga cctttttgaa 480
   accaattcca gcaacatctc gctgcgcaac aactacatgc cgagcaatca cggctttggg 540
   tcgatcgcga tcgtgacttt ctcgccggag tactcctttc gcttcaacga caactccatg 600
   aacgagttca ttcaggaccc ggcgctcacc ctcatgcacg agctgatcca ctcgttacat 660
   ggcttgtacg gcgcgaaggg gatcacgacc aagtatacca ttacgcagaa acagaaccca 720
   cttatcacga acatccgtgg gacgaacatc gaggagttcc tcacgttcgg ggggaccgac 780
   ctgaacatta tcaccagcgc ccagtccaac gacatttaca cgaacctgct ggcagattac 840
   aaaaaaattg cctccaagct ctccaaggtc caggtatcga acccgttgct caatccttac 900
   aaggacgtct tcgaggctaa gtatgggctg gataaggatg cctcaggaat ctactctgtg 960
   aacatcaaca aattcaacga catcttcaag aagctgtaca gcttcaccga gtttgacctc 1020
   gccaccaagt tccaggtcaa atgtcggcaa acgtacattg gccagtataa atattttaag 1080
   ctgtcgaatc ttctcaacga ctctatctat aacatctccg aggggtacaa tattaacaac 1140
   ttaaaagtca acttccgagg gcagaacgca aatctcaacc cacggattat tactcctatt 1200
   acaggccgcg ggctcgtcaa gaagatcatc cgattttgca aaaacattgt cagcgttaaa 1260
   ggcatccgta agtaatagga tcc                                         1283
<210> 35
   <211> 427
   <212> PRT
   <213> Artificial Sequence
<220>
   <223> Recombinant protein encoded by SEQ ID NO:34
<221> UNSURE
   <222> (424)...(425)
   <223> Any amino acid at each position
<221> UNSURE
   <222> (427)...(427)
   <223> Any amino acid
<400> 35
   Met Pro Lys Ile Asn Ser Phe Asn Tyr Asn Asp Pro Val Asn Asp Arg
   1               5                   10                  15
   Thr Ile Leu Tyr Ile Lys Pro Gly Gly Cys Gln Glu Phe Tyr Lys Ser
               20                  25                  30
   Phe Asn Ile Met Lys Asn Ile Trp Ile Ile Pro Glu Arg Asn Val Ile
           35                  40                  45
   Gly Thr Thr Pro Gln Asp Phe His Pro Pro Thr Ser Leu Lys Asn Gly
       50                  55                  60
   Asp Ser Ser Tyr Tyr Asp Pro Asn Tyr Leu Gln Ser Asp Glu Glu Lys
   65                  70                  75                  80
   Asp Arg Phe Leu Lys Ile Val Thr Lys Ile Phe Asn Arg Ile Asn Asn
                   85                  90                  95
   Asn Leu Ser Gly Gly Ile Leu Leu Glu Glu Leu Ser Lys Ala Asn Pro
               100                 105                 110
   Tyr Leu Gly Asn Asp Asn Thr Pro Asp Asn Gln Phe His Ile Gly Asp
           115                 120                 125
   Ala Ser Ala Val Glu Ile Lys Phe Ser Asn Gly Ser Gln Asp Ile Leu
       130                 135                 140
   Leu Pro Asn Val Ile Ile Met Gly Ala Glu Pro Asp Leu Phe Glu Thr
   145                 150                 155                 160
   Asn Ser Ser Asn Ile Ser Leu Arg Asn Asn Tyr Met Pro Ser Asn His
                   165                 170                 175
   Gly Phe Gly Ser Ile Ala Ile Val Thr Phe Ser Pro Glu Tyr Ser Phe
               180                 185                 190
   Arg Phe Asn Asp Asn Ser Met Asn Glu Phe Ile Gln Asp Pro Ala Leu
           195                 200                 205
   Thr Leu Met His Glu Leu Ile His Ser Leu His Gly Leu Tyr Gly Ala
       210                 215                 220
   Lys Gly Ile Thr Thr Lys Tyr Thr Ile Thr Gln Lys Gln Asn Pro Leu
   225                 230                 235                 240
   Ile Thr Asn Ile Arg Gly Thr Asn Ile Glu Glu Phe Leu Thr Phe Gly
                   245                 250                 255
   Gly Thr Asp Leu Asn Ile Ile Thr Ser Ala Gln Ser Asn Asp Ile Tyr
               260                 265                 270
   Thr Asn Leu Leu Ala Asp Tyr Lys Lys Ile Ala Ser Lys Leu Ser Lys
           275                 280                 285
   Val Gln Val Ser Asn Pro Leu Leu Asn Pro Tyr Lys Asp Val Phe Glu
       290                 295                 300
   Ala Lys Tyr Gly Leu Asp Lys Asp Ala Ser Gly Ile Tyr Ser Val Asn
   305                 310                 315                 320
   Ile Asn Lys Phe Asn Asp Ile Phe Lys Lys Leu Tyr Ser Phe Thr Glu
                   325                 330                 335
   Phe Asp Leu Ala Thr Lys Phe Gln Val Lys Cys Arg Gln Thr Tyr Ile
               340                 345                 350
   Gly Gln Tyr Lys Tyr Phe Lys Leu Ser Asn Leu Leu Asn Asp Ser Ile
           355                 360                 365
   Tyr Asn Ile Ser Glu Gly Tyr Asn Ile Asn Asn Leu Lys Val Asn Phe
       370                 375                 380
   Arg Gly Gln Asn Ala Asn Leu Asn Pro Arg Ile Ile Thr Pro Ile Thr
   385                 390                 395                 400
   Gly Arg Gly Leu Val Lys Lys Ile Ile Arg Phe Cys Lys Asn Ile Val
                   405                 410                 415
   Ser Val Lys Gly Ile Arg Lys Xaa Xaa Asp Xaa
               420                 425
<210> 36
   <211> 2415
   <212> DNA
   <213> Artificial Sequence
<220>
   <223> Synthetic polynucleotide gene sequence for the light chain with Hn segment of of C. botulinum Type E, optimized for expression in *E. coli.*
<400> 36
   catatgccga aaatcaactc gttcaactac aacgacccgg tgaatgaccg cacaatcctg 60
   tacattaagc cgggcggttg ccaggagttc tacaagagct ttaacattat gaagaacatc 120
   tggatcatcc ctgaacgcaa tgtgatcggg acaacgccac aagatttcca ccctccgact 180
   tcgctcaaaa acggggactc ctcctactac gacccaaatt acttgcaaag cgatgaggag 240
   aaagatcggt tcctgaagat tgtgacaaag atcttcaacc gtattaacaa caatctctcg 300
   gggggcatcc tcctggagga attatccaag gcgaaccctt acctgggcaa cgacaacact 360
   ccagacaacc agttccacat tggcgacgcc tccgcggtgg agatcaagtt ctcgaatggc 420
   agtcaggaca tccttctccc taatgtcatt attatgggcg ccgagccgga cctttttgaa 480
   accaattcca gcaacatctc gctgcgcaac aactacatgc cgagcaatca cggctttggg 540
   tcgatcgcga tcgtgacttt ctcgccggag tactcctttc gcttcaacga caactccatg 600
   aacgagttca ttcaggaccc ggcgctcacc ctcatgcacg agctgatcca ctcgttacat 660
   ggcttgtacg gcgcgaaggg gatcacgacc aagtatacca ttacgcagaa acagaaccca 720
   cttatcacga acatccgtgg gacgaacatc gaggagttcc tcacgttcgg ggggaccgac 780
   ctgaacatta tcaccagcgc ccagtccaac gacatttaca cgaacctgct ggcagattac 840
   aaaaaaattg cctccaagct ctccaaggtc caggtatcga acccgttgct caatccttac 900
   aaggacgtct tcgaggctaa gtatgggctg gataaggatg cctcaggaat ctactctgtg 960
   aacatcaaca aattcaacga catcttcaag aagctgtaca gcttcaccga gtttgacctc 1020
   gccaccaagt tccaggtcaa atgtcggcaa acgtacattg gccagtataa atattttaag 1080
   ctgtcgaatc ttctcaacga ctctatctat aacatctccg aggggtacaa tattaacaac 1140
   ttaaaagtca acttccgagg gcagaacgca aatctcaacc cacggattat tactcctatt 1200
   acaggccgcg ggctcgtcaa gaagatcatc cgattttgca aaaacattgt cagcgttaaa 1260
   ggcatccgta agtccatctg catcgagatc aacaacggtg agctgttctt cgtggcttcc 1320
   gagaacagtt acaacgatga caacatcaac actcctaagg agattgacga caccgtcact 1380
   tctaacaaca actacgaaaa cgacctggac caggtcatcc taaacttcaa ctccgagtcc 1440
   gcccctggtc tgtccgacga gaagctgaac ctgaccatcc agaacgacgc ttacatccca 1500
   aagtacgact ccaacggtac atccgatatc gagcagcatg acgttaacga gcttaacgtc 1560
   ttcttctact tagacgctca gaaggtgccc gagggtgaga acaacgtcaa tctcacctct 1620
   tcaattgaca cagccttgtt ggagcagcct aagatctaca ccttcttctc ctccgagttc 1680
   atcaacaacg tcaacaagcc tgtgcaggcc gcattgttcg taagctggat tcagcaggtg 1740
   ttagtagact tcactactga ggctaaccag aagtccactg ttgacaagat cgctgacatc 1800
   tccatcgtcg tcccatacat cggtctggct ctgaacatcg gcaacgaggc acagaagggc 1860
   aacttcaagg atgcccttga gttgttgggt gccggtattt tgttggagtt cgaacccgag 1920
   ctgctgatcc ctaccatcct ggtcttcacg atcaagtcct tcctgggttc ctccgacaac 1980
   aagaacaagg tcattaaggc catcaacaac gccctgaagg agcgtgacga gaagtggaag 2040
   gaagtctatt ccttcatcgt ctcgaactgg atgaccaaga tcaacaccca gttcaacaag 2100
   cgaaaggagc agatgtacca ggctctgcag aaccaggtca acgccatcaa gaccatcatc 2160
   gagtccaagt acaactccta caccctggag gagaagaacg agcttaccaa caagtacgat 2220
   atcaagcaga tcgagaacga gctgaaccag aaggtctcca tcgccatgaa caacatcgac 2280
   aggttcctga ccgagtcctc catctcctac ctgatgaagc tcatcaacga ggtcaagatc 2340
   aacaagctgc gagagtacga cgagaatgtc aagacgtacc tgctgaacta catcatccag 2400
   cacggatcca tcctg                                                  2415
<210> 37
   <211> 804
   <212> PRT
   <213> Artificial Sequence
<220>
   <223> Recombinant protein encoded by SEQ ID NO:36
<400> 37
   Met Pro Lys Ile Asn Ser Phe Asn Tyr Asn Asp Pro Val Asn Asp Arg
    1               5                  10                  15
   Thr Ile Leu Tyr Ile Lys Pro Gly Gly Cys Gln Glu Phe Tyr Lys Ser
               20                  25                  30
   Phe Asn Ile Met Lys Asn Ile Trp Ile Ile Pro Glu Arg Asn Val Ile
           35                  40                  45
   Gly Thr Thr Pro Gln Asp Phe His Pro Pro Thr Ser Leu Lys Asn Gly
       50                  55                  60
   Asp Ser Ser Tyr Tyr Asp Pro Asn Tyr Leu Gln Ser Asp Glu Glu Lys
   65                  70                  75                  80
   Asp Arg Phe Leu Lys Ile Val Thr Lys Ile Phe Asn Arg Ile Asn Asn
                   85                  90                  95
   Asn Leu Ser Gly Gly Ile Leu Leu Glu Glu Leu Ser Lys Ala Asn Pro
               100                 105                 110
   Tyr Leu Gly Asn Asp Asn Thr Pro Asp Asn Gln Phe His Ile Gly Asp
           115                 120                 125
   Ala Ser Ala Val Glu Ile Lys Phe Ser Asn Gly Ser Gln Asp Ile Leu
       130                 135                 140
   Leu Pro Asn Val Ile Ile Met Gly Ala Glu Pro Asp Leu Phe Glu Thr
   145 150 155 160
   Asn Ser Ser Asn Ile Ser Leu Arg Asn Asn Tyr Met Pro Ser Asn His
   165 170 175
   Gly Phe Gly Ser Ile Ala Ile Val Thr Phe Ser Pro Glu Tyr Ser Phe
   180 185 190
   Arg Phe Asn Asp Asn Ser Met Asn Glu Phe Ile Gln Asp Pro Ala Leu
   195 200 205
   Thr Leu Met His Glu Leu Ile His Ser Leu His Gly Leu Tyr Gly Ala
   210 215 220
   Lys Gly Ile Thr Thr Lys Tyr Thr Ile Thr Gln Lys Gln Asn Pro Leu
   225 230 235 240
   Ile Thr Asn Ile Arg Gly Thr Asn Ile Glu Glu Phe Leu Thr Phe Gly
   245 250 255
   Gly Thr Asp Leu Asn Ile Ile Thr Ser Ala Gln Ser Asn Asp Ile Tyr
   260 265 270
   Thr Asn Leu Leu Ala Asp Tyr Lys Lys Ile Ala Ser Lys Leu Ser Lys
   275 280 285
   Val Gln Val Ser Asn Pro Leu Leu Asn Pro Tyr Lys Asp Val Phe Glu
   290 295 300
   Ala Lys Tyr Gly Leu Asp Lys Asp Ala Ser Gly Ile Tyr Ser Val Asn
   305 310 315 320
   Ile Asn Lys Phe Asn Asp Ile Phe Lys Lys Leu Tyr Ser Phe Thr Glu
   325 330 335
   Phe Asp Leu Ala Thr Lys Phe Gln Val Lys Cys Arg Gln Thr Tyr Ile
   340 345 350
   Gly Gln Tyr Lys Tyr Phe Lys Leu Ser Asn Leu Leu Asn Asp Ser Ile
   355 360 365
   Tyr Asn Ile Ser Glu Gly Tyr Asn Ile Asn Asn Leu Lys Val Asn Phe
   370 375 380
   Arg Gly Gln Asn Ala Asn Leu Asn Pro Arg Ile Ile Thr Pro Ile Thr
   385 390 395 400
   Gly Arg Gly Leu Val Lys Lys Ile Ile Arg Phe Cys Lys Asn Ile Val
   405 410 415
   Ser Val Lys Gly Ile Arg Lys Ser Ile Cys Ile Glu Ile Asn Asn Gly
   420 425 430
   Glu Leu Phe Phe Val Ala Ser Glu Asn Ser Tyr Asn Asp Asp Asn Ile
   435 440 445
   Asn Thr Pro Lys Glu Ile Asp Asp Thr Val Thr Ser Asn Asn Asn Tyr
   450 455 460
   Glu Asn Asp Leu Asp Gln Val Ile Leu Asn Phe Asn Ser Glu Ser Ala
   465 470 475 480
   Pro Gly Leu Ser Asp Glu Lys Leu Asn Leu Thr Ile Gln Asn Asp Ala
   485 490 495
   Tyr Ile Pro Lys Tyr Asp Ser Asn Gly Thr Ser Asp Ile Glu Gln His
   500 505 510
   Asp Val Asn Glu Leu Asn Val Phe Phe Tyr Leu Asp Ala Gln Lys Val
   515 520 525
   Pro Glu Gly Glu Asn Asn Val Asn Leu Thr Ser Ser Ile Asp Thr Ala
   530 535 540
   Leu Leu Glu Gln Pro Lys Ile Tyr Thr Phe Phe Ser Ser Glu Phe Ile
   545 550 555 560
   Asn Asn Val Asn Lys Pro Val Gln Ala Ala Leu Phe Val Ser Trp Ile
   565 570 575
   Gln Gln Val Leu Val Asp Phe Thr Thr Glu Ala Asn Gln Lys Ser Thr
   580 585 590
   Val Asp Lys Ile Ala Asp Ile Ser Ile Val Val Pro Tyr Ile Gly Leu
           595                 600                 605
   Ala Leu Asn Ile Gly Asn Glu Ala Gln Lys Gly Asn Phe Lys Asp Ala
       610                 615                 620
   Leu Glu Leu Leu Gly Ala Gly Ile Leu Leu Glu Phe Glu Pro Glu Leu
   625                 630                 635                 640
   Leu Ile Pro Thr Ile Leu Val Phe Thr Ile Lys Ser Phe Leu Gly Ser
                   645                 650                 655
   Ser Asp Asn Lys Asn Lys Val Ile Lys Ala Ile Asn Asn Ala Leu Lys
               660                 665                 670
   Glu Arg Asp Glu Lys Trp Lys Glu Val Tyr Ser Phe Ile Val Ser Asn
           675                 680                 685
   Trp Met Thr Lys Ile Asn Thr Gln Phe Asn Lys Arg Lys Glu Gln Met
       690                 695                 700
   Tyr Gln Ala Leu Gln Asn Gln Val Asn Ala Ile Lys Thr Ile Ile Glu
   705                 710                 715                 720
   Ser Lys Tyr Asn Ser Tyr Thr Leu Glu Glu Lys Asn Glu Leu Thr Asn
                   725                 730                 735
   Lys Tyr Asp Ile Lys Gln Ile Glu Asn Glu Leu Asn Gln Lys Val Ser
               740                 745                 750
   Ile Ala Met Asn Asn Ile Asp Arg Phe Leu Thr Glu Ser Ser Ile Ser
           755                 760                 765
   Tyr Leu Met Lys Leu Ile Asn Glu Val Lys Ile Asn Lys Leu Arg Glu
       770                 775                 780
   Tyr Asp Glu Asn Val Lys Thr Tyr Leu Leu Asn Tyr Ile Ile Gln His
   785                 790                 795                 800
   Gly Ser Ile Leu
<210> 38
   <211> 1334
   <212> DNA
   <213> Artificial Sequence
<220>
   <223> Synthetic polynucleotide sequence for the light chain of of C. botulinum Type F, optimized for expression in E. *coli.*
<400> 38
   catatgccgg ttgtcatcaa ttcttttaac tacaacgacc cggtgaacga cgacacgatt 60
   ctgtacatgc aaatccctta cgaggagaag tctaaaaagt attataaggc gttcgagatc 120
   atgcgcaacg tgtggatcat cccggaacgc aacactattg ggacagaccc gtcggacttc 180
   gatccgcctg cgtcgcttga aaacggctca tcagcatact atgacccaaa ttatttgact 240
   acggacgcgg aaaaggaccg ttatctcaag accacaatca agctcttcaa gcgtattaac 300
   tccaacccgg cgggcgaggt attgcttcag gagatttcct acgccaagcc ttacctcggc 360
   aatgagcata ctcctatcaa cgagttccac cctgtgaccc gaaccacgtc tgtaaacatt 420
   aagagttcga cgaatgtaaa gtcgtcaatt attctcaacc tcttggtcct tggcgcgggg 480
   ccggacatct tcgagaactc ttcctacccg gttcgcaagc tcatggacag tgggggggtc 540
   tatgacccga gcaacgacgg gttcggttcc atcaatatcg tgaccttctc acctgagtac 600
   gagtatacat ttaacgacat cagcggcggc tacaacagta gcaccgagtc ctttatcgcc 660
   gacccggcca tcagcctcgc tcacgagctc atccacgccc tgcacgggct gtacggggcc 720
   cggggcgtta catataagga gaccatcaaa gtgaagcagg cgccactcat gattgccgaa 780
   aagccaatcc gattggagga gttcctgaca ttcgggggcc aggacctgaa tattatcact 840
   agtgcaatga aggagaagat ttataacaac ctgctcgcga actatgagaa gatcgccact 900
   cgcttatccc gggtgaactc cgccccaccg gagtatgaca ttaacgagta taaagactac 960
   ttccagtgga agtatggact ggataaaaac gcggacgggt cttacaccgt gaacgagaac 1020
   aaattcaacg agatctacaa gaagctctac agcttcacgg agatcgacct cgcgaacaag 1080
   ttcaaggtga agtgccggaa cacgtatttc atcaagtacg gcttcttaaa ggtgccaaac 1140
   ctgttagacg acgacattta taccgtatcg gagggcttca atattggtaa tctggccgtg 1200
   aacaatcgcg gccagaatat taaacttaac ccgaaaatta tcgactcgat cccagacaag 1260
   gggttagttg agaagatcgt caagttctgc aagtcggtca tccctcgcaa ggggacgaag 1320
   aattaatagg atcc                                                   1334
<210> 39
   <211> 443
   <212> PRT
   <213> Artificial Sequence
<220>
   <223> Recombinant protein encoded by SEQ ID NO: 38
<221> UNSURE
   <222> (441) ... (442)
   <223> Any amino acid at each position
<400> 39
   Met Pro Val Val Ile Asn Ser Phe Asn Tyr Asn Asp Pro Val Asn Asp
    1               5                  10                  15
   Asp Thr Ile Leu Tyr Met Gln Ile Pro Tyr Glu Glu Lys Ser Lys Lys
               20                  25                  30
   Tyr Tyr Lys Ala Phe Glu Ile Met Arg Asn Val Trp Ile Ile Pro Glu
           35                  40                  45
   Arg Asn Thr Ile Gly Thr Asp Pro Ser Asp Phe Asp Pro Pro Ala Ser
       50                  55                  60
   Leu Glu Asn Gly Ser Ser Ala Tyr Tyr Asp Pro Asn Tyr Leu Thr Thr
   65                  70                  75                  80
   Asp Ala Glu Lys Asp Arg Tyr Leu Lys Thr Thr Ile Lys Leu Phe Lys
                   85                  90                  95
   Arg Ile Asn Ser Asn Pro Ala Gly Glu Val Leu Leu Gln Glu Ile Ser
               100                 105                 110
   Tyr Ala Lys Pro Tyr Leu Gly Asn Glu His Thr Pro Ile Asn Glu Phe
           115                 120                 125
   His Pro Val Thr Arg Thr Thr Ser Val Asn Ile Lys Ser Ser Thr Asn
       130                 135                 140
   Val Lys Ser Ser Ile Ile Leu Asn Leu Leu Val Leu Gly Ala Gly Pro
   145                 150                 155                 160
   Asp Ile Phe Glu Asn Ser Ser Tyr Pro Val Arg Lys Leu Met Asp Ser
                   165                 170                 175
   Gly Gly Val Tyr Asp Pro Ser Asn Asp Gly Phe Gly Ser Ile Asn Ile
               180                 185                 190
   Val Thr Phe Ser Pro Glu Tyr Glu Tyr Thr Phe Asn Asp Ile Ser Gly
           195                 200                 205
   Gly Tyr Asn Ser Ser Thr Glu Ser Phe Ile Ala Asp Pro Ala Ile Ser
       210                 215                 220
   Leu Ala His Glu Leu Ile His Ala Leu His Gly Leu Tyr Gly Ala Arg
   225                 230                 235                 240
   Gly Val Thr Tyr Lys Glu Thr Ile Lys Val Lys Gln Ala Pro Leu Met
                   245                 250                 255
   Ile Ala Glu Lys Pro Ile Arg Leu Glu Glu Phe Leu Thr Phe Gly Gly
               260                 265                 270
   Gln Asp Leu Asn Ile Ile Thr Ser Ala Met Lys Glu Lys Ile Tyr Asn
           275                 280                 285
   Asn Leu Leu Ala Asn Tyr Glu Lys Ile Ala Thr Arg Leu Ser Arg Val
       290                 295                 300
   Asn Ser Ala Pro Pro Glu Tyr Asp Ile Asn Glu Tyr Lys Asp Tyr Phe
   305                 310                 315                 320
   Gln Trp Lys Tyr Gly Leu Asp Lys Asn Ala Asp Gly Ser Tyr Thr Val
                   325                 330                 335
   Asn Glu Asn Lys Phe Asn Glu Ile Tyr Lys Lys Leu Tyr Ser Phe Thr
               340                 345                 350
   Glu Ile Asp Leu Ala Asn Lys Phe Lys Val Lys Cys Arg Asn Thr Tyr
           355                 360                 365
   Phe Ile Lys Tyr Gly Phe Leu Lys Val Pro Asn Leu Leu Asp Asp Asp
       370                 375                 380
   Ile Tyr Thr Val Ser Glu Gly Phe Asn Ile Gly Asn Leu Ala Val Asn
   385                 390                 395                 400
   Asn Arg Gly Gln Asn Ile Lys Leu Asn Pro Lys Ile Ile Asp Ser Ile
                   405                 410                 415
   Pro Asp Lys Gly Leu Val Glu Lys Ile Val Lys Phe Cys Lys Ser Val
               420                 425                 430
   Ile Pro Arg Lys Gly Thr Lys Asn Xaa Xaa Asp
           435                 440
<210> 40
   <211> 2577
   <212> DNA
   <213> Artificial Sequence
<220>
   <223> Synthetic polynucleotide sequence for the light chain with Hn segment of of C. botulinum Type F, optimized for expression in *E*. *coli.*
<400> 40
   catatgccgg ttgtcatcaa ttcttttaac tacaacgacc cggtgaacga cgacacgatt 60
   ctgtacatgc aaatccctta cgaggagaag tctaaaaagt attataaggc gttcgagatc 120
   atgcgcaacg tgtggatcat cccggaacgc aacactattg ggacagaccc gtcggacttc 180
   gatccgcctg cgtcgcttga aaacggctca tcagcatact atgacccaaa ttatttgact 240
   acggacgcgg aaaaggaccg ttatctcaag accacaatca agctcttcaa gcgtattaac 300
   tccaacccgg cgggcgaggt attgcttcag gagatttcct acgccaagcc ttacctcggc 360
   aatgagcata ctcctatcaa cgagttccac cctgtgaccc gaaccacgtc tgtaaacatt 420
   aagagttcga cgaatgtaaa gtcgtcaatt attctcaacc tcttggtcct tggcgcgggg 480
   ccggacatct tcgagaactc ttcctacccg gttcgcaagc tcatggacag tgggggggtc 540
   tatgacccga gcaacgacgg gttcggttcc atcaatatcg tgaccttctc acctgagtac 600
   gagtatacat ttaacgacat cagcggcggc tacaacagta gcaccgagtc ctttatcgcc 660
   gacccggcca tcagcctcgc tcacgagctc atccacgccc tgcacgggct gtacggggcc 720
   cggggcgtta catataagga gaccatcaaa gtgaagcagg cgccactcat gattgccgaa 780
   aagccaatcc gattggagga gttcctgaca ttcgggggcc aggacctgaa tattatcact 840
   agtgcaatga aggagaagat ttataacaac ctgctcgcga actatgagaa gatcgccact 900
   cgcttatccc gggtgaactc cgccccaccg gagtatgaca ttaacgagta taaagactac 960
   ttccagtgga agtatggact ggataaaaac gcggacgggt cttacaccgt gaacgagaac 1020
   aaattcaacg agatctacaa gaagctctac agcttcacgg agatcgacct cgcgaacaag 1080
   ttcaaggtga agtgccggaa cacgtatttc atcaagtacg gcttcttaaa ggtgccaaac 1140
   ctgttagacg acgacattta taccgtatcg gagggcttca atattggtaa tctggccgtg 1200
   aacaatcgcg gccagaatat taaacttaac ccgaaaatta tcgactcgat cccagacaag 1260
   gggttagttg agaagatcgt caagttctgc aagtcggtca tccctcgcaa ggggacgaag 1320
   aattgcaagt ccgtcatccc acgtaagggt accaaggccc caccacgtct gtgtattaga 1380
   gtcaacaact cagaattatt ctttgtcgct tccgagtcaa gctacaacga gaacgatatt 1440
   aacacaccta aagagattga cgatactacc aacctaaaca acaactaccg gaacaacttg 1500
   gatgaggtta ttttggatta caactcacag accatccctc aaatttccaa ccgtacctta 1560
   aacactcttg tccaagacaa ctcctacgtt ccaagatacg attctaacgg tacctcagag 1620
   atcgaggagt atgatgttgt tgactttaac gtctttttct atttgcatgc ccagaaggtg 1680
   ccagaaggtg aaaccaacat ctcattgact tcttccattg ataccgcctt gttggaagag 1740
   tccaaggata tcttcttttc ttcggagttt atcgatacta tcaacaagcc tgtcaacgcc 1800
   gctctgttca ttgattggat tagcaaggtc atcagagatt ttaccactga agctactcaa 1860
   aagtccactg ttgataagat tgctgacatc tctttgattg tcccctatgt cggtcttgct 1920
   ttgaacatca ttattgaggc agaaaagggt aactttgagg aggcttttga attgttggga 1980
   gttggtattt tgttggagtt tgttccagaa cttaccattc ctgtcatttt agtttttacg 2040
   atcaagtcct acatcgattc atacgagaac aagaataaag caattaaagc tattaacaac 2100
   tccttgatcg aaagagaggc taagtggaag gaaatctact catggattgt atcaaactgg 2160
   cttactagaa ttaacactca atttaacaag agaaaggagc aaatgtacca ggctctgcaa 2220
   aaccaagtcg atgctatcaa gactgcaatt gaatacaagt acaacaacta tacttccgat 2280
   gagaagaaca gacttgaatc tgaatacaat atcaacaaca ttgaagaaga gttgaacaag 2340
   aaagtttctt tggctatgaa gaatatcgaa agatttatga ccgaatcctc tatctcttac 2400
   ttgatgaagt tgatcaatga ggccaaggtt ggtaagttga agaagtacga taaccacgtt 2460
   aagagcgatc tgctgaacta cattctcgac cacagatcaa tcctgggaga gcagacaaac 2520
   gagctgagtg atttggttac ttccactttg aactcctcca ttccatttga gctttct    2577
<210> 41
   <211> 858
   <212> PRT
   <213> Artificial Sequence
<220>
   <223> Recombinant protein encoded by SEQ ID NO:40
<400> 41
   Met Pro Val Val Ile Asn Ser Phe Asn Tyr Asn Asp Pro Val Asn Asp
    1               5                  10                  15
   Asp Thr Ile Leu Tyr Met Gln Ile Pro Tyr Glu Glu Lys Ser Lys Lys
               20                  25                  30
   Tyr Tyr Lys Ala Phe Glu Ile Met Arg Asn Val Trp Ile Ile Pro Glu
           35                  40                  45
   Arg Asn Thr Ile Gly Thr Asp Pro Ser Asp Phe Asp Pro Pro Ala Ser
       50                  55                  60
   Leu Glu Asn Gly Ser Ser Ala Tyr Tyr Asp Pro Asn Tyr Leu Thr Thr
   65                  70                  75                  80
   Asp Ala Glu Lys Asp Arg Tyr Leu Lys Thr Thr Ile Lys Leu Phe Lys
                   85                  90                  95
   Arg Ile Asn Ser Asn Pro Ala Gly Glu Val Leu Leu Gln Glu Ile Ser
               100                 105                 110
   Tyr Ala Lys Pro Tyr Leu Gly Asn Glu His Thr Pro Ile Asn Glu Phe
           115                 120                 125
   His Pro Val Thr Arg Thr Thr Ser Val Asn Ile Lys Ser Ser Thr Asn
       130                 135                 140
   Val Lys Ser Ser Ile Ile Leu Asn Leu Leu Val Leu Gly Ala Gly Pro
   145                 150                 155                 160
   Asp Ile Phe Glu Asn Ser Ser Tyr Pro Val Arg Lys Leu Met Asp Ser
                   165                 170                 175
   Gly Gly Val Tyr Asp Pro Ser Asn Asp Gly Phe Gly Ser Ile Asn Ile
               180                 185                 190
   Val Thr Phe Ser Pro Glu Tyr Glu Tyr Thr Phe Asn Asp Ile Ser Gly
           195                 200                 205
   Gly Tyr Asn Ser Ser Thr Glu Ser Phe Ile Ala Asp Pro Ala Ile Ser
       210                 215                 220
   Leu Ala His Glu Leu Ile His Ala Leu His Gly Leu Tyr Gly Ala Arg
   225                 230                 235                 240
   Gly Val Thr Tyr Lys Glu Thr Ile Lys Val Lys Gln Ala Pro Leu Met
                   245                 250                 255
   Ile Ala Glu Lys Pro Ile Arg Leu Glu Glu Phe Leu Thr Phe Gly Gly
               260                 265                 270
   Gln Asp Leu Asn Ile Ile Thr Ser Ala Met Lys Glu Lys Ile Tyr Asn
           275                 280                 285
   Asn Leu Leu Ala Asn Tyr Glu Lys Ile Ala Thr Arg Leu Ser Arg Val
       290                 295                 300
   Asn Ser Ala Pro Pro Glu Tyr Asp Ile Asn Glu Tyr Lys Asp Tyr Phe
   305                 310                 315                 320
   Gln Trp Lys Tyr Gly Leu Asp Lys Asn Ala Asp Gly Ser Tyr Thr Val
                   325                 330                 335
   Asn Glu Asn Lys Phe Asn Glu Ile Tyr Lys Lys Leu Tyr Ser Phe Thr
               340                 345                 350
   Glu Ile Asp Leu Ala Asn Lys Phe Lys Val Lys Cys Arg Asn Thr Tyr
           355                 360                 365
   Phe Ile Lys Tyr Gly Phe Leu Lys Val Pro Asn Leu Leu Asp Asp Asp
       370                 375                 380
   Ile Tyr Thr Val Ser Glu Gly Phe Asn Ile Gly Asn Leu Ala Val Asn
   385                 390                 395                 400
   Asn Arg Gly Gln Asn Ile Lys Leu Asn Pro Lys Ile Ile Asp Ser Ile
                   405                 410                 415
   Pro Asp Lys Gly Leu Val Glu Lys Ile Val Lys Phe Cys Lys Ser Val
               420                 425                 430
   Ile Pro Arg Lys Gly Thr Lys Asn Cys Lys Ser Val Ile Pro Arg Lys
           435                 440                 445
   Gly Thr Lys Ala Pro Pro Arg Leu Cys Ile Arg Val Asn Asn Ser Glu
       450                 455                 460
   Leu Phe Phe Val Ala Ser Glu Ser Ser Tyr Asn Glu Asn Asp Ile Asn
   465                 470                 475                 480
   Thr Pro Lys Glu Ile Asp Asp Thr Thr Asn Leu Asn Asn Asn Tyr Arg
                   485                 490                 495
   Asn Asn Leu Asp Glu Val Ile Leu Asp Tyr Asn Ser Gln Thr Ile Pro
               500                 505                 510
   Gln Ile Ser Asn Arg Thr Leu Asn Thr Leu Val Gln Asp Asn Ser Tyr
           515                 520                 525
   Val Pro Arg Tyr Asp Ser Asn Gly Thr Ser Glu Ile Glu Glu Tyr Asp
       530                 535                 540
   Val Val Asp Phe Asn Val Phe Phe Tyr Leu His Ala Gln Lys Val Pro
   545                 550                 555                 560
   Glu Gly Glu Thr Asn Ile Ser Leu Thr Ser Ser Ile Asp Thr Ala Leu
                   565                 570                 575
   Leu Glu Glu Ser Lys Asp Ile Phe Phe Ser Ser Glu Phe Ile Asp Thr
               580                 585                 590
   Ile Asn Lys Pro Val Asn Ala Ala Leu Phe Ile Asp Trp Ile Ser Lys
           595                 600                 605
   Val Ile Arg Asp Phe Thr Thr Glu Ala Thr Gln Lys Ser Thr Val Asp
       610                 615                 620
   Lys Ile Ala Asp Ile Ser Leu Ile Val Pro Tyr Val Gly Leu Ala Leu
   625                 630                 635                 640
   Asn Ile Ile Ile Glu Ala Glu Lys Gly Asn Phe Glu Glu Ala Phe Glu
                   645                 650                 655
   Leu Leu Gly Val Gly Ile Leu Leu Glu Phe Val Pro Glu Leu Thr Ile
               660                 665                 670
   Pro Val Ile Leu Val Phe Thr Ile Lys Ser Tyr Ile Asp Ser Tyr Glu
           675                 680                 685
   Asn Lys Asn Lys Ala Ile Lys Ala Ile Asn Asn Ser Leu Ile Glu Arg
       690                 695                 700
   Glu Ala Lys Trp Lys Glu Ile Tyr Ser Trp Ile Val Ser Asn Trp Leu
   705                 710                 715                 720
   Thr Arg Ile Asn Thr Gln Phe Asn Lys Arg Lys Glu Gln Met Tyr Gln
                   725                 730                 735
   Ala Leu Gln Asn Gln Val Asp Ala Ile Lys Thr Ala Ile Glu Tyr Lys
               740                 745                 750
   Tyr Asn Asn Tyr Thr Ser Asp Glu Lys Asn Arg Leu Glu Ser Glu Tyr
           755                 760                 765
   Asn Ile Asn Asn Ile Glu Glu Glu Leu Asn Lys Lys Val Ser Leu Ala
       770                 775                 780
   Met Lys Asn Ile Glu Arg Phe Met Thr Glu Ser Ser Ile Ser Tyr Leu
   785                 790                 795                 800
   Met Lys Leu Ile Asn Glu Ala Lys Val Gly Lys Leu Lys Lys Tyr Asp
                   805                 810                 815
   Asn His Val Lys Ser Asp Leu Leu Asn Tyr Ile Leu Asp His Arg Ser
               820                 825                 830
   Ile Leu Gly Glu Gln Thr Asn Glu Leu Ser Asp Leu Val Thr Ser Thr
           835                 840                 845
   Leu Asn Ser Ser Ile Pro Phe Glu Leu Ser
       850                 855
<210> 42
   <211> 1337
   <212> DNA
   <213> Artificial Sequence
<220>
   <223> Synthetic polynucleotide sequence for the light chain of of C. botulinum Type G, optimized for expression in *E*. *coli.*
<400> 42
   catatgccgg tcaatattaa gaacttcaat tacaacgacc cgatcaataa tgacgatatc 60
   attatgatgg agcctttcaa cgacccaggt ccaggcacgt attacaaggc ttttcggatc 120
   atcgaccgca tttggatcgt cccggagcgc ttcacgtacg gcttccaacc tgaccagttc 180
   aatgcaagca caggggtttt cagcaaggac gtctacgagt actatgaccc aacttacctg 240
   aagactgacg cggagaagga caaattcctg aagacgatga tcaagttgtt caaccgcatt 300
   aactccaagc cgtccggcca gcgactgctt gatatgattg tggacgccat cccttacctc 360
   ggaaacgcct ctacgccacc ggacaagttc gcggcaaacg ttgcaaacgt gtccatcaac 420
   aagaaaatta ttcagccggg ggccgaggac cagattaagg gacttatgac taatctgatc 480
   atcttcgggc cggggcctgt actctcggac aacttcacgg acagcatgat tatgaacggc 540
   cattcaccga tctcagaagg attcggggca cgtatgatga tccggttctg cccgagttgc 600
   ctcaacgtct tcaacaacgt ccaggaaaat aaggatacat cgatcttctc ccgccgtgcc 660
   tacttcgcgg acccagcgtt aacccttatg cacgagttaa tccacgtatt gcacggcctc 720
   tacggcatta agatctcgaa cttacctatt accccaaaca cgaaagagtt cttcatgcaa 780
   cacagcgacc cggttcaggc cgaggaatta tacaccttcg gcgggcacga cccaagtgtt 840
   atctcaccgt ctaccgatat gaatatctac aacaaggccc tgcaaaactt ccaggacatc 900
   gcaaaccggc ttaacattgt ctcatcggca caggggtctg gtatcgacat ctccctgtat 960
   aagcagatct acaagaataa gtacgacttc gtagaagacc cgaacggcaa gtactcggtg 1020
   gacaaggaca agtttgacaa actctacaaa gctctcatgt tcggtttcac agagacaaat 1080
   cttgccggag agtacgggat caagacgcgg tactcgtatt tttccgagta cctgccgcct 1140
   attaagacgg agaagttgct cgataacacc atttacactc agaatgaggg gttcaacatc 1200
   gcctctaaga atctcaagac cgagttcaat ggtcagaaca aggcggtgaa caaagaggcg 1260
   tatgaggaga ttagtctgga acacttggtg atctaccgaa ttgcgatgtg taagcctgtg 1320
   atgtactaat aggatcc                                                1337
<210> 43
   <211> 444
   <212> PRT
   <213> Artificial Sequence
<220>
   <223> Recombinant protein encoded by SEQ ID NO:42
<221> UNSURE
   <222> (442)...(443)
   <223> Any amino acid at each position
<400> 43
   Met Pro Val Asn Ile Lys Asn Phe Asn Tyr Asn Asp Pro Ile Asn Asn
    1              5                   10                  15
   Asp Asp Ile Ile Met Met Glu Pro Phe Asn Asp Pro Gly Pro Gly Thr
               20                  25                  30
   Tyr Tyr Lys Ala Phe Arg Ile Ile Asp Arg Ile Trp Ile Val Pro Glu
           35                  40                  45
   Arg Phe Thr Tyr Gly Phe Gln Pro Asp Gln Phe Asn Ala Ser Thr Gly
       50                  55                  60
   Val Phe Ser Lys Asp Val Tyr Glu Tyr Tyr Asp Pro Thr Tyr Leu Lys
   65                  70                  75                  80
   Thr Asp Ala Glu Lys Asp Lys Phe Leu Lys Thr Met Ile Lys Leu Phe
                   85                  90                  95
   Asn Arg Ile Asn Ser Lys Pro Ser Gly Gln Arg Leu Leu Asp Met Ile
               100                 105                 110
   Val Asp Ala Ile Pro Tyr Leu Gly Asn Ala Ser Thr Pro Pro Asp Lys
           115                 120                 125
   Phe Ala Ala Asn Val Ala Asn Val Ser Ile Asn Lys Lys Ile Ile Gln
       130                 135                 140
   Pro Gly Ala Glu Asp Gln Ile Lys Gly Leu Met Thr Asn Leu Ile Ile
   145                 150                 155                 160
   Phe Gly Pro Gly Pro Val Leu Ser Asp Asn Phe Thr Asp Ser Met Ile
                   165                 170                 175
   Met Asn Gly His Ser Pro Ile Ser Glu Gly Phe Gly Ala Arg Met Met
               180                 185                 190
   Ile Arg Phe Cys Pro Ser Cys Leu Asn Val Phe Asn Asn Val Gln Glu
           195                 200                 205
   Asn Lys Asp Thr Ser Ile Phe Ser Arg Arg Ala Tyr Phe Ala Asp Pro
       210                 215                 220
   Ala Leu Thr Leu Met His Glu Leu Ile His Val Leu His Gly Leu Tyr
   225                 230                 235                 240
   Gly Ile Lys Ile Ser Asn Leu Pro Ile Thr Pro Asn Thr Lys Glu Phe
                   245                 250                 255
   Phe Met Gln His Ser Asp Pro Val Gln Ala Glu Glu Leu Tyr Thr Phe
                   260                 265                 270
   Gly Gly His Asp Pro Ser Val Ile Ser Pro Ser Thr Asp Met Asn Ile
               275                 280                 285
   Tyr Asn Lys Ala Leu Gln Asn Phe Gln Asp Ile Ala Asn Arg Leu Asn
       290                 295                 300
   Ile Val Ser Ser Ala Gln Gly Ser Gly Ile Asp Ile Ser Leu Tyr Lys
   305                 310                 315                 320
   Gln Ile Tyr Lys Asn Lys Tyr Asp Phe Val Glu Asp Pro Asn Gly Lys
                   325                 330                 335
   Tyr Ser Val Asp Lys Asp Lys Phe Asp Lys Leu Tyr Lys Ala Leu Met
                   340                 345                 350
   Phe Gly Phe Thr Glu Thr Asn Leu Ala Gly Glu Tyr Gly Ile Lys Thr
               355                 360                 365
   Arg Tyr Ser Tyr Phe Ser Glu Tyr Leu Pro Pro Ile Lys Thr Glu Lys
       370                 375                 380
   Leu Leu Asp Asn Thr Ile Tyr Thr Gln Asn Glu Gly Phe Asn Ile Ala
   385                 390                 395                 400
   Ser Lys Asn Leu Lys Thr Glu Phe Asn Gly Gln Asn Lys Ala Val Asn
                   405                 410                 415
   Lys Glu Ala Tyr Glu Glu Ile Ser Leu Glu His Leu Val Ile Tyr Arg
                   420                 425                 430
   Ile Ala Met Cys Lys Pro Val Met Tyr Xaa Xaa Asp
               435                 440
<210> 44
   <211> 2547
   <212> DNA
   <213> Artificial Sequence
<220>
   <223> Synthetic polynucleotide sequence for the light chain with Hn segment of of C. botulinum Type G, optimized for expression in *E. coli.*
<400> 44
   catatgccgg tcaatattaa gaacttcaat tacaacgacc cgatcaataa tgacgatatc 60
   attatgatgg agcctttcaa cgacccaggt ccaggcacgt attacaaggc ttttcggatc 120
   atcgaccgca tttggatcgt cccggagcgc ttcacgtacg gcttccaacc tgaccagttc 180
   aatgcaagca caggggtttt cagcaaggac gtctacgagt actatgaccc aacttacctg 240
   aagactgacg cggagaagga caaattcctg aagacgatga tcaagttgtt caaccgcatt 300
   aactccaagc cgtccggcca gcgactgctt gatatgattg tggacgccat cccttacctc 360
   ggaaacgcct ctacgccacc ggacaagttc gcggcaaacg ttgcaaacgt gtccatcaac 420
   aagaaaatta ttcagccggg ggccgaggac cagattaagg gacttatgac taatctgatc 480
   atcttcgggc cggggcctgt actctcggac aacttcacgg acagcatgat tatgaacggc 540
   cattcaccga tctcagaagg attcggggca cgtatgatga tccggttctg cccgagttgc 600
   ctcaacgtct tcaacaacgt ccaggaaaat aaggatacat cgatcttctc ccgccgtgcc 660
   tacttcgcgg acccagcgtt aacccttatg cacgagttaa tccacgtatt gcacggcctc 720
   tacggcatta agatctcgaa cttacctatt accccaaaca cgaaagagtt cttcatgcaa 780
   cacagcgacc cggttcaggc cgaggaatta tacaccttcg gcgggcacga cccaagtgtt 840
   atctcaccgt ctaccgatat gaatatctac aacaaggccc tgcaaaactt ccaggacatc 900
   gcaaaccggc ttaacattgt ctcatcggca caggggtctg gtatcgacat ctccctgtat 960
   aagcagatct acaagaataa gtacgacttc gtagaagacc cgaacggcaa gtactcggtg 1020
   gacaaggaca agtttgacaa actctacaaa gctctcatgt tcggtttcac agagacaaat 1080
   cttgccggag agtacgggat caagacgcgg tactcgtatt tttccgagta cctgccgcct 1140
   attaagacgg agaagttgct cgataacacc atttacactc agaatgaggg gttcaacatc 1200
   gcctctaaga atctcaagac cgagttcaat ggtcagaaca aggcggtgaa caaagaggcg 1260
   tatgaggaga ttagtctgga acacttggtg atctaccgaa ttgcgatgtg taagcctgtg 1320
   atgtacaaga acaccggtaa gtccgagcag tgtatcatcg tcaacaacga ggacttgttc 1380
   ttcatcgcca acaaggactc cttctccaag gacttggcca aggctgagac catcgcctac 1440
   aacacccaga acaacaccat cgagaacaac ttctccatcg accagctgat cttggacaac 1500
   gacctgtcct ccggtatcga cctgccaaac gagaacaccg agccattcac caacttcgac 1560
   gacatcgaca tcccagtcta catcaagcag tccgccctga agaagatctt cgtcgacggt 1620
   gactccttgt tcgagtacct gcacgcccag accttcccat ccaacatcga gaaccagttg 1680
   accaactccc tgaacgacgc tttgagaaac aacaacaagg tctacacctt cttctccact 1740
   aacttggtcg agaaggccaa cactgtcgtc ggtgcctcct tgttcgtcaa ctgggtcaag 1800
   ggtgtcatcg acgacttcac ctccgagtcc acccaaaagt ccaccatcga caaggtctcc 1860
   gacgtctcca tcatcatccc atacatcggt ccagccctga acgtcggtaa cgagaccgct 1920
   aaggagaact tcaagaacgc cttcgagatc ggtggtgccg ccatcctgat ggagttcatc 1980
   ccagagttga tcgtcccaat cgtcggtttc ttcaccttgg agtcctacgt cggtaacaag 2040
   ggtcacatca tcatgaccat ctccaacgcc ctgaagaaga gagaccagaa gtggaccgac 2100
   atgtacggtt tgatcgtctc ccagtggttg tccaccgtca acacccagtt ctacaccatc 2160
   aaggagagaa tgtacaacgc cttgaacaac cagtcccagg ccatcgagaa gatcatcgag 2220
   gaccagtaca accgttactc cgaggaggac aagatgaaca tcaacatcga cttcaacgac 2280
   atcgacttca agctgaacca gtccatcaac ctggccatca acaacatcga cgacttcatc 2340
   aaccagtgtt ccatctccta cctgatgaac cgtatgatcc cactggccgt caagaagttg 2400
   aaggacttcg acgacaacct gaagcgtgac ctgctggagt acatcgacac caacgagttg 2460
   tacctgctgg acgaggtcaa catcttgaag tccaaggtca acagacactt gaaggactcc 2520
   atcccattcg acttgtcctt gtacacc                                     2547
<210> 45
   <211> 848
   <212> PRT
   <213> Artificial Sequence
<220>
   <223> Recombinant protein encoded by SEQ ID NO:44
<400> 45
   Met Pro Val Asn Ile Lys Asn Phe Asn Tyr Asn Asp Pro Ile Asn Asn
    1               5                  10                  15
   Asp Asp Ile Ile Met Met Glu Pro Phe Asn Asp Pro Gly Pro Gly Thr
               20                  25                  30
   Tyr Tyr Lys Ala Phe Arg Ile Ile Asp Arg Ile Trp Ile Val Pro Glu
           35                  40                  45
   Arg Phe Thr Tyr Gly Phe Gln Pro Asp Gln Phe Asn Ala Ser Thr Gly
       50                  55                  60
   Val Phe Ser Lys Asp Val Tyr Glu Tyr Tyr Asp Pro Thr Tyr Leu Lys
   65                  70                  75                  80
   Thr Asp Ala Glu Lys Asp Lys Phe Leu Lys Thr Met Ile Lys Leu Phe
                   85                  90                  95
   Asn Arg Ile Asn Ser Lys Pro Ser Gly Gln Arg Leu Leu Asp Met Ile
               100                 105                 110
   Val Asp Ala Ile Pro Tyr Leu Gly Asn Ala Ser Thr Pro Pro Asp Lys
           115                 120                 125
   Phe Ala Ala Asn Val Ala Asn Val Ser Ile Asn Lys Lys Ile Ile Gln
       130                 135                 140
   Pro Gly Ala Glu Asp Gln Ile Lys Gly Leu Met Thr Asn Leu Ile Ile
   145                 150                 155                 160
   Phe Gly Pro Gly Pro Val Leu Ser Asp Asn Phe Thr Asp Ser Met Ile
                   165                 170                 175
   Met Asn Gly His Ser Pro Ile Ser Glu Gly Phe Gly Ala Arg Met Met
               180                 185                 190
   Ile Arg Phe Cys Pro Ser Cys Leu Asn Val Phe Asn Asn Val Gln Glu
           195                 200                 205
   Asn Lys Asp Thr Ser Ile Phe Ser Arg Arg Ala Tyr Phe Ala Asp Pro
       210                 215                 220
   Ala Leu Thr Leu Met His Glu Leu Ile His Val Leu His Gly Leu Tyr
   225                 230                 235                 240
   Gly Ile Lys Ile Ser Asn Leu Pro Ile Thr Pro Asn Thr Lys Glu Phe
                   245                 250                 255
   Phe Met Gln His Ser Asp Pro Val Gln Ala Glu Glu Leu Tyr Thr Phe
               260                 265                 270
   Gly Gly His Asp Pro Ser Val Ile Ser Pro Ser Thr Asp Met Asn Ile
           275                 280                 285
   Tyr Asn Lys Ala Leu Gln Asn Phe Gln Asp Ile Ala Asn Arg Leu Asn
       290                 295                 300
   Ile Val Ser Ser Ala Gln Gly Ser Gly Ile Asp Ile Ser Leu Tyr Lys
   305                 310                 315                 320
   Gln Ile Tyr Lys Asn Lys Tyr Asp Phe Val Glu Asp Pro Asn Gly Lys
                   325                 330                 335
   Tyr Ser Val Asp Lys Asp Lys Phe Asp Lys Leu Tyr Lys Ala Leu Met
               340                 345                 350
   Phe Gly Phe Thr Glu Thr Asn Leu Ala Gly Glu Tyr Gly Ile Lys Thr
           355                 360                 365
   Arg Tyr Ser Tyr Phe Ser Glu Tyr Leu Pro Pro Ile Lys Thr Glu Lys
       370                 375                 380
   Leu Leu Asp Asn Thr Ile Tyr Thr Gln Asn Glu Gly Phe Asn Ile Ala
   385                 390                 395                 400
   Ser Lys Asn Leu Lys Thr Glu Phe Asn Gly Gln Asn Lys Ala Val Asn
                   405                 410                 415
   Lys Glu Ala Tyr Glu Glu Ile Ser Leu Glu His Leu Val Ile Tyr Arg
               420                 425                 430
   Ile Ala Met Cys Lys Pro Val Met Tyr Lys Asn Thr Gly Lys Ser Glu
           435                 440                 445
   Gln Cys Ile Ile Val Asn Asn Glu Asp Leu Phe Phe Ile Ala Asn Lys
       450                 455                 460
   Asp Ser Phe Ser Lys Asp Leu Ala Lys Ala Glu Thr Ile Ala Tyr Asn
   465                 470                 475                 480
   Thr Gln Asn Asn Thr Ile Glu Asn Asn Phe Ser Ile Asp Gln Leu Ile
                   485                 490                 495
   Leu Asp Asn Asp Leu Ser Ser Gly Ile Asp Leu Pro Asn Glu Asn Thr
               500                 505                 510
   Glu Pro Phe Thr Asn Phe Asp Asp Ile Asp Ile Pro Val Tyr Ile Lys
           515                 520                 525
   Gln Ser Ala Leu Lys Lys Ile Phe Val Asp Gly Asp Ser Leu Phe Glu
       530                 535                 540
   Tyr Leu His Ala Gln Thr Phe Pro Ser Asn Ile Glu Asn Gln Leu Thr
   545                 550                 555                 560
   Asn Ser Leu Asn Asp Ala Leu Arg Asn Asn Asn Lys Val Tyr Thr Phe
                   565                 570                 575
   Phe Ser Thr Asn Leu Val Glu Lys Ala Asn Thr Val Val Gly Ala Ser
               580                 585                 590
   Leu Phe Val Asn Trp Val Lys Gly Val Ile Asp Asp Phe Thr Ser Glu
           595                 600                 605
   Ser Thr Gln Lys Ser Thr Ile Asp Lys Val Ser Asp Val Ser Ile Ile
       610                 615                 620
   Ile Pro Tyr Ile Gly Pro Ala Leu Asn Val Gly Asn Glu Thr Ala Lys
   625 630 635 640
   Glu Asn Phe Lys Asn Ala Phe Glu Ile Gly Gly Ala Ala Ile Leu Met
   645 650 655
   Glu Phe Ile Pro Glu Leu Ile Val Pro Ile Val Gly Phe Phe Thr Leu
   660 665 670
   Glu Ser Tyr Val Gly Asn Lys Gly His Ile Ile Met Thr Ile Ser Asn
   675 680 685
   Ala Leu Lys Lys Arg Asp Gln Lys Trp Thr Asp Met Tyr Gly Leu Ile
   690 695 700
   Val Ser Gln Trp Leu Ser Thr Val Asn Thr Gln Phe Tyr Thr Ile Lys
   705 710 715 720
   Glu Arg Met Tyr Asn Ala Leu Asn Asn Gln Ser Gln Ala Ile Glu Lys
   725 730 735
   Ile Ile Glu Asp Gln Tyr Asn Arg Tyr Ser Glu Glu Asp Lys Met Asn
   740 745 750
   Ile Asn Ile Asp Phe Asn Asp Ile Asp Phe Lys Leu Asn Gln Ser Ile
   755 760 765
   Asn Leu Ala Ile Asn Asn Ile Asp Asp Phe Ile Asn Gln Cys Ser Ile
   770 775 780
   Ser Tyr Leu Met Asn Arg Met Ile Pro Leu Ala Val Lys Lys Leu Lys
   785 790 795 800
   Asp Phe Asp Asp Asn Leu Lys Arg Asp Leu Leu Glu Tyr Ile Asp Thr
   805 810 815
   Asn Glu Leu Tyr Leu Leu Asp Glu Val Asn Ile Leu Lys Ser Lys Val
   820 825 830
   Asn Arg His Leu Lys Asp Ser Ile Pro Phe Asp Leu Ser Leu Tyr Thr
   835 840 845
<210> 46
   <211> 7
   <212> PRT
   <213> Artificial Sequence
<220>
   <223> Synthetic peptide; competative inhibitor of Zn protease
<400> 46
   Cys Arg Ala Thr Lys Met Leu
   1 5
<210> 47
   <211> 449
   <212> PRT
   <213> Artificial Sequence
<220>
   <223> Synthetic botulinum neurotoxin light chain of serotype A based on wild-type Clostridium botulinum sequence
<400> 47
   Met Val Gln Phe Val Asn Lys Gln Phe Asn Tyr Lys Asp Pro Val Asn
    1               5                  10                  15
   Gly Val Asp Ile Ala Tyr Ile Lys Ile Pro Asn Val Gly Gln Met Gln
               20                  25                  30
   Pro Val Lys Ala Phe Lys Ile His Asn Lys Ile Trp Val Ile Pro Glu
           35                  40                  45
   Arg Asp Thr Phe Thr Asn Pro Glu Glu Gly Asp Leu Asn Pro Pro Pro
       50                  55                  60
   Glu Ala Lys Gln Val Pro Val Ser Tyr Tyr Asp Ser Thr Tyr Leu Ser
   65                  70                  75                  80
   Thr Asp Asn Glu Lys Asp Asn Tyr Leu Lys Gly Val Thr Lys Leu Phe
                   85                  90                  95
   Glu Arg Ile Tyr Ser Thr Asp Leu Gly Arg Met Leu Leu Thr Ser Ile
               100                 105                 110
   Val Arg Gly Ile Pro Phe Trp Gly Gly Ser Thr Ile Asp Thr Glu Leu
           115                 120                 125
   Lys Val Ile Asp Thr Asn Cys Ile Asn Val Ile Gln Pro Asp Gly Ser
       130                 135                 140
   Tyr Arg Ser Glu Glu Leu Asn Leu Val Ile Ile Gly Pro Ser Ala Asp
   145                 150                 155                 160
   Ile Ile Gln Phe Glu Cys Lys Ser Phe Gly His Glu Val Leu Asn Leu
                   165                 170                 175
   Thr Arg Asn Gly Tyr Gly Ser Thr Gln Tyr Ile Arg Phe Ser Pro Asp
               180                 185                 190
   Phe Thr Phe Gly Phe Glu Glu Ser Leu Glu Val Asp Thr Asn Pro Leu
           195                 200                 205
   Leu Gly Ala Gly Lys Phe Ala Thr Asp Pro Ala Val Thr Leu Ala His
       210                 215                 220
   Glu Leu Ile His Ala Gly His Arg Leu Tyr Gly Ile Ala Ile Asn Pro
   225                 230                 235                 240
   Asn Arg Val Phe Lys Val Asn Thr Asn Ala Tyr Tyr Glu Met Ser Gly
                   245                 250                 255
   Leu Glu Val Ser Phe Glu Glu Leu Arg Thr Phe Gly Gly His Asp Ala
               260                 265                 270
   Lys Phe Ile Asp Ser Leu Gln Glu Asn Glu Phe Arg Leu Tyr Tyr Tyr
           275                 280                 285
   Asn Lys Phe Lys Asp Ile Ala Ser Thr Leu Asn Lys Ala Lys Ser Ile
       290                 295                 300
   Val Gly Thr Thr Ala Ser Leu Gln Tyr Met Lys Asn Val Phe Lys Glu
   305                 310                 315                 320
   Lys Tyr Leu Leu Ser Glu Asp Thr Ser Gly Lys Phe Ser Val Asp Lys
                   325                 330                 335
   Leu Lys Phe Asp Lys Leu Tyr Lys Met Leu Thr Glu Ile Tyr Thr Glu
               340                 345                 350
   Asp Asn Phe Val Lys Phe Phe Lys Val Leu Asn Arg Lys Thr Tyr Leu
           355                 360                 365
   Asn Phe Asp Lys Ala Val Phe Lys Ile Asn Ile Val Pro Lys Val Asn
       370                 375                 380
   Tyr Thr Ile Tyr Asp Gly Phe Asn Leu Arg Asn Thr Asn Leu Ala Ala
   385                 390                 395                 400
   Asn Phe Asn Gly Gln Asn Thr Glu Ile Asn Asn Met Asn Phe Thr Lys
                   405                 410                 415
   Leu Lys Asn Phe Thr Gly Leu Phe Glu Phe Tyr Lys Leu Leu Cys Val
               420                 425                 430
   Arg Gly Ile Ile Thr Ser Lys Thr Lys Ser Leu Asp Lys Gly Tyr Asn
           435                 440                 445
   Lys

## Description

### FIELD OF THE INVENTION

This invention is directed to construction, expression, and purification of synthetic DNA molecules encoding polypeptides comprising botulinum neurotoxin A light chains (BoNT/A LC), useful in methods of vaccination against botulism using the expressed peptides.

### BACKGROUND OF THE INVENTION

The sporulating, obligate anaerobic, gram-positive bacillus *Clostridium* produces eight forms of antigenically distinct exotoxins.

These neurotoxins are expressed as 150-kDa single polypeptides (termed dichains) containing a disulfide bond between the 50-kDa N-terminal light chain (LC) and the 100-kDa C-terminal heavy chain (HC). A post-translational cryptic cleavage generates the two chains connected by a disulfide bond. The LC contains the toxic, zinc-endopeptidase catalytic domain. The 100-kDa HC may be further proteolyzed into a 50-kDa N-terminal membrane-spanning domain (Hₙ) and a 50-kDa C-terminal receptor-binding domain (H_{c}).

The BoNTs and their LCs are targets of vaccine development, drug design, and mechanism studies because of their potential role in biological warfare, wide therapeutic applications, and potential to facilitate elucidation of the mechanism of membrane exocytosis. In spite of such immense importance, studies of the LC have been limited by its availability. Commercially available LC is prepared by separating it from the dichain toxins under denaturing conditions. These preparations therefore retain some contaminating toxicity of the dichain, have low solubility, and often begin to proteolytically degrade and start losing activity within hours of storage in solution.

The LC of serotype A has been separated and purified from the full-length toxin by QAB-Sephadex chromatography from 2 M urea; however, the preparation suffers from low solubility (Shone and Tranter, 1995). The LC of serotype C was similarly obtained at a level of <5 mg/10 L culture of *C. botulinum* (Syuto and Kubo, 1981). These preparations almost invariably contain contaminating full-length toxins, and the commercially available preparations precipitate from solution or undergo proteolytic degradation upon hours of storage in solution. More recently the LC of tetanus neurotoxin (Li *et al.,* 1994) and of BoNT/A (Zhou *et al.,* 1995) were expressed in *E*. *coli* as maltose-binding proteins and purified in 0.5 mg quantities from 1-L cultures (Zhou *et al.,* 1995). However, the poor expression of the cloned products, probably due to rare codon usage or high A + T composition in clostridial DNA (Makoff *et al.,* 1989, Winkler and Wood, 1988), remained a major hurdle in obtaining adequate amount of the protein for structural and functional studies.

New-generation, recombinant vaccines have been developed by USAMRIID (e.g. Dertzbaugh MT, September 11, 2001, U.S. Pat. No. 6,287,566; U.S. Appln. No. 09/910,186 filed July 20,2001; and U.S. Appln. No. 09/611,419 filed July 6,2000) and commercial sources (e.g. Ophidian Pharmaceuticals, Inc. Williams JA, July 6,1999, U.S. Pat. No. 5,919,665; using clones supplied by USAMRIID).

Recombinant production methods alleviate many of the problems associated with the toxoid, such as the need for a dedicated manufacturing facility. Presently, many cGMP facilities are in existence and available that could manufacture a recombinant product. There would be no need to culture large quantities of a hazardous toxin-producing bacterium. Production yields from a genetically engineered product are expected to be high. Recombinant products would be purer, less reactogenic, and more fully characterized. Thus, the cost of a recombinant product would be expected to be much lower than a toxoid because there would be no expenditures required to support a dedicated facility, and the higher production yields would reduce the cost of therapeutic and research products.

However, recombinant methods as described in the publications above do not yield optimal results because botulinum codons are not translated well in other organisms commonly used for production, such as *E. coli* or yeast. Furthermore, no easily translatable, recombinant form of the non-neurotoxic, mutated light chain presently exists. Recombinant forms of both functional and non-neurotoxic botulinum neurotoxin that may be translated efficiently in either *E. coli* or yeast are needed for research and therapeutic purposes.

Many clinical disorders are presently being treated with a botulinum neurotoxin complex that is isolated from the bacterium, *Clostridium botulinum.* There is no data to demonstrate that the binding proteins play any role in the therapeutic effects of the drug. The binding proteins, however, probably contribute to the immunological response in thos patients that become non-repsonsive to drug treatment. Recombinant products could be manufactured under conditions that are more amenable to product characterization. Chimeras of the drug product could also be produced by domain switching. Chimeras could potentially increase the number of potential useful drug products.

WO 98/07864 relates to a recombinant BoNT/A DNA construct which codes for the entire light-chain plus the first 423 amino acids of the heavy chain, as a single polypeptide. The first 912 nucleotides of the light-chain have a synthetic sequence with an *E*. *coli* codon bias. The remainder (nucleotides 913 to 1138) is constructed from native sequence.

Li et al. (1999), Protein Expression and Purification, 17, 339-344 relates to the production of recombinant BoNT/A Lc in *E*. *coli.* The BoNTA/Lc DNA fragment is described merely as "from *C. botulinum* neurotoxin type A". It is therefore presumed to be the native DNA.

Clayton et al. (1995), Infection and Immunity 63, 2738-2742 prepared a synthetic gene coding for the C-fragment (about half) of the heavy chain of BoNTA, by substituting the most frequently used codon in *E*. *coli* for each amino acid, for the codon of the native sequence, and making conservative codon substitutions to reduce the length of poly(A) sequences. The authors expressed this gene in *E*. *coli* and carried out satisfactory immunogenicity experiments with the protein produced.

WO 98/08540 relates, inter alia, to the construction of a synthetic gene encoding the C-fragment of BONT/A heavy chain. In order to improve the expression of C-fragment protein in *E*. *coli,* a synthetic version of the gene was created, in which non-preferred codons were replaced with preferred codons.

### SUMMARY OF THE INVENTION

The present invention relates to the design and construction of synthetic DNA molecules that encode BONT/A LC and is capable of being expressed in heterologous prokaryotic or eukaryotic hosts. The invention is based, in part, on modifying the wild-type BONT sequence according to the codon usage normally found in genes that are highly expressed in the host organism. By selecting codons rich in G+C content, the sythetic DNA molecules may be designed to lower the high A+T rich base composition found in clostridial genes.

Especially, the present invention describes the construction and over-expression of a synthetic gene for BONT/A LC in *E. coli*. The high level of expression obtained enabled purification of gram quantities of LC from 1 L of culture, as well as extensive characterization. The preparation of the rBONT/A LC was highly soluble, stable at 4°C for at least 6 months, and had the expected enzymatic and functional properties. For the first time, a cysteine residue was tentatively identified in the vicinity of the active site which, when modified by mercuric compounds, led to complete loss of enzymatic activity.

The invention provides a nucleic acid molecule comprising the nucleotide sequence 9-1355 of SEQ ID NO: 4 encoding a botulinum neurotoxin serotype A light chain or comprising a nucleotide sequence encoding a truncation thereof which lacks the sequence coding for the last 21 or 22 amino acids at the carboxy terminus of the botulinum neurotoxin serotype A light chain.

The invention further provides an expression vector carrying the nucleic acid molecule defined above; (a) an *Escherichia coli* host cell and (b) a yeast host cell containing the vector, especially (c) an E. coli or yeast cell which produces a protein comprising a botulinum neurotoxin light chain serotype A or the said truncation thereof; and a method for producing a polypeptide comprising a *C. botulinum* neurotoxin light chain of serotype A or a truncation thereof which lacks the last 21 or 22 amino acids at the carboxy terminus thereof, said method comprising culturing a host cell (c) above under conditions wherein the nucleic acid molecule carried by the expression vector is expressed.

According to the invention, BONT/A LC may be expressed in a heterologous host system by itself or as a fusion to another protein or carrier. For example, the BONT LC may be fused to a synthetic or wild-type BONT heavy chain or fragment thereof. BONT Lc of the invention may or may not have catalytic activity as a zinc protease. In some embodiments of the invention, catalytically inactive BONT LC is fused to a BONT heavy chain, forming a mutant holotoxin. Non-enzymatic, nontoxic mutant holotoxins are capable of being internalized into nerve cells. In addition, mutant holotoxins may be used as transporters to carry other molecules into cholinergic nerve cells.

### BRIEF DESCRIPTION OF THE DRAWINGS

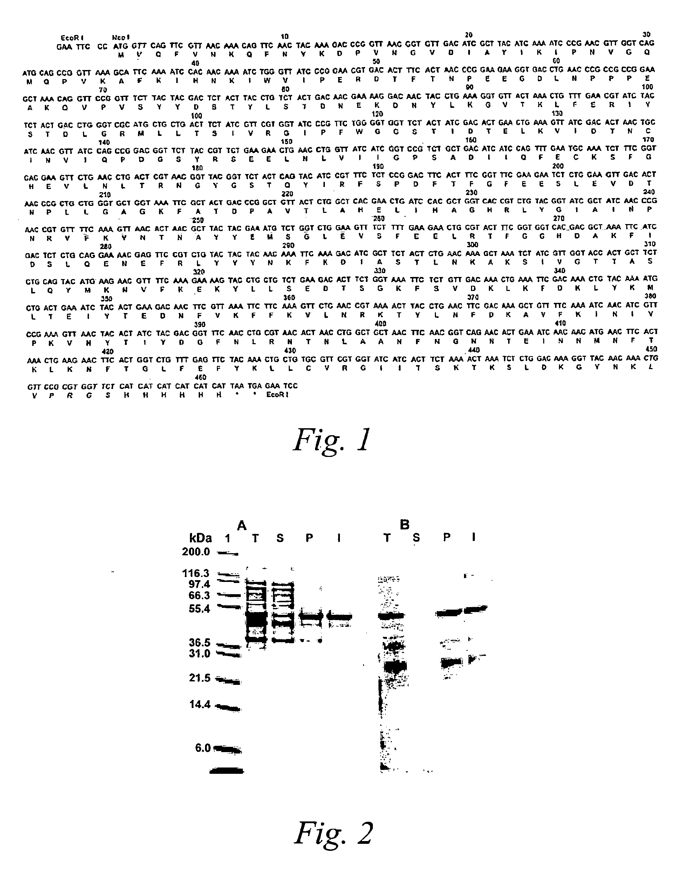

Figure 1. Nucleotide sequence ofrBoNT/A LC and the corresponding amino acid sequence. The codon in *italics* (i.e., encoding the penultimate val residue) and at the 5' end of the gene was introduced to create and maintain the *Nco* I restrictions enzyme site. Codon in *italics* (i.e., encoding LVPRGS) at the 3' end of the gene encode a thrombin protease cleavage site for removing the His tag after purification.
Figure 2. SDS-PAGE followed by Coomassie stain (A) and Western blot (B) of crude and purified BONT/A LC expressed in *E. coli* containing the synthetic gene for BONT/A LC in a multicopy plasmid pET24. Total cellular protein (T), soluble supernatant (S), insoluble pellet (P), and purified inclusion bodies (I) were prepared as described in Section 2. Lane 1 shows Novex wide-range molecular-mass markers (0.8-3.0 µg/band). The sarkosyl solubilized inclusion bodies of the LC had the same electrophoretic behavior as (I). About 20 µg of protein was applied per lane. Western blot used affinity-purified rabbit polyclonal antibodies against a 16-residue N-terminal sequence of the BONT/A LC as the primary antibody and a peroxidase-coupled goat anti-rabbit IgG (H+L) as the secondary antibody. Bands were visualized by a chromogenic substrate.

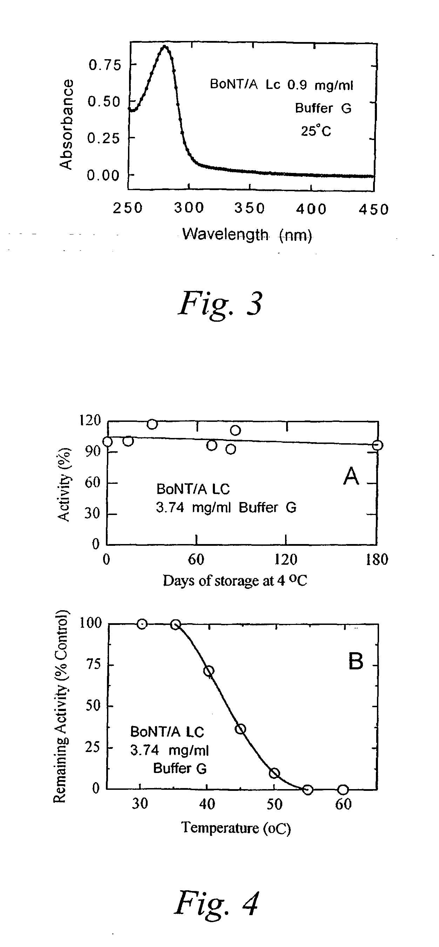

Figure 3. UV-visible absorption spectrum of the rBoNT/A LC.
Figure 4. Long-term stability at 4°C (A) and thermal stability (B) of the rBoNT/A LC. (A) Aliquots of the LC from one single preparation were assayed at the indicated times; (B) 50 µl aliquots of the LC in buffer G containing 1 mM DTT and 501tM ZnCl₂ were taken in Eppendorf tubes and heated for 5 min at the indicated temperatures. After cooling on ice for 60 min, the supernatants were assayed for proteolytic activity.

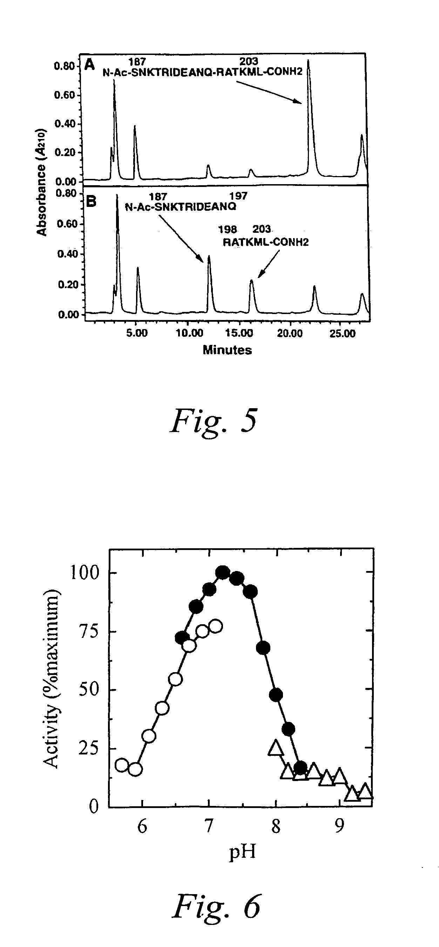

Figure 5. Proteolysis of the synthetic peptide substrate by the rBoNT/A LC. The peptide (1.1 mM) was incubated for 5 min (A) or 200 min (B) with the rBoNT/A LC. The reaction products were analyzed by reverse-phase HPLC. The first three peaks represent the solvent front (<4 min) and reduced DTT (5.2 min) in the reaction mixture. Sequence of the substrate and the sequences of the products are shown in panels A and B, respectively. The numbers above the sequences represent the LC residue numbers corresponding to the sequence of SNAP-25. The product peaks (not labeled in Panel A) were identified by sequence determination by MS-MS.
Figure 6. Effect of pH on the endopeptidase activity of the rBoNT/A LC. Activities were measured at various pH of 0.1 M buffers: MES (-O-), HEPES (-●-), and bis-HCI (-Δ-) containing 0.9 mM substrate peptide Maximum activity (100%) was 334 nmol/min/mg LC.

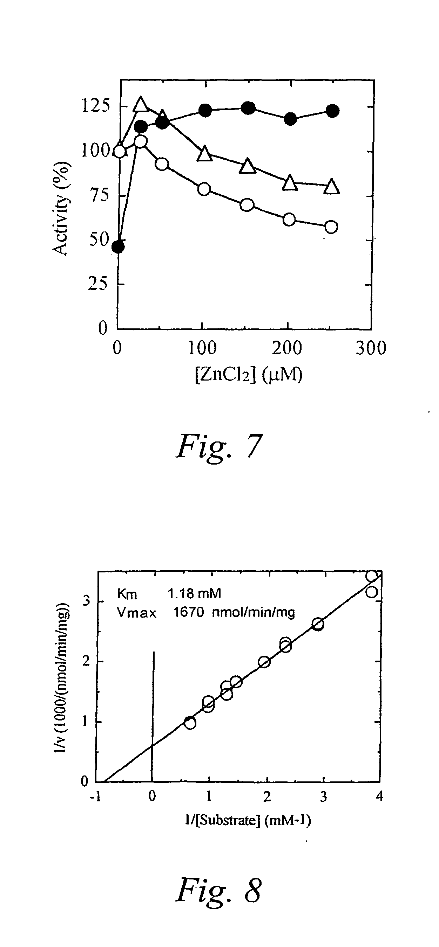

Figure 7. Inhibition of endopeptidase activity of the rBoNT/A LC by excess Zn²⁺ and protection from inhibition by DTT. The LC was assayed in SO mM HEPES, pH 7.4, containing 0.9 mM substrate peptide in the absence (-O-) and presence of 5 mM DTT (-●-) or 5 mM mercaptoethanol (-Δ-) containing the indicated concentrations of ZnCl₂. One hundred percent activity (290 nmol/min/mg LC) represents the activity obtained in the absence of any added thiol or Zn²⁺.
Figure 8. Determination of -*K*ₘ and Vₘₐₓ from the double-reciprocal (Lineweaver-Burke) plot of initial rates of proteolysis versus substrate concentration by the rBoNT/A LC. The reaction mixtures (0.03 ml) contained 0.25 mM ZnCl₂, 0.5 mM DTT, 50 mM HEPES, pH 7.4, and 0.016 mg rBoNT/A LC. The *K*ₘ and *V*ₘₐₓ were calculated as 0.9 mM and 1500 nmol/min/mg, respectively.

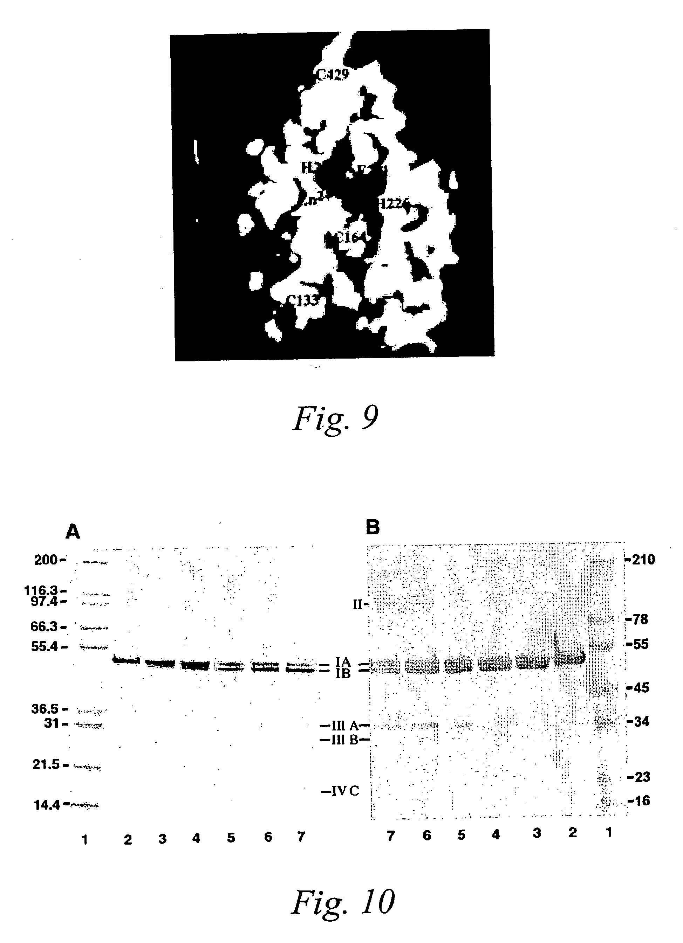

Figure 9. Location of the three Cys residues in the BONT/A LC. Molecular surface of the LC portion of the BONT/A dichain based on its three-dimensional structure (Lacy and Stevens, 1999) is shown. The three Cys residues (yellow), active-site His and asp residues (red), the Zn²⁺ atom (blue) at the active site, and the 'pit' leading to the active site are highlighted. The side chain of Cys- 164 lines the surface and forms part of the wall of the 'pit' leading to the active site. The 'pit' acts as an access route of the substrate.
Figure 10. Time course of proteolysis of BoNT/A LC as followed by SDS-PAGB (A) and Western blot (B). Aliquots of 25ml of the LC (0.2 mg/ml) were incubated at 4°C. At intervals (see below), 25µl of 2 x SDS-load buffer was added to an aliquot and boiled. Two SDS gels were run in parallel. One gel was stained by Coomassie (A) and the proteins from the other were transferred to a nitrocellulose membrane for Western blot (B). Lane 1 in panel A shows Novex Mark-12 molecular weight markers and lane 1 in panel B shows the Novex prestained SeeBlue molecular weight markers. In both panels A and B, lanes 2-7 show 0, 2, 4, 14, 21, and 28 days of incubation, respectively, ofLC. Identity of the protein bands between panels A and B is arbitrary, and the same nomenclature is used throughout the paper.

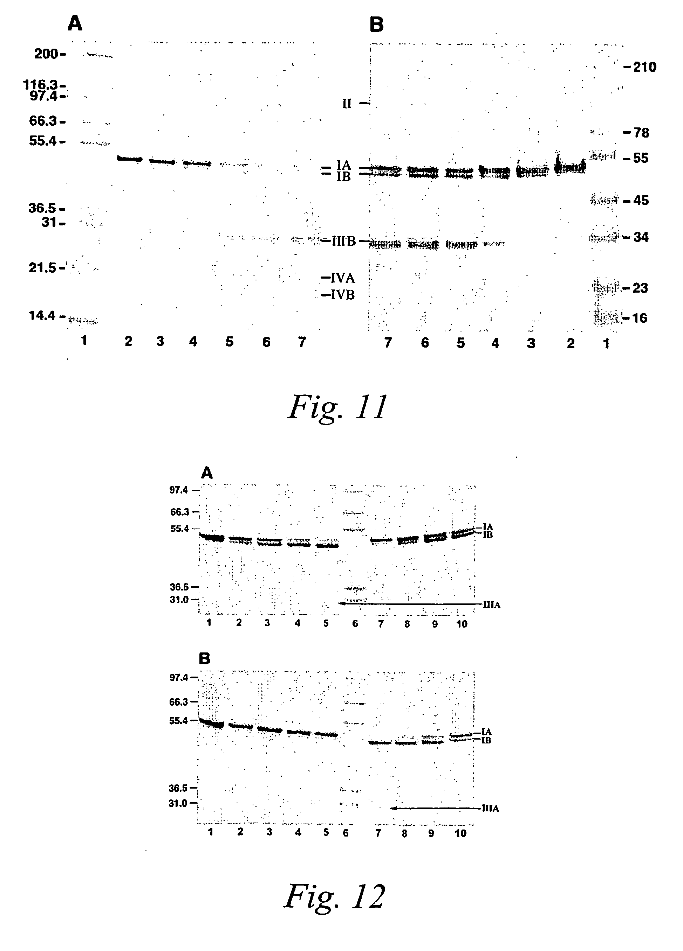

Figure 11. Enhancement of the proteolysis ofBoNT/A LC by ZnCl₂ as followed by SDS-PAGE (A) and Western blot (B). All conditions are same as in Figure 1, except that 0.25 mM ZnCl₂ was added to the incubation mixture of the LC.

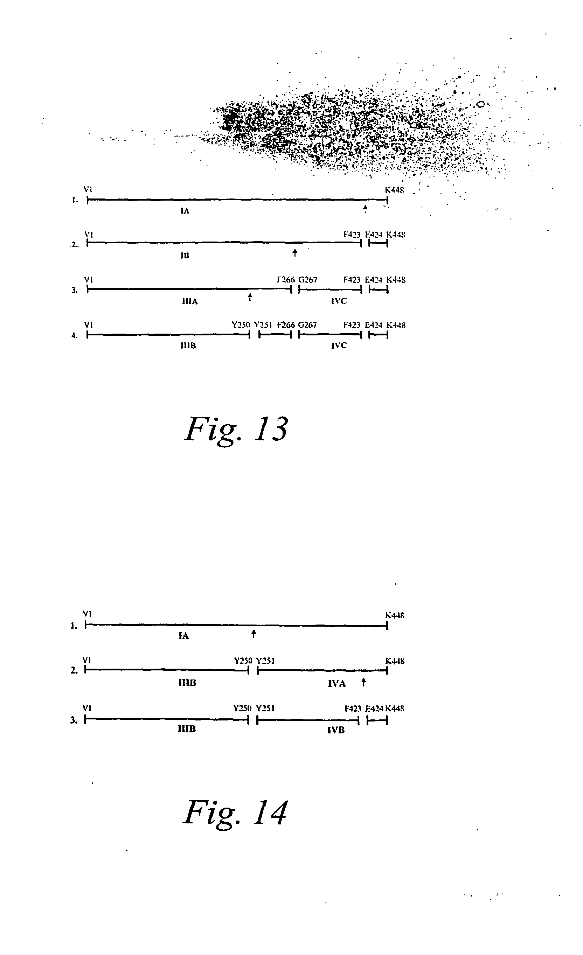

Figure 12. Protection of BoNT/A LC from proteolysis by the metal chelator TPEN (A) and the competitive peptide inhibitor CRATKML (B), followed as a time course by SDS-PAGE. (A) the LC (0.2 mg/ml) was incubated in small aliquots with 10 mM EDTA (lanes 2-5) or with 5 mM TPEN (lanes 7-10). Lanes 2 and 7,3 and 8, 4 and 9 and 5 and 10 show 6,14,21, and 28 days of incubation, respectively, (B) The LC was incubated with 1 mM peptide inhibitor containing 5 mM DTT (lanes 2-5) or without the peptide inhibitor (lanes 10-7) at 4°C. DTT, which does not have an effect on proteolysis, was added to maintain the peptide in monomer form. Lanes 2 and 10, 3 and 9, 4 and 8, and 5 and 7 show 6, 14, 21 and 28 days of incubation, respectively. In both panels A and B, lane 1 represents LC alone at day 0, and lane 6 has molecular weight markers (labels on left). The protein band IIIA (see Figure 1) was faint in this experiment and was not captured in the photographic reproduction; therefore its location in the original gel is shown by arrows in the figure. Note that (a) presence (lanes 2-5, A) and absence (lanes 10-7, B) of EDTA had little effect on proteolysis of IA to IB and finally to IIIA, (b) TPEN (lanes 7-10, A) significantly reduced the rate of conversion ofIA to IB and prevented formation of IIIA during the course of the experiment, and (c) the peptide inhibitor (lanes 2-5, B) drastically reduced the proteolysis of IA to IB and prevented the formation of IIIA.
Figure 13. Scheme L Steps in the self-proteolysis of BoNT/A LC in the absence of added zinc. Arrows show the sites of proteolysis. Full-length LC is denoted by IA. The fragments IB, IIIB, and IVC correspond to the fragment designations in Figure 1. The primary event is the C-terminal truncation to form IB followed by cleavage between Y286 and G287 producing IIIA and IVC. The fragment IIIA in turn is further proteolyzed between Y251 and Y252 to generate IIIB. Lengths of the fragments (e.g., IV-K448) are based on mass determined by MALDI-MS and N-terminal amino acid sequence shown in Table 5. The C-terminal peptide B424-K448, although shown here as a single peptide for convenience, is in fact a mixture of several peptides (see Tables 4 and 5).
Figure 14. Scheme II. Steps in the self-proteolysis of BoNT/A LC in the presence of added zinc. Arrows show the sites of proteolysis. The fragments IIIB, IVA, and IVB correspond to the fragment designations in Figure 2. Unlike the steps shown in Scheme I, IA may bypass the C-terminal truncation and initial formation of IIIA but undergo proteolysis between Y251 and Y2S2 in directly forming IIIB. The fragment IVA is further cleaved into IVB. Although a C-terminal cleavage of IVB into IVC is possible, it was not observed here (see Figure 11) this species in the presence of added zinc. See Figure 11 and Scheme I for other explanations.

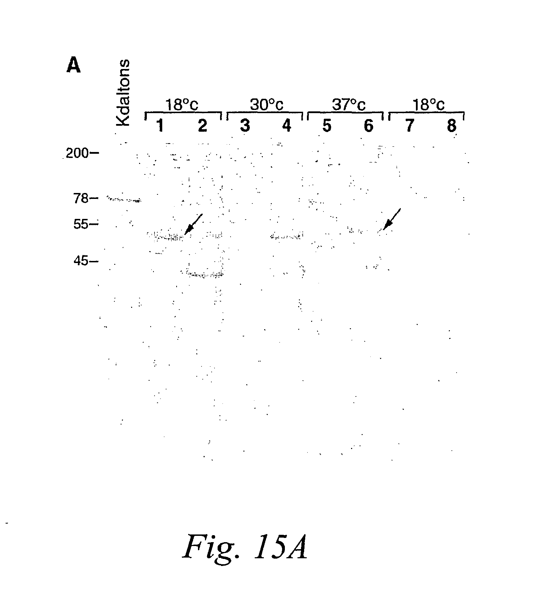

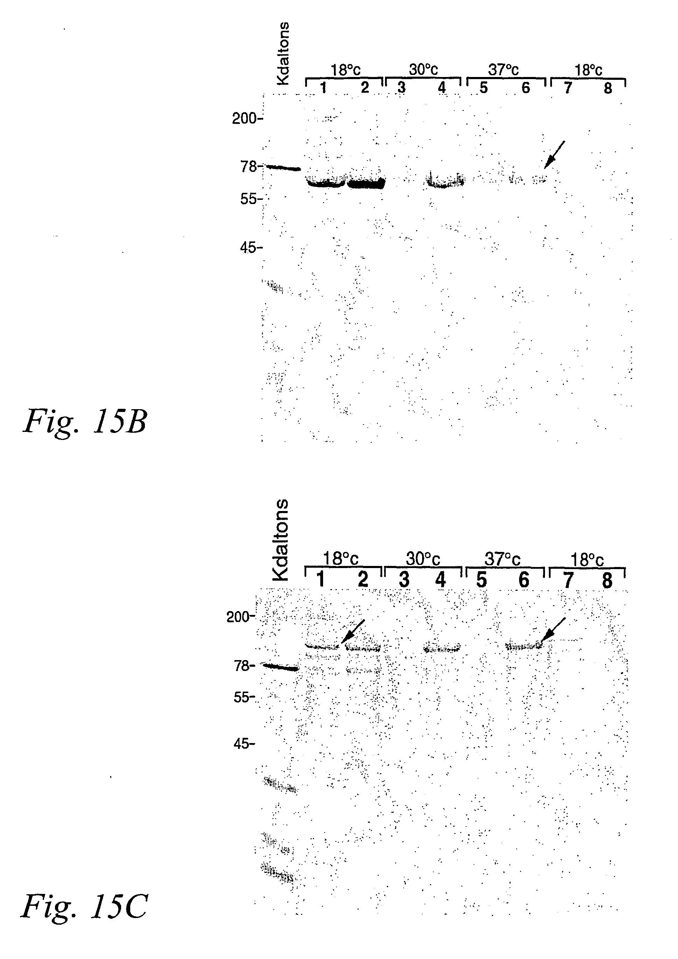

Figure 15. SDS-PAGE of (A) LCA, (B) LCA+Belt, and (C) LCA+Xloc, expressed at 18°C, 30°C and 37°C. Odd numbered lanes (1, 3, 5 and 7) are the soluble fractions and even number lanes (2, 4, 6 and 8) are the insoluble fractions. Lanes 7 and 8 are control cells with the plasmid lacking the insert Arrows show the expressed product at 18°C (soluble) and 37°C (insoluble).

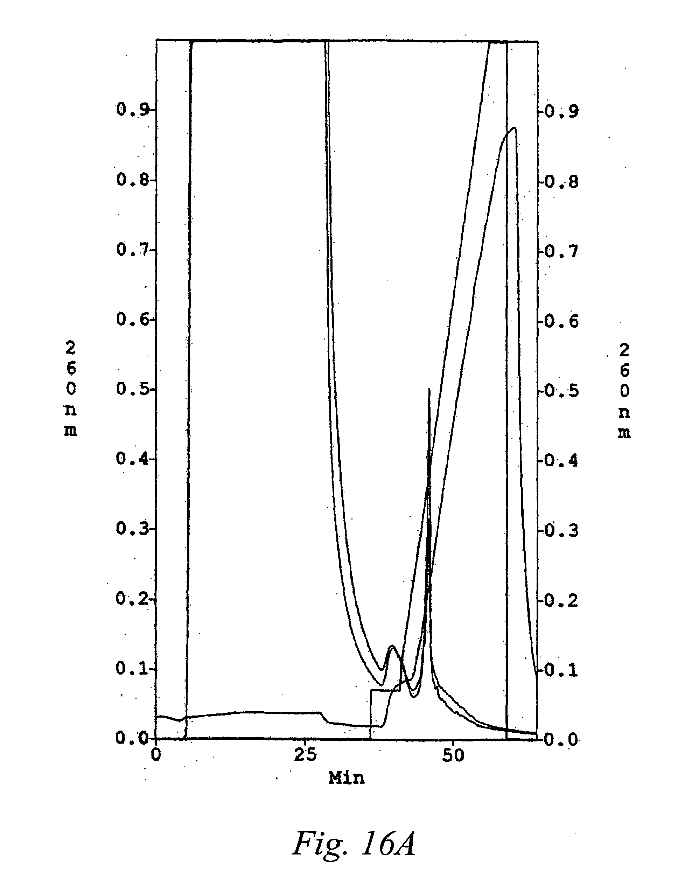

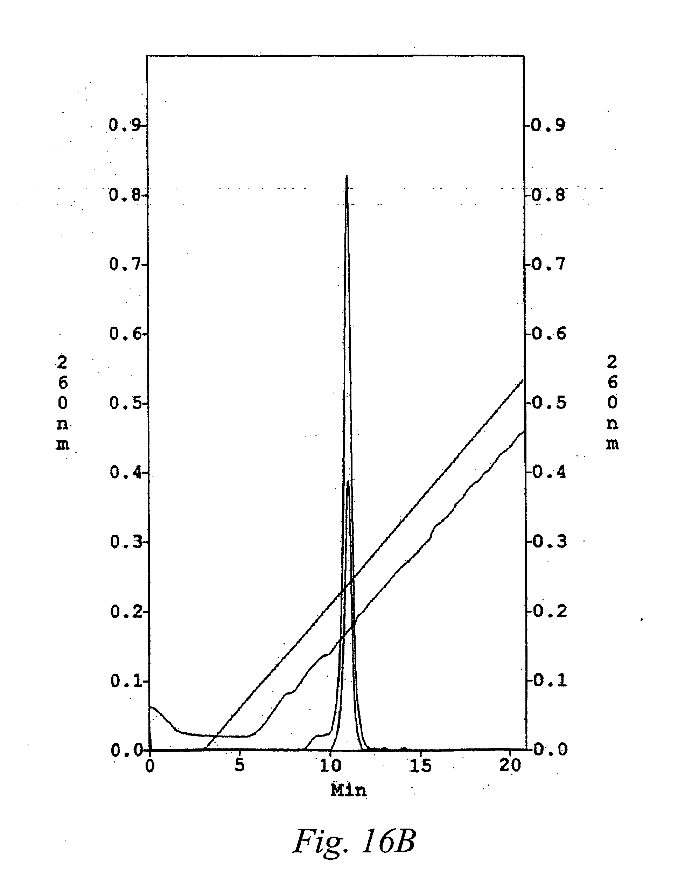

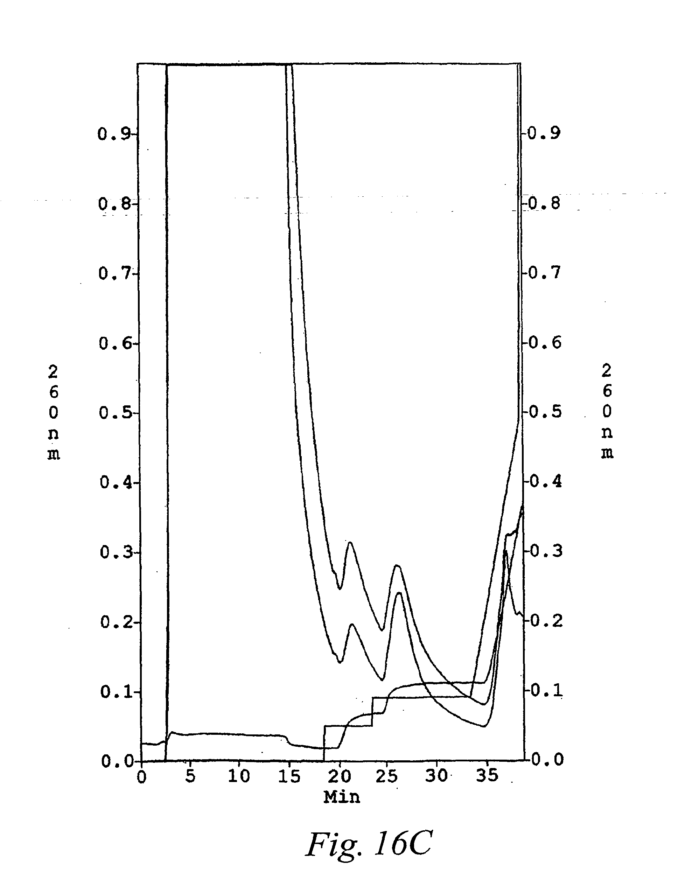

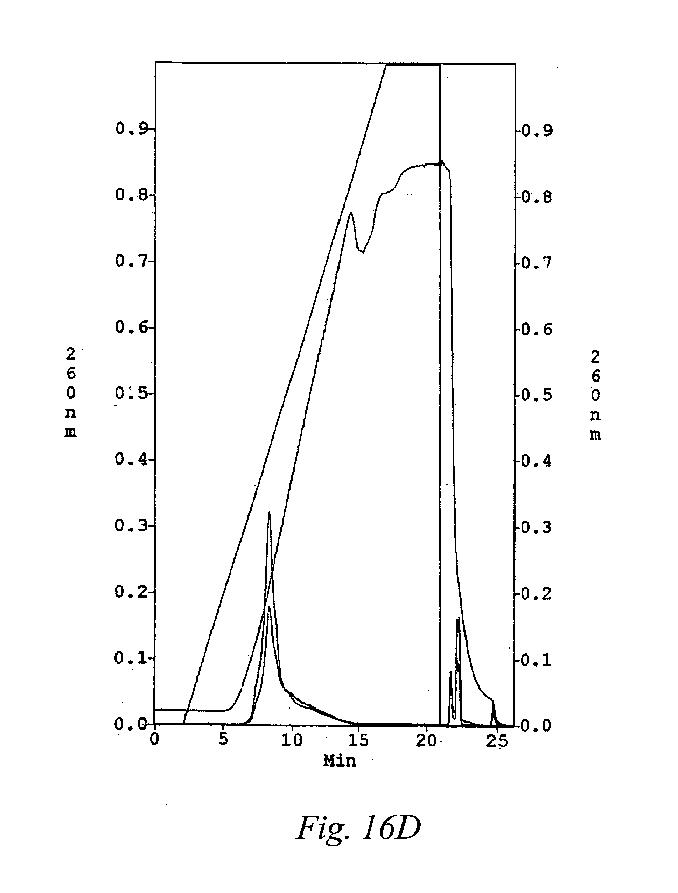

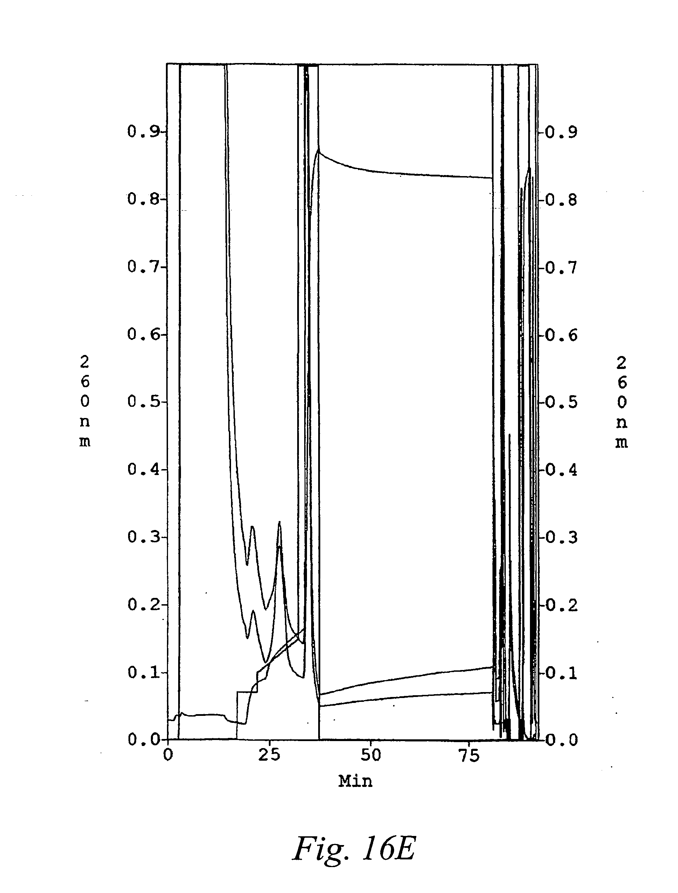

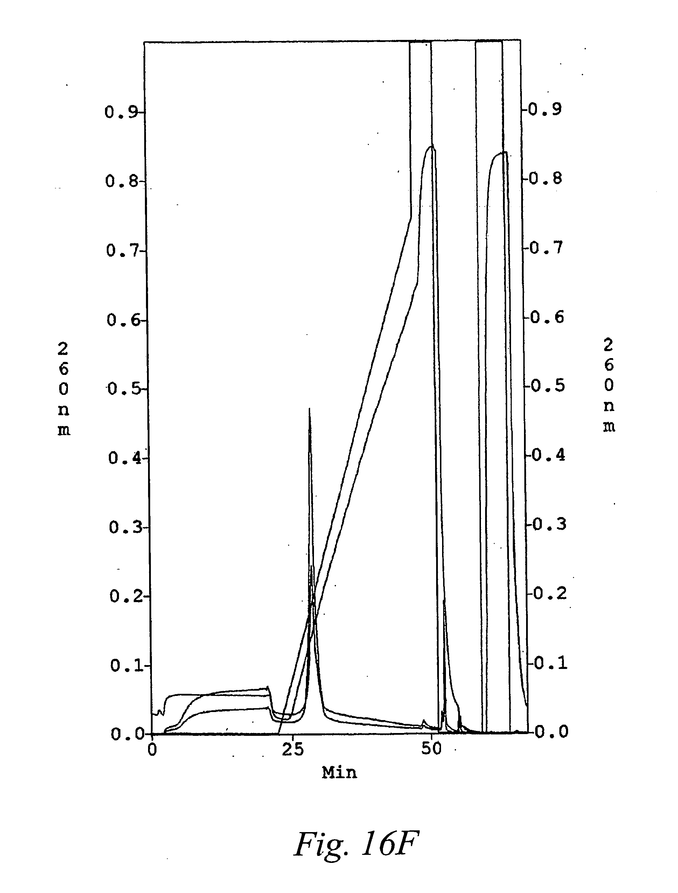

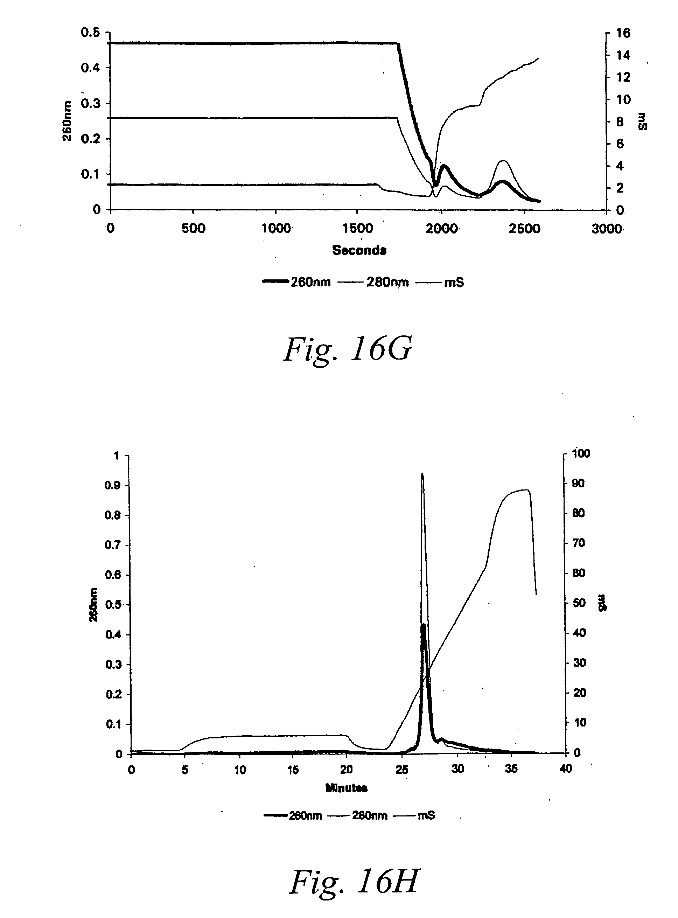

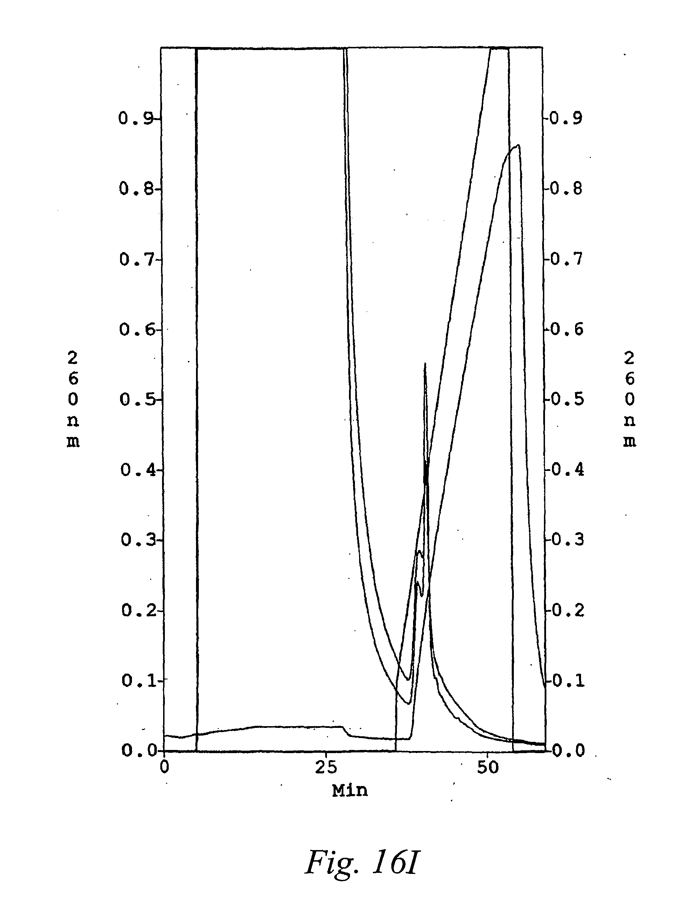

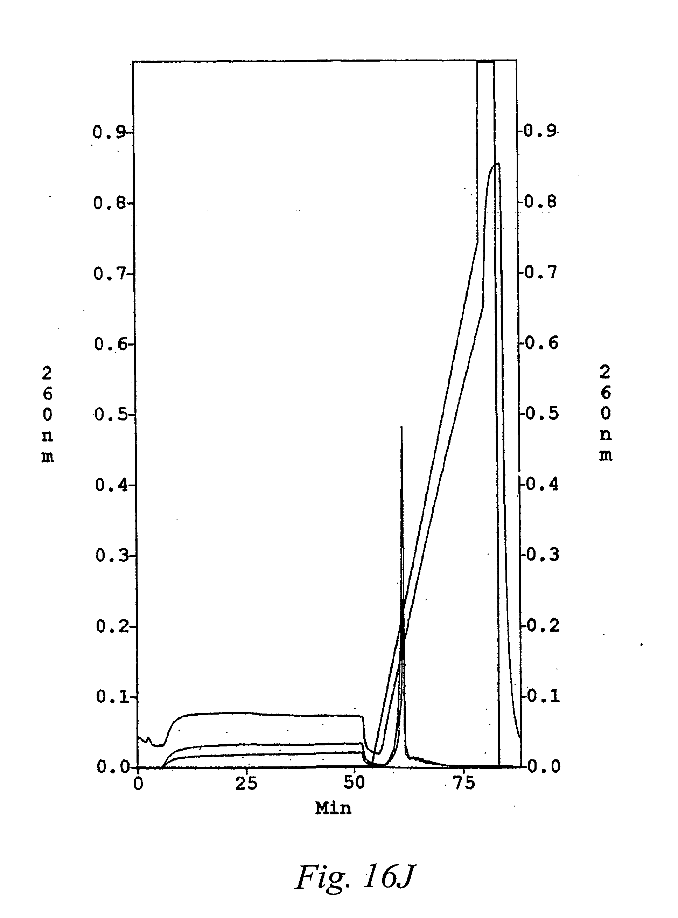

Figure 16 (Figure 2, Manuscript). HPLC elution profiles from HS column of LcA (A, B), LcA+Belt-(C, D), LcA+Hn (E, F), and LcB (G,H) and from a Source S column of LcA (I, J).

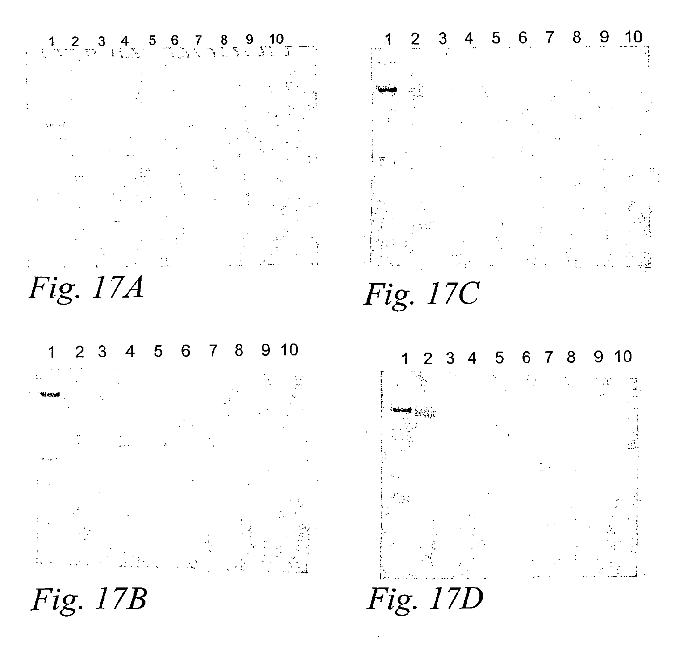

Figure 17. SDS-PAGE (A) and Western blots of purified LcA constructs using rabbit peptide sera against LcA (B), LcA+Belt (C) and LcA+Hn (D). Lanes from all figures are identical. Lane 1, Novex See Blue prestained molecular weight markers; Lane 2, purified BoNt-A; Lane 3, LcA-HIS; Lane 4, LcA-phosphate buffer; Lane 5, LcA-NaAcetate buffer; Lane 6, LcA+Belt; Lane 7, LcA+Hn, nicked; Lane 8, LcA+Hn, un-nicked; Lane 9, negative control pBT24a construct, no insert; Lane 10, LcB.

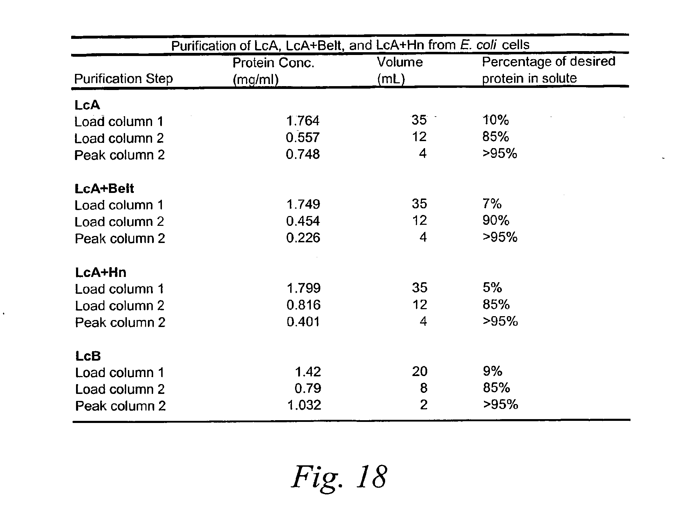

Figure 18. Mass spectrum for cleaved BoNT/A Lc.
Figure 19. Western blot using a monoclonal antibody to phosphorylated tyrosine.

### DETAILED DESCRIPTION OF THE INVENTION

In some embodiments the invention provides methods and nucleic acids for expressing *Clostridium botulinum* genes in other prokaryotes and eukaryotes. More specifically, the invention provides methods and nucleic acids for expressing botulinum neurotoxin (BoNT) light chains (LC) in *Escherichia coli* or *Pichia pastoris.* In order to be expressed in *Escherichia coli* or *Pichia pastoris,* the sequence of DNA encoding wild-type BoNT LC is engineered to replace some *Clostridium* codons that are rare or unrecognized in the host organism and to reduce the A+T content. The recombinant or synthetic DNA molecules of the invention are preferably designed with codon usage normally found in genes that are highly expressed in the host organism, e.g. *Escherichia coli* or *Pichia pastoris.* By selecting codons rich in G+C content, synthetic DNA molecules may also be designed to lower the A+T-rich base composition found in the Clostridial genes. According to the invention, a host cell is a cell of any organism other than Clostridium. Nonlimiting examples of host cells include gram negative bacteria, yeast, mammalian cells, and plant cells.

In some embodiments of the invention, upon expression of the DNA, a BoNT LC is produced in a heterologous host system by itself or as a fusion with another protein or a carrier. Proteins with which BoNT LCs may be fused include BoNT HCs, maltose-bonding proteins, other neurotoxins, neuropeptides, and autofluorescent proteins. A synthetic light chain gene may be genetically fused to a gene encoding a BoNT HC, producing recombinant botulinum toxin.

In some embodiments of the invention, BoNT LC is produced that is (i) substantially free of contaminating toxicity, (ii) moderately to highly soluble in aqueous media, (iii) stable for at least about six months at 4° C, (iv) catalytically active, (v) functionally active, or combinations thereof. In some embodiments of the invention, gram quantities of BoNT LC may be obtained per liter of culture medium. In some embodiments of the invention, a recombinant BoNT LC may reduce any immunological response that may result from the presence of binding proteins associated with the recombinant BoNT LC.

In some embodiments, the invention provides a nucleic acid encoding BoNT LC that substantially lacks catalytic activity as a zinc protease as measured by the SNAP-25 assay described in Examples 8, 17 and 25 below. In some embodiments, the invention provides nucleic acids that encode recombinant BoNT LC substantially lacking catalytic activity as a zinc protease, wherein amino acids in or spatially near the active site are deleted, replaced or modified relative to wild-type native BoNT. Catalytically inactive BoNT LC may be fused with BoNT HC to form a mutant recombinant holotoxin. Such holotoxins may be used to carry molecules, e.g., drugs, into cholinergic nerve cells.

In some embodiments, this invention provides a nucleic acid comprising a nucleic acid sequence encoding the N-terminal portion of a full length botulinum neurotoxin (BoNT) of BoNT serotype A, wherein said nucleic acid is expressible in a recombinant organism selected from *Escherichia coli* and *Pichia pastoris.* In some preferred embodiments, the nucleic acid corresponds in length and encoded amino acid sequence to the BoNT light chain (LC).

A synthetic DNA molecule of the invention designed by using *E. coli* codons is expressed fairly well in *P. pastoris.* Similarly, a synthetic gene using P. *pastoris* codons also appears to be expressed well in *E. coli.*

In some embodiments, this invention provides an expression vector comprising a nucleic acid of this invention, whereby LC is produced upon transfection of a host organism with the expression vector. Another embodiment of this invention provides a method of preparing a polypeptide comprising the BoNT LC of serotype A, said method comprising culturing a recombinant host organism transfected with an expression vector of this invention under conditions wherein the BoNT LC is expressed. Preferably, the recombinant host organism is a eukaryote. In another preferred embodiment, the method of this invention further comprises recovering insoluble protein from the host organism, whereby a fraction enriched in BoNT LC is obtained. *E. coli* is a preferred host for expressing catalytically-active (i.e., proteolytically-active) LC. *Pichia pastoris* is a preferred host organism for expressing inactive or mutated LC. *Pichia pastoris* has SNARE proteins which probably get inactivated by catalytically-active LC.

In some embodiments, the invention provides for the preparation of an immunogenic composition comprising a suitable carrier and the BoNT LC serotype A. Preferably, the immunogenic composition is prepared by culturing a recombinant organism transfected with an expression vector encoding BoNT LC. More preferably, the immunogenic composition is prepared by a method wherein an insoluble protein fraction enriched in BoNT LC is recovered from said recombinant organism. More preferably, the immunogenic composition is prepared by the method of Example 30.

According to some non-limiting embodiments, the invention provides reagents and compositions that are useful for developing therapeutic interventions against BoNT. For example, the recombinant BoNT nucleic acids and polypeptides of the invention produced therefrom may be used to screen for botulinum neurotoxin inhibitors.

In some embodiments, the invention is useful to provide therapeutic agents for clinical disorders such as dystonias, spasticity, and pain. According to these embodiments, the agents may be prepared by first expressing and purifying BoNT LC independently of any portion of the heavy chain. The BoNT LC so produced is then fused to the heavy chain or fragments thereof, e.g., HN and HC. Alternatively, BoNT LC may be coexpressed and/or copurified with BoNT HC or fragments thereof and then fused to BoNT HC or fragments thereof These agents may be used in clinical (human) or veterinary (non-human animal) applications.

In some embodiments, the invention is useful to provide agents that may be useful for treating disorders associated with cholinergic nerve function, SNAP-25, VAMP, syntaxin or combinations thereof. In some embodiments, the invention provides agents that may be useful for reducing any immunological response that may result from the presence of binding proteins associated with the agents. For example, the native BoNT holotoxin is highly immunogenic and some patients become refractory to continued treatment with it over time as their protective antitoxin titer rises. The efficacy of holotoxin-based drugs (e.g., BOTOX, Myobloc/Neurobloc, Dysport) may be improved by pretreating patients having a high titer of anti-holotoxin antibodies with a holotoxin fragment such as Lc, Hn, or Hc. These fragments may bind the anti-holotoxin antibodies making them unavailable for binding the subsequently administered holotoxin. This may work for a short time (months to a few years) realizing eventually that the antibody level may be built up so much that the drug can no longer be effective even with the addition of fragments. At this point in time, the patients will have to use a different serotype toxin drug or a chimera of the toxin (i.e., mixing toxin domains).

In further embodiments, the invention provides an immunogenic composition comprising a suitable carrier and a BoNT LC serotype A, prepared by culturing a recombinant organism transfected with an expression vector of the invention encoding BoNT LC. More preferably, the immunogenic composition is prepared by a method wherein an insoluble protein fraction enriched in BoNT LC is recovered from said recombinant organism.

The LC is present in immunogenic compositions of the invention in an amount sufficient to induce an immunogenic response thereto.

Two of the major advantages of the recombinant botulinum neurotoxins and fragments produced by the invention are the safety and high yields possible. First, the recombinantly-produced botulinum neurotoxin (rBoNT) protein fragments are completely nontoxic and are, thus, very safe. The fermentation of the host cell harboring the rBoNT gene (e.g., *Escherichia coli* or *Pichia pastoris*) does not require the high biological containment facilities presently needed to ferment the spore-forming *Clostridium botulinum* required for the production of the neurotoxin light chains. Second, synthetic DNA molecules of the invention can be placed in high expression systems and used to make much larger quantities of the BoNT fragments than toxin produced by the parent organism, *Clostridium botulinum.* Thus, there may be immense cost savings because it will be easier and safer to produce much larger quantities of the proteins for various uses including vaccination.

Synthetic DNA molecules as described herein may be transfected into suitable host organisms to create recombinant production organisms. Cultures of these recombinant organisms can then be used to produce recombinant BoNT fragments or holotoxins. Exemplary techniques for transfection and production of BoNT fragments are shown in the Examples. Alternative techniques are well documented in the literature *See. e.g*., Maniatis, Fritsch & Sambrook, "Molecular Cloning: A Laboratory Manual" (1982); Ausubel, "Current Protocols in Molecular Biology" (1991); "DNA Cloning: A Practical Approach," Volumes I and II (D.N. Glover, ed., 1985); "Oligonucleotide Synthesis" (MJ. Gait, ed., 1984); "Nucleic Acid Hybridization" (B.D. Hames & SJ. Higgins, eds., 1985); "Transcription and Translation" (B.D. Hames & S.J. Higgins, eds., 1984); "Animal Cell Culture" (R.I. Freshney, ed., 1986); "Immobilized Cells and Enzymes" (IRL Press, 1986); B. Perbal, "A Practical Guide to Molecular Cloning" (1984), and Sambrook, et al., "Molecular Cloning: a Laboratory Manual" (1989). Such techniques are explained fully in the literature. Modification of these techniques within the scope of this invention is within the skill in the art.

Recombinant forms of botulinum neutotoxin light chain may be useful in one or more of the following applications: strabismus and other disorders of ocular motility, dystonia, blepharospasm, cervical dystonia, oromandibular dystonia, laryngeal dystonia (spasmodic dysphonia), limb dystonia, hemifacial spasm and other facial dyskinesias, tremors of the head and heads, eyelid, cervical, and other tics, spasticity (e.g. anal), Stiff-Person syndrome, bladder dysfunction (e.g. in patients with spinal-cord injury), segmental myoclonus and other hyperkinetic disorders, cosmetic treatment of glabelar frown lines and other facial wrinkles, and all conditions characterized by hyperactivity of the lower motor neuron, See Cardoso and Jankovic, 1995 and references cited therein. The light chain may further be used to control autonomic nerve function (U.S. Patent No. 5,766,605) or tiptoe-walking due to stiff muscles common in children with cerebral palsy, according to findings published in the November 2001 issue of *Pediatrics.*

Absolute contraindications to the use of BONT are allergy to the drug and infection or inflammation at the proposed injection site whereas myasthenia gravis, Eaton-Lambert syndrome, motor neuron disease, and coagulopathy are relative contraindications (National Institutes Of Health Consensus Development Conference Statement On Clinical Use Of Botulinum Toxin 1991; Report Of The Therapeutics And Technology Assessment Subcommittee Of The American Academy Of Neurology 1990). Safety for use during pregnancy and lactation has not been firmly established (National Institutes Of Health Consensus Development Conference Statement On Clinical Use Of Botulinum Toxin 1991).

The invention contemplates the production of isoforms of the light chain as well as chimeras with other domains of the toxin or other proteins.

The invention contemplates the production of any protein or polypeptide that has biological activity/functionality similar to the wild-type botulinum neurotoxin light chain, e.g. cell binding, translocation across membrane, catalytic activity sufficient to inactivate critical proteins in a cell involved with protein trafficking, release of various chemical trasmitters (i.e., acetylcholine, glutamate, etc), hormones, etc.

For example, the light chain and translocation domain may be combined with a protein or peptide that targets a different receptor and/or cell-type: In addition, the invention contemplates therapeutic delivery of synthetic DNA molecules of the invention to cells via viral vectors such as adenovirus or other gene therapy techniques.

### EXAMPLES

In order to facilitate a more complete understanding of the invention, a number of nonlimiting Examples are provided below for illustration purposes only. To advance these purposes, the Examples are arranged in four sets: Examples 1-13, Examples 14-20, Examples 21-29, and Example 30.

### EXAMPLE 1

### Chemicals, Buffers, and Reagents

Buffer T (20 mM Tris-HCI, pH 9.2) and buffer G (50 mM sodium glycine, pH 9.0) were used as indicated. SKL (sodium N-lauryl sarcosine or sarkosyl) was from Sigma. Highly purified (>95%) full-length BoNT/A was purchased from List Biologicals (Campbell, CA). Rabbit polyclonal antibodies against a 16-residue N-terminal sequence (PFVNKQFNYKDPVNGV; SEQ ID NO:1) of the BONT/A LC were produced and affinity purified by Research Genetics (Huntsville, AL). Peroxidase-coupled goat anti-rabbit and anti-mouse IgG (H + L) and ABTS substrate were from Kirkegaard Perry Laboratories (Gaithersburg, MD). Oligonucleotides, designed for E. *coli* codon usage (Anderson and Kurland, 1990) and ranging in size from 70 to 100 nucleotides, were synthesized by Macromolecular Resources (Fort Collins, CO).

### EXAMPLE 2

### Construction and Expression of a Synthetic DNA Encoding rBoNT/A LC

The DNA encoding the enzymatic LC domain ofBoNT/A was assembled from three segments, a 335-base pair (bp) *Sal* I*-Sph* I fragment, a 600-bp *Sph I-Kpn* I fragment, and a 460-bp *Kpn* I *EcoR* I fragment. To construct the first segment, six oligonucleotide pairs were annealed, ligated, and, after PCR. amplification, inserted into pGEM3Zf at *Sal* I-*Sph* I restriction enzyme sites. The second segment was built by annealing and ligating eight oligonucleotide pairs, followed by its amplification and insertion into the *Sph* I and *Kpn* I sites of pGEM3Zf. The final segment was constructed by annealing and ligating six oligonucleotide pairs, followed by its amplification and insertion into the *Kpn* I*-EcoR* I sites of pGEM3Zf. Nucleotide sequencing of gene fragments in pGEM3Zf was performed to identify clones in each group with minimal misincorporations. *In vitro* mutagenesis was performed to correct the misincorporations in the BoNT/A LC minigene fragments. Directional gene assembly via 600-bp and 460-bp fragments in pGEM3Zf was followed by the insertion of the 335-bp fragment.

In the design of the synthetic DNA, the 5' oligonucleotide for amplifying the gene's 5' terminus consisted of an anchored *Sal* I site followed by an *EcoR* I site and an *Nco* I site to facilitate directional subcloning into the *E. coli* expression vector, pET24d. The 3' oligonucleotide contained a hexahistidine tag with a thrombin protease cleavage site for creating a carboxyl-terminal removable histidine tag. The 3' end also included the restriction enzyme sites for *BamH* I and *EcoR* I.

The full-length gene was excised from pGEM3Zf 5 with a *Nco* I*-EcoR* I and subcloned into a similarly digested pET24d vector. The resulting ligated construct was used to transform *E. coli* BL21(DE3) cells. Two clones were assayed for their ability to express rBoNTA LC. Single colonies were inoculated into 5 ml of Luria broth (LB) containing 50 µg/ml of kanamycin and grown overnight at 37°C. The overnight cultures (500 µL) were used to inoculate 50 ml of LB containing 50 µg/ml of kanamycin. When the cultures reached OD₆₀₀ of 0.8, induction was initiated by addition of isopropyl-β-D-thiogalactoside (IPTG) (final concentration, 1.0 mM). The cultures were induced for 2 hr at 37°C, harvested, and analyzed for expressed products on SDS-PAGE.

### Results

A synthetic DNA encoding rBoNTA LC was designed with *E. coli* codon usage, constructed, and expressed in *E*. *coli.* The native nucleic acid sequence from *C*. *botulinum* type A NTCC 2916 (Thompson *et al.,* 1990) was used as the template for preparing synthetic LC sequences of the invention.

At the 5' end of the DNA, an *Nco.* I restriction enzyme site was employed as a cloning site and palindrome to provide an initiation codon. The use of this *Nco* I site necessitated the use of a filler codon (GTT) between the Met initiation codon (ATG) and the codon (CAG) specifying the first amino acid residue in the LC (i.e., Q). This resulted in the introduction of one extra amino acid, Val, as the N-terminal residue (after the initiating Met). This extra and new amino acid, however, did not interfere with expression or activity (see later). The length of the LC (448 residues) to be expressed was chosen from the sequence of amino acids around the nicking site (DasGupta and Dekleva, 1990) (Figure 1). At the C-terminal end (i.e., DKGYNK; residues 444-449 of SEQ ID NO:5), a hexa-His tag was incorporated for affinity purification and a thrombin cleavage site (LVPRGS; residues 450-455 of SEQ ID NO:5) was incorporated for removing the hexa-His tag. The expressed protein therefore contained a total of 461 (1+448+6+6) residues (Figure 1 and SEQ ID NO:5). The synthetic gene thus constructed in pBT24d vector was highly and efficiently expressed in *E*. *coli,* accounting for about 25% of the total protein (Figure 2).

### EXAMPLE 3

### Fermentation

A frozen stock seed culture of recombinant *E. coli* harboring the synthetic DNA encoding the LC of BoNT/A was grown at 37°C to an OD₆₀₀) of 2.682 in a shake flask containing 100 ml of the following defined medium: casamino acids (1.4 g/L); yeast extract (2 g/L); (NH₄)₂SO₄ (1.85 g/L); K₂HPO₄ (30 g/L); MgSO₄•7H₂O (2 g/L); thiamine•HCl (0.015 g/L); glucose (18.1 g/L); trace elements solution (3 ml/L) consisting of FeCl₃•6H₂O, 27 g; ZnCl₂•4H₂O, 1.3 g, CoCl₂•H₂O, 2 g; Na₂Mo₄•2H₂O, 2g; CaCl₂•2H₂O, 1 g; CuCl₂•2H₂O, 1 g; H₃BO₃, 0.5 g; distilled H₂O, 1000 ml; and HCl, 100 ml. In addition, 0.0156 g/L of ZnCl was added to trace minerals to make the concentration of Zn five times greater in the shake flask and fermentor. Kanamycin (50 µg/L) was added as an antibiotic. The shake flask culture was used to inoculate a 5-L BioFlo III fermentor (New Brunswick Scientific, Edison, NJ) containing 4.3 L of the medium described above. Later in the growth (5.5 hr), 14.1 g/L of casamino acids was added and a glucose feed was initiated to maintain a glucose concentration of 1 g/L. Growth continued for 8 hr until an OD₆₀₀ of 49.9 was reached. Cell induction was then initiated at this time by adding IPTG (final concentration, 1.5 mM). Induction continued for 4 hr after adding IPTG, and cells (OD₆₀₀ of 112.62) were harvested by centrifugation (Beckman, Palo Alto, CA) at 7000 rpm for 15 min at 4°C. Cells were washed with cold 0.9% saline and centrifuged at 7000 rpm for min and frozen at -70°C. Wet cell yield was 58 g/L.

### EXAMPLE 4

### Extraction and Purification of Light Chain as Inclusion Bodies

In a typical preparation, 12 g of *E*. *coli* cells was suspended in a total volume of 30 ml of buffer T containing 5 mM MgCl₂, 1.5 mM PMSP, 10 mM β-mercaptoethanol, and 2 mg of DNAse. The cell suspension was subjected to 10 cycles of 2-min sonication (at 60% power in a Fisher Model 300 Sonic Dismembrator) and 2-min cooling on ice. After centrifugation for 15 min at 10,000 x g, the supernatant was discarded. The pellet was suspended in 30 ml the above buffer. The cycle of sonication and centrifugation was repeated five more times; MgCl₂ and DNAse were omitted from the buffer during the last two cycles. The resulting pellet contained the rBoNT/A LC, that appeared ~70% pure by SDS PAGE (Figure 2). The pellet was stored at 4°C as a white suspension in 15 ml of buffer T containing 1.5 mM PMSF and 10 mM β-mercaptoethanol.

### Results

The expressed LC appeared exclusively in the insoluble pellet fraction (Figure 2). Including MgCl₂ and DNAse in the cell suspension ensured a clean separation of the pellet from the supernatant after sonication and centrifugation. The white suspension of the purified BoNT/A LC migrated as a 52-kDa band and appeared to be ~70% pure on SDS-PAGE (Figure 2A), as determined by densitometric analysis. Minor contaminant bands with ~100-kDa, 37-40 kDa, and ~25 kDa also reacted with the antibody in the Western blot (Figure 2B). While fragments smaller than 50 kDa may have arisen from proteolysis of the LC (DasGupta and Foley, 1989), the origin of the 100-kDa species in the reducing SDS-PAGE gels is not clear since the species also reacts with the affinity-purified antibodies against a small sequence of the LC. Molecular mass determination by MALDI-MS gave 52.774 (+50) kDa as the predominant species along with minor species of 106.028 (±100) kDa and 25.00 (±25) kDa. Amino acid sequence determination of the LC identified V-Q-F-V-N-K-Q as the amino-terminal sequence, as expected for the constructed gene (Figure 1) and identical (with the exception of the penultimate valine) to that of the published sequence of BoNT/A (Thompson *et al..* 1990).

### EXAMPLE 5

### Solubilization of the Inclusion Bodies to Obtain Active rBoNT/A LC

In a typical experiment, 0.75 ml of the white rBoNT/A LC suspension (from an equivalent of 600 mg of wet cells) was centrifuged in a 2-ml Eppendorf tube and the supernatant was discarded. The pellet was suspended by mild sonication in 0.9 ml of 50 mM Tris-HCI, pH 9. A 20% solution (0.9 ml) of SKL in water was added to the suspension at room temperature and was mixed by inversion several times. Within 2 min, the pellet became completely soluble. Any remaining turbidity was cleared by further diluting with 50 mM Tris-HCl, pH 9.0, or was removed by centrifugation. The SKL-solubilized LC was dialyzed against 200 volumes of buffer G containing 1 mM DTT with one to two daily changes at 4°C for 1 week. The yield of the soluble rBoNT/A LC was 12 mg (3.9 mg/ml), which was stored in a glass tube at 4°C.

### Results

The purified inclusion bodies were solubilized in 10% SKL and the SKL was removed by dialysis against buffer G containing 1 mM DTT (see Section 2). The use of a 10% SKL solution ensured solubilization within 2 min of incubation, and the LC solution was immediately subjected to extensive dialysis to remove the detergent. Starting with an equivalent of 600 mg of the wet *E. coli* cells, 12 mg of the soluble LC was obtained, corresponding to 20 mg LC per gram of wet cells. This corresponds to a yield of 1.16 g of the pure protein per liter of cell culture.

### EXAMPLE 6

### Properties of the Purified BoNT/A LC

The UV-visible absorption spectrum (Figure 3) shows the rBoNT/A LC with a single maximum at 278 nm as a simple protein. Although a number of minor bands were observed in the SDS-PAGE gel (Figure 2), absence of any other absorbance bands in the UV-visible range suggests the absence of any nonmetal cofactor in the preparation. The LC was expressed as a C-terminally His-tagged protein. In the presence of 6 M GuHCl, the rBoNT/A LC was bound to Ni-resin and was eluted with immiadzole-containing buffers as a more purified form. Without GuHCl, the rBoNT/A LC did not bind to Ni-resin. This result suggests that the LC retained the His-tag after expression and purification, but in the absence of GuHCI, the His-tag was not exposed to solvent to chelate with the Ni-resin. Because the rBoNT/A LC had catalytic properties comparable to those of the dicchain (see below), removal of the His-tag from the purified protein was not attempted.

The purified LC was stable for at least 6 months when stored at 4°C in buffer G containing I mM DTT (Figure 4A). During this period, the protein remained fully soluble, did not show any degradation as analyzed SDS-PAGE, and retained its initial catalytic activity. An LC preparation obtained by prolonged solubilization in 0.5% SKL at room temperature, however, precipitated after 3 months of storage at 4°C and lost most of its initial catalytic activity. The LC (1 mg/ml of 50 mM Na-phosphate) precipitated from solution below pH 8 either at 4°C or at 25°C. Thermal stability of the LC (3.74 mg/ml of buffer G containing 1 mM DTT and 50 µM ZnCl₂) was investigated by incubating aliquots for 45 min at various temperatures. After cooling on ice for 45 min, the catalytic activities in the supernatants were measured. The midpoint of thermal unfolding Tₘ as measured by activity was 43°C (Figure 4B). At room temperature, increasing concentration of MgCl₂ also precipitated the LC from solution: at 6 mM MgCl₂, >80% of the LC precipitated.

### EXAMPLE 7

### Preparation of Apo-rBoNT/A LC

One milliliter of rBoNT/A LC (2.73 mg) was dialyzed overnight against 250 ml of buffer G containing 5 mM BDTA and 1 mM DTT. EDTA was removed by further dialysis for 60 hr against three changes of 250 ml of buffer G containing 1 mM DTT.

### EXAMPLE 8

### Assay of Proteolytic Activity of BoNT/A LC

BoNT/A cleaves the glutamyl-arginine bond between residues 197 and 198 of the 206-residue SNAP-25. Schmidt and Bostian (1995) showed that a synthetic 17-residue peptide representing residues 187-203 of SNAP-25 was sufficient for detecting endopeptidase activity of BONT/A and allowing routine assay for the neuotoxin activity. The peptide thus probably mimics the structure of SNAP-25 *in vivo* (Bi et *al*., 1995). The same peptide was used in an identical method to assay the proeolytic activity of the BONT/A LC.

The assay is based on HPLC separation and measurement of the nicked products from a 17-residue C-terminal peptide of SNAP-25 (Figure 5), corresponding to residues 187-203, which is the minimum length required for BoNT/A proteolytic activity (Schmidt and Bostian, 1995,1997). Unless otherwise noted, a 0.03-ml assay mixture containing 0.8-1.0 mM substrate, 0.25 mM ZnCl₂, 5.0 mM DTT, 50 mM Na-HEPES buffer (pH 7.4), and BONT/A LC was incubated at 37°C for 15-80 min. The amounts of uncleaved substrate and the products were measured after separation by reverse-phase HPLC (Waters) on a Hi-Pore C18 column, 0.45 x 25 cm (Bio-Rad Laboratories, Hercules, CA) with the Millennium software (Waters) package. Solvent A was 0.1% TFA and solvent B was 70% acetonitrile/0.1% TFA. The flow rate was 1.0 ml/min at 25°C. After the column was equilibrated with 10% B, the sample was injected, and the column was held at 10% B for 2.5 min. A linear gradient to 36% B over 21 min was followed by 100% B for 6 min. Kinetic parameters for the synthetic substrate were calculated from Lineweaver-Burk plots of activity with peptide concentrations from 0.26 to 1.7 mM.

### Catalytic Activity of the LC

The BoNT/A LC is zinc-endopeptidase specific for the cleaving the peptide bond between residues 197 (Glu) to and 198 (Arg) of SNAP-25. Incubating the 17-mer synthetic peptide representing residues 187-203 of SNAP-25 with the LC at 37°C for 5-200 min generated only two peptides (Figure 5). That no other peptide fragments were generated by this prolonged incubation proves that the contaminants present in the LC preparation were devoid of any proteolytic activity. Incubating the LC with BSA also failed to produce any proteolytic fragment. In contrast to the HoNT/A dichain, whose activity ruin is greatly enhanced by BSA (Schmidt and Bostian, 1997), the rate of cleavage of the synthetic peptide substrate was unaffected by the presence of BSA.

Proteolytic activity of the purified rBoNT/A LC linearly increased with the increasing amount of the LC in the reaction mixture. The time course of activity (at 0.8-1.0 mM substrate concentration), however, was not linear, but progressively declined, possibly due to a high Kₘ for the substrate peptide (see below). Therefore, routine assays depended on initial activities representing <30% substrate conversion.

Substrate *K*ₘ for the LC was fourfold lower than that reported for the dichain (Schmidt and Bostian, 1995). This may be due to shielding of the active site by a 'belt' from the translocation domain (Hₙ) in the dichain neurotoxin (Lacy *et al.*, 1998; Lacy and Stevens, 1999). Thus, the 'belt' may pose a steric hindrance for substrate binding by the dichain (high *K*ₘ). Nonetheless, the catalytic efficiency *k*_{cat}/*K*ₘ of the free rBoNT/A LC was somewhat higher than that of he dichain.

### Optimum pH, Salts, and Buffers

An optimum pH of 7.2 for the proteolysis of the synthetic substrate by the rBoNT/A LC was determined by assaying in three different buffer systems (0.1M) ranging in pH from 5.0 to 9.0 (Figure 6). For comparison, the optimum pH values of BoNT/B and tetanus neurotoxin, two members of the clostridial neurotoxin family, are 6.5-7.0, and 6.5-7.5, respectively (Foran *et al.,* 1994). Tris-HCI appeared to have an inhibitory effect on proteolysis, presumably due to chelation with the zinc at the active site. The activity at pH 7.4 was 25% higher in a 50 mM HEPES buffer than in 100 mM HEPES. Adding 50 mM NaCl, KCl, or NaPO₄ (pH 7.4) to the standard reaction mixture reduced activity 40-50%. Thus, high salt concentrations inhibited the proteolytic reaction.

### Effect of Metals and Thiol Reagents on Activity

BONT/A LC is a zinc-endopeptidase. Activity of the rBoNT/A LC was completely inhibited by including the metal chelator EDTA (1 mM) in the reaction mixture (Table 1). Adding low concentrations of ZnCl₂ (1-50 µM) in the assay mixture slightly stimulated the activity (5%-10%) and higher concentrations of ZnCl₂ inhibited the activity (Figure 7). The results suggest that the active site should be almost saturated with Zn²⁺ for optimum activity. The metal was tightly bound to the active site of the LC, as the extraction, purification, or dialysis buffers were devoid of Zn²⁺. Like Zn²⁺, other divalent metal ions, notably, MnCl₂ and NiSO₄, also inhibited the LC reaction to various extents in the absence of added thiol (Table 1). Addin 5 mM DTT to the reaction mixture neutralized the inhibitory effect of Zn²⁺ (Figure 7).

Neurotoxic or proteolytic activity of the dichain BONT/A probably requires an initial reduction of the disulfide bond between the LC and the HC (de Paiva *et al.,* 1993). Therefore, the proteolytic assay mixture of BONT/A with the synthetic or natural substrates were supplemented with 5-10 mM DTT (Washbourne et al., 1997; Schmidt and Bostian, 1995, 1997). In the absence of Zn²⁺, 5 mM DTT in the reaction mixture significantly inhibited the activity of the LC (Table 1 and Figure 7). Similarly, L-cys, dithioerythreitol, and glutathione inhibited the activity to various extents, while β-mercaptoethanol stimulated the activity in the absence of added Zn²⁺. These results were unexpected as the LC does not possess any disulfide bonds and the invariant Cys responsible for the interchain disulfide is far from the active site. One explanation for these effects is the formation of a mixed disulfide between a protein thiol and the exogenous thiol. To investigate the importance of a protein Cys residue on activity, several sulfhydryl reagents were incubated in the proteolytic assay mixture (Table 1). Both HgCl₂ and *p*-Cl-mercuric benzoate completly abolished the activity of LC. Preincubating the LC with these two reagents, then diluting with the proteolytic reaction mixture, also gave the same results. These results suggest the presence of a protein thiol in the vicinity of the active site of the LC.

**Table 1. Effect of Metal Ions and Thiols and Thiol Reagents on the Activity of the rBoNT/A LC**

| Thiol reagent | Concentration (mM) | % Activity | Metal reagent | Concentration (mM) | % Activity |
|---|---|---|---|---|---|
| None^{a} | | 100 | EDTA | 1 | 00 |
| Dithiothreitol | 5 | 45 | ZnCl₂ | 0.25 | 60 |
| Dithioerythreitol | 5 | 60 | - | 1 | 10 |
| β-Mercaptoethanol | 5 | 120 | - | 0.25 | |
| Glutathione, reduced | 5 | 75 | +Dithiothreitol | 5 | 125 |
| Glutathione, oxidixed | 5 | 75 | MnCl₂ | 1 | 40 |
| S-Nitrosoglutathione | 5 | 55 | MgCl₂ | 1 | 90 |
| L-Cysteine | 5 | 20 | CaCl₂ | 1 | 75 |
| *p*-C1-Mercuribenzoate | 0.050 | 00 | FeCl₃ | 1 | 35 |
| Mercuric chloride | 0.013 | 00 | CoCl₂ | 1 | 90 |
| Iodoacetamide | 10 | 80 | CuSO₄ | 1 | 95 |
| | | | NiSO₄ | 1 | 55 |

| | | | | | |
|---|---|---|---|---|---|
| ^{a} The reaction mixture contained only the substrate and the rBoNT/A Lc. Other conditions are as described in Examples 8-20. | | | | | |

### Steady-State Kinetic Parameters

The dependence of reaction rates on the substrate concentration was determined at 0.26-1.7 mM substrate at pH 7.4. A double reciprocal plot of the reaction rates versus substrate concentrations (Figure 8) yielded a *K*ₘ of 1.18 mM and a *V*ₘₐₓ of 1670 (equivalent to 2390 considering a 70% pure LC) nmol/min/mg LC (*k*_{cat} = 1.39/sec or 1.99 if 70% pure). For comparison, the maximum rate of cleavage of the peptide substrate by the native, dichain toxin is reported to be 1900 nmol/min/mg (*k*_{cat} = 4.7/sec), while the *K*ₘ is 5 mM (Schmidt and Bostian, 1997). The lower *K*ₘ for the LC may be due to a more exposed active site in the free LC than in the LC of the dichain, where the active site is shielded from the solvent by elements of the membrane-spanning domain *H*_{N} (28-29). The catalytic efficiency *k*_{cat}/*K*ₘ of the rBoNT/A LC,1.18 (1.69 if 70% pure), is thus higher than that of the dichain, 0.94 (Schmidt and Bostian, 1995, 1997).

### Apo-BoNT/A LC

The rBoNT/A LC was incubated with the metal chelator EDTA and after extensive dialysis, the activity of the apo-BONT/A LC was measured in the standard reaction mixture. In the absence of any exogenous Zn²⁺ or thiol, the preparation had 17% activity of the holo-BONT/A LC from which the apoprotein was made (Table 2). This result suggests that the bound Zn²⁺ was not completely removed by the EDTA treatment and dialysis. Nonetheless, adding 5 mM DTT and 250 µM ZnCl₂ to the assay mixture restored 70% of the activity of the holo-LC. Moreover, in the presence of 5 mM DTT and 250 µM MnCl₂, MgCl₂, or CaCl₂, 20-30% of the original activity was restored.

**Table 2. Activities of the Apo-BoNT/A LC With and Without Addition of Divalent Metal long to the Reaction Mixtures**

| LC form | Divalent metal | % Activity | % Activity recovered^{a} |
|---|---|---|---|
| Holo-LC | +Zn²⁺ | 100 | - |
| Apo-LC | +None | 15 | - |
| | +Zn²⁺ | 70 | 65 |
| | +Mn²⁺ | 20 | 10 |
| | +Mg²⁺ | 20 | 10 |
| | +Ca²⁺ | 30 | 20 |
| | +Fe²⁺ | 0 | - |

| | | | |
|---|---|---|---|
| ^{a}Represents percentage of the lost activity of Zn-free apo-rBoNT/A LC that was recovered by adding the indicated metal ions. | | | |

### EXAMPLE 9

### Vaccination of Animals

Purified rBoNTA LC was tested for its ability to elicit protective immunity in Cr1 :CD-1 (ICR) male mice (Charles River) weighing 16-22 g. Two concentrations of recombinant LC (5 and 15 g) with and with-out adsorption to a 0.2% Alhydrogel (Superfos Biosector, Kvisgaard, Denmark) were administered in 0.9% saline in a total volume of 100 µl. Groups of 10 mice including a naive control (saline alone) received three doses ofLC at 0, 2, and 4 weeks. Mice were bled from the retroorbital sinus 12 days postvaccination and their antibodies assayed for titers to toxin. Animals were challenged with native BoNT/A dichain toxin 15 days postvaccination.

The animal room was maintained at 21±2° C with a relative humidity 30-70%, a 12/12-hr light/dark cycle with no twilight, and 10-15 air changes/hour. Mice were housed in solid-bottom, polycarbonate Micro-Isolator^{™} cages (Lab Products, Inc., Seaford, DE) with paper chip bedding (Alpha-Dri^{™}, Shepherd Specialty Papers, Inc., Kalamazoo, MI) and provided food (Harlan Teklad diet No. 7022, NIH-07) and water *ad libitum.* All procedures were reviewed and approved by the Institutional Animal Care and Use Committee and performed in an AAALAC International-accredited facility in accordance with recommendations in the Guide for the Care and Use of Laboratory Animals, 1996 (National Academy Press, National Academy of Sciences, Washington, D.C.).

### EXAMPLE 10

### ELISA

Highly purified (>95%) BoNT/A toxin was diluted to 2 µg/ml in phosphate-buffered saline (PBS), pH 7.4 (Sigma Chemical Co., St Louis, MO) and was dispensed (100 µl/well) into microtiter plates (Immulon 2, Dynatech Laboratories, Chantilly, VA). The plates were incubated overnight in a humidity box at 40°C. Five percent skim milk (Difco, Detroit, MI) in PBS with 0.01% Thimerosal® was used to block nonspecific binding and as an antibody diluent. The plates were washed with PBS plus 0.1% Tween 20 between each step. Mouse sera were initially diluted 1:100 and then diluted fourfold for a total of eight dilutions (1:100 to 1: 1,600,000). Diluted sera were added in duplicate to toxin-coated wells (100 µl/well). The secondary antibody was horseradish peroxidaso-conjugated, goat anti-mouse IgG diluted 1:1000. The primary and secondary antibodies were incubated 90 and 60 min, respectively at 37°C. ABTS substrate (100 µl/well) was added as the color developer. The plates were incubated at room temperature for 30 min. The absorbance was measured with a microplate reader at 405 nm. A mouse monoclonal antibody, 5BA2.3, was used as the positive control in each assay; naive mouse serum was added as a negative control in each assay. The titer was defined as the geometric mean of the ELISA titer to BoNT/A toxin.

### EXAMPLE 11

### Biological Effects of the rBoNT/A LC

LC prepared from dichain BoNTs always had residual toxicity due to some contaminating dichain forms (Maisey *et al.,* 1988). To demonstrate and confirm that the rBoNT/A LC was nontoxic, 5-15 µg of the LC was injected per mouse, a dose that was 15,000-45,000 times higher than an equivalent lethal dose of the BoNT/A dichain. Table 3 shows that all the mice survived three successive injections. All of their antisera had high titers against BoNT/A, but these antibodies failed to protect the animals upon subsequent challenge with relatively low doses (10² LD₅₀) of the toxic BoNT/A dichain. Even when the ELISA titers were boosted 20-fold by using the aluminum hydroxide adjuvant, the animals were not immune to modest levels of BoNT/A challenge (Table 3). Comparable vaccination with BoNT/A Hc protected animals from challenge with as high as 10⁶ LD₅₀ (Smith, 1998). These results clearly demonstrate that the rBoNT/A LC was nontoxic to the animals and confirms earlier observations that LC does not possess any neutralizing epitope(s) (Chen *et al.,* 1997; Dertzbaugh and West, 1996).

**Table 3. Survival of Mice After Vaccination with the rBoNT/A LC and Subsequent Challenge by BoNT/A Dichain**

| Dose*^{a}* | | Survival at given BoNT/A dichain challenge^{c} | |
|---|---|---|---|
| (µg/mouse) | ELISA Titer*^{b}* | 10²LD₅₀ | 10³LD₅₀ |
| 0*^{a}* | <100 | 0/5 | 0/5 |
| 5*^{d}* | 18,000 | 0/10 | 0/10 |
| 15*^{d}* | 63,100 | 0/10 | 0/10 |
| 0*^{e}* | <100 | 0/5 | 0/5 |
| 5*^{e}* | 985 | 0/10 | 0/10 |
| 15*^{e}* | 2800 | 0/10 | 0/10 |

Although the LC by itself is nontoxic, in digitonin-permeabilized chromaffin cells (Bittner *et al.,1989)* and direct microinjection into the cytosol of sea urchin eggs (Bi et *al.,* 1995; Steinhardt *et al.,* 1994), it blocks membrane exocytosis. To demonstrate that the rBoNT/A LC preparation retained this property of inhibiting membrane exocytosis, sea urchin eggs were microinjected with the LC. Eggs of the sea urchin, *Lytechinus pictus,* are an excellent model system for the study of exocytosis. Unfertilized eggs have a layer of vesicles, the cortical granules, docked at the plasma membrane. The SNARE complexes of docked vesicles are inaccessible to the BoNTs. Thus, plasma membrane resealing of the unfertilized sea urchin egg is unaffected by microinjection with botulinum toxins A, B, and C1 (Bi *et al.,* 1995; Steinhardt *et al.,* 1994). Fertilization triggers exocytosis of the cortical granuoles. After fertilization, the vesicles available for exocytosis are largely undocked and the docking proteins of undocked vesicles are susceptible to proteolysis by injected clostridial neurotoxins.

For fertilized eggs injected with rBoNT/A LC, about 100 min at 20°C was required to inhibit plasma membrane resealing after mechanical wounding with a glass micropipet. Eggs that successfully resealed showed a transient dye loss for about 1-2 min after micropuncture. Eggs that failed to reseal continuously lost dye and lost control of intracellular free calcium, leading to cell death. Five of five fertilized eggs wounded between 36 and 70 min after injection with the rBoNT/A LC resealed successfully, as did five of five unfertilized injected eggs. Six of six fertilized eggs wounded between 106 and 145 min after injection failed to reseal, indicating that the recombinant light chain actively inhibited exocytosis. Thus, the rBoNT/A LC had a similar effect as BoNT/B in inhibiting membrane exocytosis and resealing of plasma membrane of sea urchin eggs (Steinhardt *et al*., 1994).

### EXAMPLE 12

### Exocytosis Experiments

Plasma membrane resealing after micropuncture with a glass pipette requires calcium-regulated exocytosis (Bi *et al.,* 1995). This exocytosis is dependent on docking proteins (the SNARE complex) that are sensitive to proteolysis by the clostridial neurotoxins (Steinhardt *et al.,* 1994). Sea urchin (*Lytechinus pictus*) eggs were used to test the biological activity of the rBoNT/A LC. The microinjection medium contained 19 volumes of the rBoNT/A LC (3.7 mg/ml) in 45 mM potassium aspartate, 5 mM HBPES, pH 8.1, and one volume of 55 mM fura-2 in 100 mM KCI and 10 mM HEPES, pH 7.1. Injection levels were 5-10% of egg volume. The plasma membrane resealing after micropuncture with a glass pipette was monitored by recording the emission from fura-2 upon excitation at 358 nm (the calcium-insensitive wave-length).

### EXAMPLE 13

### Other Analytical Methods

Protein concentration was determined by BCA assay (Pierce) with bovine serum albumin (BSA) as a standard. Reducing SDS-PAGE with 10% tricine-gels (Novex) was according to Laemli (1970). The gels were stained with Coomassie brilliant blue. Western blots were prepared by using a primary polyclonal antibody against a 16-residue N-terminal sequence of BONT/A LC and a peroxidase-coupled goat anti-rabbit IgG (H+L) as the secondary antibody. Absorption spectrum at 25°C was recorded in a Hewlett-Packard 8452 diode array spectrophotometer. The N-terminal amino acid sequence of the BONT/A LC was determined by Edman degradation in an Applied Biosystems Precise Sequencer in the 0- to 20-pmol detection range. Molecular mass was determined by MALDI-MS in a PE Biosystems Voyager DE instrument Sinapinic acid was used as the matrix and the sample was spotted on a stainless steel plate that was not washed with water or TFA. Other conditions in the experiment were accelerating voltage 25,000 V, guide wire voltage 0.3%, and laser 2500.

### EXAMPLE 14

### Chemicals, Buffers and Reagents

Buffer P (50 mM Na-phosphate, ph 6.5) was used for Examples 14-20. TPEN and ZnCl₂ were from Sigma. Affinity-purified, peroxidase-coupled goat anti-rabbit and anti-mouse IgG (H+L) and ABTS substrate were from Kirkegaard Perry Laboratories (Gaithersburg, MD). The inhibitor peptide (Ac-CRATKML-NH₂) (Schmitd *et al.,* 1998) was synthesized and purified by Cell Essentials (Boston, MA).

### EXAMPLE 15

### BoNT/A LC Purification

The rBONT/A LC was expressed by low-temperature IPTG induction in *E*. *coli* BL21 (DE3) cells as a soluble protein from a synthetic gene in a pET24a-derived multicopy plasmid (Clontech, Inc.). Construction of the gene and expression of the protein as described (Ahmed and Smith, 2000 [J. Protein Chem. 19, 475-487]) was modified as follows : a stop codon replaced the histidine tag at the carboxy terminus of the gene, and induction and expression was at 18°C for 22-24 hr. The LC was purified to near homogeneity by NaCl gradient elution from each of two successive cation exchange columns (MonoS) in buffer P. Details of the expression and purification will be published elsewhere (manuscript in preparation). A typical preparation had a specific activity of 2-3 µmol/min/mg in cleaving the 17-residue substrate peptide (see later) when assayed in the presence of 0.25 mM ZnCl₂; in the absence of added zinc, activity was 50%. The purified LC was thus partially resolved of the bound zinc. The purified protein (1-4 ml) in buffer P was stored at -20°C. Under this condition, the protein remains stable and retains its catalytic activity for at least 1 year.

### EXAMPLE 16

### DS-PAGE, Transfer on PVDF Membrane, and Western Blot

SDS-PAGE under reducing conditions (Laemmli, 1970) was carried out on a 1-mm-thick 10% tricine gels (Novex) as described (Schagger and von Jagow, 1987). Samples were prepared in 0.4% SDS, 5% β-mercaptoethanol, 12% glycerol, and 450 mM Tris-HCl, ph 8.45, by boiling for 5 min. The running buffer contained 0.1% SDS in 0.1 M Tris-0.1M Tricine, ph 8.3. The gels were stained with Coomassie Brilliant Blue. Electrophoretic transfer of peptides from SDS-PAGE gels onto PVDF membrane used 10 mM CAPS-NaOH buffer, pH 11.0, containing 10% methanol as the transfer buffer. Protein bands on the PVDF membranes were visualized by 1 min of staining with Coomassie Brilliant Blue followed by destaining in 10% acetic acid-5% methanol. The stained bands were cut out from the dried membranes for amino-terminal sequence determination. Western blots on nitro-cellulose membranes were prepared using a primary polyclonal antibody against a 16-residue N-terminal sequence of BoNT/A LC and a peroxidase-coupled goat anti-rabbit IgG (H + L) as the secondary antibody (Ahmed and Smith, 2000).

### EXAMPLE 17

### Proteolysis Experiments

Before each experiment, aliquots of the protein were thawed to room temperature and were immediately passed through a PD-10 column to remove the EDTA. The protein was collected in buffer P and stored on ice. The EDTA-free BoNT/A LC was mixed with predetermined concentrations of ZnCl₂, EDTA, TPEN, or the inhibitor peptide (see later) and 20-50 µl was distributed in screw-capped Eppendorf tubes. The tubes were incubated at 4°C or at 22°C. The final concentration of the protein was 0.18-0.20 mg/ml in these incubation mixtures. At various time intervals an equal volume (20-50µl) of SDS-load buffer was added to a tube for SDS-PAGE analysis.

A 100 mM stock solution of TPEN was prepared in ethanol (95%). Stock solutions of the competitive inhibitor peptide Ac-CRATKML-NH₂ (Schmidt *et al.,* 1998) (5 mM), ZnCl₂ (1-4 mM), and BDTA (20 mM) were prepared in buffer P. Unless otherwise mentioned, final concentrations of these reagents in the incubation mixtures with the LC were TPEN 5 mM, EDTA 5 mM, peptide 1 mM, and ZnCl₂ 0.25 mM.

### Results: Cleavage and Fragmentation of BoNT/A LC

Figure 10 shows that the BoNT/A LC undergoes cleavage and fragmentation that increases with time. The intensity of the band representing the full-length LC with a polypeptide mass of~52 kDa (IA) gradually diminished with time and a new protein band of~50 kDa (IB) appeared in its place. The results suggest truncation of about 2 kDa mass from the full-length LC. In Western blots (Figure 10B), both IA and IB also reacted with a rabbit polyclonal antibody raised against a 16-residue amino-terminal sequence of LC. This result suggests that the truncation from the full-length LC must occur at the C-terminus. Indeed, amino-terminal sequencing of the isolated, truncated protein (see later) showed the amino terminus was intact. Interestingly, preservation of the N-terminus of full-length BoNT/A neurotoxin was also observed after its posttranslation modification in bacterial culture (DasGupta and Dekleva, 1990). As the truncated protein IB accumulated, a protein band of ~100 kDa (II) appeared that was detected easily in the Western blot (Figure 10B). Figure 10 also shows that at 2 weeks of incubation, the LC fragmented into IIIA + IIIB and IVC. The larger fragment (IIIA) above the 34-kDa marker was followed by a fainter fragment (IIIB) just below the 34-kDa marker. The results of this time course experiment also suggested that IIIB was formed from IIIA. Both of these fragments must represent the N-terminus of the LC, as they reacted with the antibody (Figure 10B). On the other hand, a much smaller fragment (IVC) moving faster than the 23-kDa marker was probably the C-terminal fragment, as it failed to react with the antibody (specific for the N-terminus of the LC) in the Western blot. The truncation and fragmentation shown in Figure 10 were independent of the batch of *E. coli* cell culture or the batch of purification of the LC.

### Results: Zinc Accelerates the Truncation and Fragmentation

The BoNT/A LC is known to be highly substrate specific. Therefore, the truncation of about 2 kDa from the C-terminus or fragmentation into larger fragments upon storage of the LC at 4°C described in Figure 10 might appear to be due to the presence of some contaminating protease in the LC preparation. However, no additional Coomassie-stained protein bands were detected when 0.4-4.0 µg of the LC was electrophoresed in the presence of SDS. BoNT/A LC is a zinc-endopeptidase. Figure 11 shows that when LC was incubated with 0.25 mM ZnCl₂, the rate of fragmentation was greatly increased so that the antibody-reacting fragment IIIB and an antibody-nonreacting fragment IVA appeared within 2 days of incubation (Figure 11A, B). Fragment IVB appeared later in the time course. Qualitatively, the results are similar to those in Figure 10 except that in the presence of ZnCl₂, the rate of fragmentation was higher, fragment IIIB was formed without showing the initial formation of IIIA, and initial formation of IVA gave rise to IVB. The rate enhancement by zinc could be partly due to formation of holo-LC from the partially Zn-resolved LC (see Section 2). Because there was no fragment IVC (Figure 10) detected in this experiment (Figure 11), zinc must also have a structural role in the LC. From the results shown in Figure 11A it is not possible to judge if the C-terminal truncation of IA in forming IB and dimerization in forming II precede the fragmentation into III and IV. However, in some other experiments, using a lower concentration of ZnCl₂, it was possible to show that formation of IIIB occurred before formation of IB and that fragmentation was the last event.

The rates of C-terminal truncation and fragmentation of LC either in the absence or in the presence of ZnCl₂ were much higher when incubated at 22°C than at 4°C. In fact, amino-terminal sequence was determined on the fragments generated by incubation at 22°C for 2 days only (see later).

### Results: Metal Chelator TPEN Inhibits Truncation and Fragmentation

As shown in Figure 11, if the C-terminal truncation and fragmentation of the LC was indeed dependent on the presence of zinc, removing zinc from the incubation mixture and from the active site of the LC would be expected to abolish the truncation and fragmentation events. However, zinc is very tightly bound to the active site of LC. Extensive treatment with 10 mM EDTA in the cold (Ahmed and Smith, 2000) or with 10 mM EDTA at room temperature (Li and Singh, 2000) failed to completely remove zinc from the active site of the LC. In agreement with these observations, including 10 mM EDTA failed to protect the LC from C-terminal truncation and processing (Figure 12A). In contrast, the metal chelator TPEN largely protected the LC from truncation and fragmentation (Figure 12A). It was also found that, at 1 mM TPEN, the LC showed no activity when assayed for 5 min. Because the incubation mixture with TPEN did not contain any exogenous metal or zinc, any chelation by TPEN must have involved the active-site zinc of the LC. These results also suggest that truncation and fragmentation of the LC upon storage 4°C or at room temperature were autocatalytic.

### EXAMPLE 18

### Separation of Peptides with HPLC and Their Characterization by ESIMS-MS

For mass and sequence determination, peptides were separated on an Agilent Technologies Series 1100 liquid chromatograph with a 0.8 x 100 mm Poros-2 R/H column (PerSeptive Biosystems, Inc.). The mobile phase was 0.1% formic acid (solvent A) and 80% acetonitrile in 0.1% formic acid (solvent B). The peptides were eluted with a linear gradient of 0-100% B over 15 min at a flow rate of 0.2 ml/min. The injection volume was 10 µl. The peptides were detected and structurally characterized on a Finnigan LCQ Deca mass spectrometer employing data-dependent MS/MS. Molecular mass was also determined by MALDI-MS with a PE Biosystems Voyager DE instrument. Sinapinic acid was used as the matrix, and the sample was spotted on a stainless steel plate that was not washed with water or TFA. Other conditions in the experiment were accelerating voltage 25,000 V, guide wire voltage 0.3%, and laser 2500.

### Results: Amino Acid Sequence of the Small Peptides Generated by C-Terminal Processing

To map the sites of proteolysis, the small peptides were isolated by ultrafiltration of a C-terminally truncated LC mixture. Amino acid sequences of these peptides were determined by BSIMS-MS (Table 4). The peptides with G433 at the amino terminus (peptide 4) and K438 at the carboxy terminus (peptide 5) indicated cleavage by a trypsin-like protease on the R432-G433 and K438-T439 bonds, respectively. Of these, only the lysyl bond at K438 was reported to be cleaved by a clostridial endogenous protease or by trypsin (DasGupta and Dekleva, 1990). However, a cleavage at the K444-G445 bond as reported before by an endogenous clostridial protease (DasGupta and Dekleva, 1990) was not detected. Neither was cleavage detected at K440-S441 or at K427-L428 bonds, the other potential sites of tryptic cleavage. Although these results indicated that the LC preparations did not contain a protease activity that could cleave at K427-L428, K440-S441, and K444-G445, it is equally possible that some of the small peptides generated by cleavage at these sites were lost during sample preparation. Interesting findings of this experiment (Table 4) are the peptides with N-terminus of T420 (peptide 1) and V431 (peptide 3), as the preceding residues at F419-T420 and C430-V431 bonds, respectively, are certainly not the sites of "tryptic" cleavage.

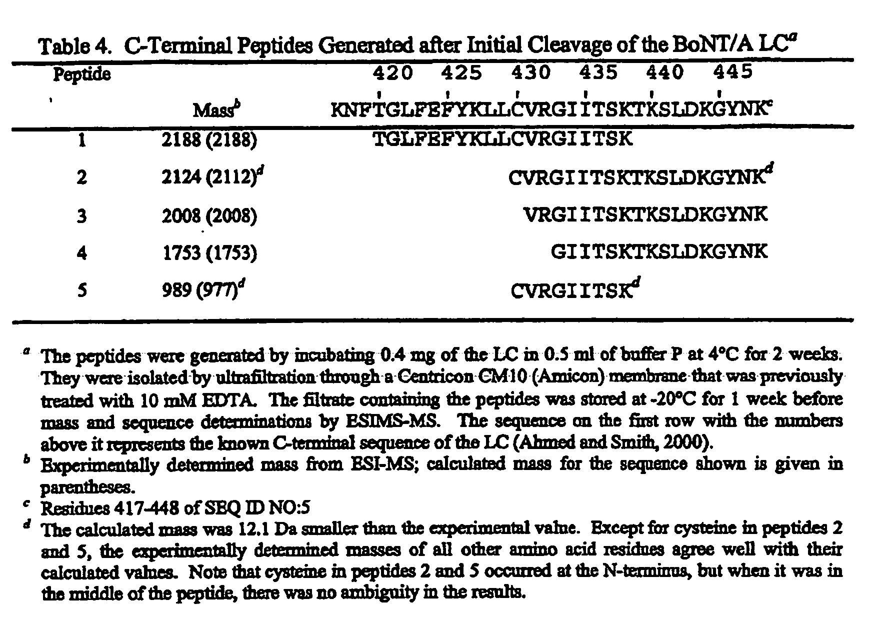

.

The sequence data from the ESIMS-MS results for the peptides 2 and 5 agree very well with the residue stretches V432-K449 and with the residue stretches V432-K449 and with the residue stretches V431-K438, respectively. However the experimentally determined mass for "C430," the residue at the amino side of V431 in both peptides, was greater by 12.1 Dalton than the theoretical mass for cysteine. At this stage, there is some uncertainty regarding the discrepancy in the mass of this "cysteine." Chemical modification experiments using iodoacetamide or acidified methanol failed to shift the masses of these peptides, indicating that the suspected "cysteine" did not have a free sulfhydryl group nor was a contaminating aspartic acid. Cysteine in proteins are known to occur as derivatives such as cysteine sulfenic acids (Ahmed and Claiborne, 1992; Claiborne *et al*., 1999). Attempts are being made to decipher the chemical nature of this "cysteine." If indeed it was a modified C430, cleavages at the carboxy ends of F419, C430, and V431 in addition to R432, K438, and K43 8 indicate that the proteolytic activity in this preparation was not "tryptic" in nature, but had a broad specificity.

### Results: Identity of the Large Peptides Generated by Fragmentation

The large peptides generated by fragmentation in the middle of the LC were identified by comparing the mass determined by MS with a calculated mass for a stretch of sequence based on the amino-terminal sequence determination (Table 5). Agreements between the experimental and calculated values were within 0.07%. Identity of IIIA as having a sequence range of V1-F266 was based on the kinetics of its (and of IVC's) appearance on SDS-PAGE (Figures 10 and 11) and N-terminal sequence of IVC. The sequence data along with Western blot results clearly demonstrated that the amino terminus of the LC (IA and IB) remained unchanged during the prolonged incubation period. Although the C-terminal sequence of the peptides IIIA and IIIB was not determined, N-terminal sequences of the peptides IV A, IVB, and IVC (Table 5) indicate that fragmentation of IA and IB (Figures 10 and 11) occurred by cleavage at the Y250-Y251 and F266-G267 bonds. Again, if the cleavages of these tyrosyl and phenylalanyl bonds were catalyzed by a protease, it must have been "nontryptic" in nature. Identity of the peptides IVB and IVC as having F423 at the C-terminal indicated that a C-terminal processing of the LC at F423-E424 remained undetected in the small peptide isolation experiment (see previous section). This result nonetheless supports that C-terminal processing occurred at phenylalanyl bonds in addition to lysyl, arginyl, valyl, and (most likely) cysteinyl bonds.

**Table 5. Identity of the Polypeptides Generated by Proteolysis of the BoNT/A LC**

| Peptide*^{a}* | Mass (Exp) | Mass (Calc) | Sequence range | N-terminal sequence |
|---|---|---|---|---|
| IA | 51,315 | 51,318 | V1-K448 | 2-VQFVNKQ |
| IB | 48,866 | 48,870 | VI-Y426 | 2-VQFVNKQ |
| II | 97,727*^{b}* | | | |
| | 97,870*^{b}* | | | |
| IIIA | n.d.*^{c}* | 32,270 | V1-F266 | 2-VQFVNKQ |
| IIIB | 28,111 | 28,130 | V1-Y251 | 2-VQFVNKQ |
| IVA | 23,207 | 23,207 | Y252-K448 | 252-YEMSGLE |
| IVB | 20,319 | 20,319 | Y252-F423 | 252-YEMSGLE |
| IVC | 18,400 | 18,400 | G267-F423 | 267-GGHDAKF |

| | | | | |
|---|---|---|---|---|
| *^{a}*Peptide designations are from Figures 10 and 11. Mass was determined by ESIMS-MS. Masses of the peptides IA and IB were determined separately. Peptides were generated by incubating the LC (1.8 mg/ml buffer P) alone or in the presence of 0.25 mM ZnCl₂ for 2 days at 22°C. Partial precipitation of the protein was visible after 1 day and was removed by centrifugation before ESI analysis. Masses of IIIB, IVA, and IVB were determined in samples containing ZnCl₂ and those of IA, IB, IIIA, and IVC were determined in samples with no ZnCl₂. Calculated masses are for the sequence ranges shown based on N-terminal sequence and mass data. The N-terminal sequences were determined separately for IA, IB, and IIIA in solutions and for IIIB, IVA, IVB and IVC on PVDF membrane after separation by SDS-PAGE and transfer on membrane. ^{b} Data from MALDI-MS determined in a sample containing IB with an initial concentration of 0.2 mg/ml. ^{c} Mass could not be detected in several experiments, probably due either to precipitation or to irreversible binding to column resin. Although a peptide with a lower mass can have slower mobility than a homologous higher mass peptide in SDS-PAGE due to charge differences (Ahmed *et al,* 1986), the kinetics of appearance of IIIB from IIIA (Figure 1) and their identification by N-terminal sequence determination suggest that IIIA must be larger than IIIB. Identity of IIIA as having a sequence of V1-F266 with a mass of 32,270 was based on N-terminal amino acid sequence determination and SDS-PAGE results (Figures 10 and 11). | | | | |

### EXAMPLE 19

### Other Analytical Methods

The enzymatic assay was based on HPLC separation and measurement of the nicked products from a 17-residue C-terminal peptide of SNAP-25 corresponding to residues 187-203 (Schmidt and Bostian, 1995). Initially protein concentrations were determined by BCA assay (Pierce) with bovine serum albumin (BSA) as a standard. After it was established by repeated measurements that a 1-mg/ml BoNT/A LC thus determined has A^{0.1%} (1 cm light path) value of 1.0 at 278 nm (0.98 at 280 nm), protein concentration was determined from absorbance at 278 nm. For comparison, the calculated *A*^{0,196} value of the LC at 280 nm in water (Pace *et al.,* 1995) is 0.948. Absorption spectra were recorded in a Hewlett-Packard 8452 diode array spectrophotometer. The N-terminal amino acid sequence of the LC was determined by Edman degradation in the Applied Biosystems Procise Sequences in the 0- to 20-pmol detection range.

### EXAMPLE 20

### A Specific Competitive Inhibitor of LC Activity Was an Effective Inhibitor of Truncation and Fragmentation

Autocatalytic truncation and fragmentation of proteins can arise from chemical catalysis and from enzymatic catalysis. To differentiate these two possibilities, a peptide specifically synthesized as a competitive inhibitor ofBoNT/A proteolytic activity (Schmidt *et al.,* 1998) was used. This peptide inhibitor, with a sequence of CRATKML (SEQ ID. NO:46), competitively inhibits the cleavage of a 17-residue substrate peptide based on SNAP-25 by BoNT/A neurotoxin with a *K*ᵢ of 2 uM (Schmidt *et al.,* 1998). At a 1 mM inhibitor peptide concentration, the LC showed no activity when assayed for 5 min. Figure 12B shows that when the LC was incubated with 1 mM peptide inhibitor, both C-terminal truncation and fragmentation at the interior ofLC were largely prevented. In the presence of the peptide inhibitor, however, the LC underwent a very slow cleavage, as can be expected in an enzymatic activity with a competitive inhibitor. Densitometric scanning of the gel showed that after 28 days, in the presence of the peptide inhibitor, less than 10% of the LC (IA) was converted into the C-terminally truncated form (IB). In contrast, in the absence of the peptide inhibitor, more than 80% of the LC (IA) was converted into the truncated form (IB). Results of this experiment prove that loss of 10-28 residues from the C-terminus of LC followed by fragmentation into two major peptides (Figures 10 and 11, Tables 4 and 5) occurred at the active site of the LC and that these reactions were enzymatic. The results also provide direct evidence that the cleavage reactions were not due to any contaminating protease in the preparation of the LC.

### EXAMPLE 21

### Materials

PCR-TOPO and 1-Shot cells were from Invitrogen. pBT24a plasmid and BL21 (DE3) cells were obtained from Novagen. All were prepared by standard methods. Proteins were visualized by SDS-PAGE and stained either with Coomasie or Colloidal Coomasie (Novex). Westerns (Novex) were reacted with a rabbit primary antibody (Research Genetics, Inc., Huntsville, AL) against the n-terminal 16 amino acids (PFVNKQFNYKDPVNGV) of the LC of type A and were visualized with a horseradish peroxidase conjugated goat anti-rabbit secondary anti-body and TMB peroxidase substrate (Kirkegaard and Perry Laboratories). Bacterial media was from Difco. Purification of the expressed proteins was on a Pharmacia model 500 FPLC system with programmed elution and A₂₈₀ monitoring (Pharmacia, Uppsala, Sweden). Columns were a Pharmacia HR 10/10 Mono S cation-exchange column, a Pharmacia Mono S 5/5 cation exchange column, and a Perseptive Biosystems POROS 20 HS cation exchange column. Pretreatment of the expressed proteins was with DNAse (Sigma, Inc.) and dialysis was with Pierce Slide-A-Lyzer 10k MWCO cassettes. The SNAP-25 substrate peptide (Quality Controlled Biochemicals, Hopkinton, MA) and its cleavage products were separated on a Hi-Pore C18 column, 0.45 X 25 cm (Bio-Rad Laboratories) and analyzed with the Millennium Software Package (Waters, Inc.). Src (p60c-src) recombinant phosphokinase, substrate peptide, and anti-phosphotyrosine monoclonal antibody 4G10 were from Upstate Biotechnology, Lake Placid, NY. [γ-³²P]ATP, 3000Ci/mmol, was from Dupont-NEN.

### EXAMPLE 22

### Preparation of recombinant neurotoxin clones

New restriction sites were added by PCR to the 5' and 3' ends (Nde1 and HindIII, respectively) of the synthetic DNA molecules coding for the Lc (M₁, to K₄₄₉), the Lc plus belt (LC+Belt; M₁, to F₅₅₀) and the Lc plus translocation region (LC+Xloc; M₁ to Q₆₅₉). PCR products were subcloned into pCR-TOPO and the sequences confirmed by DNA sequencing. The inserts were cut from the subcloning vector and ligated behind the Ndel site of pET24a, 80 as to begin expression with the initial methionine of the LC. The plasmid was transformed into *E. coli* BL21 (DE3) cells for expression.

### EXAMPLE 23

### Expression of neurotoxins

One hundred ml of Terrific Broth (TB) plus kanamycin was inoculated with the appropriate clone and grown overnight, with shaking, at 37°C. Fifty ml of LcA or 100 ml LcA+Belt and LcA+Hn of overnight growth was added to 1 liter TB plus kanamycin and shaking incubation continued at 37°C for an additional 1.25 hours. While cultures were placed on ice for 5 to 10 minutes, the OD₆₀₀ was read and adjusted to approximately 0.4 to 0.6, then IPTG was added to 1 mM for induction of protein expression. Duplicate cultures were grown at 37°C (4 hours), 30°C (10 hours) and 18°C (22 hours). At harvesting, the OD₆₀₀ was read again, cells were pelleted and frozen at -70°C if not used immediately. Data points are the mean of three separate measurements of the appropriate bands from SDS-PAGE gels scanned and digitally analyzed with an AlphaImager 2000 densitometer and AlphaImager Documentation and Analysis Software (AlphaInotech, San Leandro, CA).

### Expression at low temperatures markedly increases yields of soluble product, while addition of portions of the Hn does not increase the yield of soluble product

To study the effects of low temperature induction on the expression of LcA, expression was performed at 18°C, 30°C and 37°C. Figure 15A shows the decreasing solubility of LcA at these three temperatures, with concomitant decrease in the soluble product, from 55.5% at 18°C to 5.2% at 37°C. Yields of soluble LcA were highest at 18°C, with LcA making up approximately 10% of the cell protein. Addition of the belt and Hn portions of the neurotoxin to LcA did not increase solubility (Figures 15A, 15B and 15C), although addition of the full Hn region reduced expression and yield (Figure 15C).

Constructs were grown both in Luria Broth (LB) and Terrific Broth (TB), with no apparent difference in the quality or percent solubility of the products. Total yield was far greater for growth in TB, 17.97 g/l verses 7.77 g/l for LB. Optimal expression conditions for the Lc were considered to be the construct lacking either the belt or the Hn region at 18°C for 20-24 hours in TB.

### EXAMPLE 24

### Sample Preparation and Purfication of LC

One gram *E. coli* cell paste was resuspended into 20 ml of buffer A (20 mM NaAcetate, 2 mM EDTA, pH5.4). The suspended cells were disrupted by sonicating for 12 cycles of 30 seconds followed by 30 seconds of incubation on ice using a medium size probe at 65% output The resulting cell lysate was centrifuged (Sorval) at 15,000 x g for 15 minutes at 4°C to separate the proteins into soluble and insoluble fractions. The soluble fraction was diluted 1:1 in equilibration buffer B (20 mM NaAcetate, 2 mM EDTA, pH5.8) and used as starting material for the chromatography.

A HR 10/10 Mono S cation-exchange column was extensively cleaned between runs by sequentially running through it: 1 M NaCl through at 3 ml/min for 5 minutes; 20 mM NaOH for 10 minutes at 1 ml/min; 70% ethanol in ddwater for 30 minutes at 1ml/min; 1M NaCl in buffer B for 15 minutes at 1ml/min; then reequilibrated with buffer B at 2 ml/min for 5 minutes. The diluted lysate was then loaded at a flow rate of 2 ml/min (150 cm/h). The column was washed with 24 ml (3 bed volumes) of buffer B. Flow through and wash were collected separately and stored for subsequent analysis. Protein was eluted from the column with a linear gradient from 0 to 70% 1 M NaCl in buffer B over 8 minutes. Two-ml fractions were collected throughout the gradient. Fractions eluting between 10 and 22 mSiemanns (mS) were positive for rBoNTA(L_{c}) as shown by Western blot analysis. The pooled fractions were diluted 1:3 with buffer C (20 mM NaAcetate, 2 mM EDTA, pH6.2) and loaded onto a Mono S 5/5 cation exchange column equilibrated with buffer C at a flow rate of 2.5 ml/min. The column was washed with 10 ml (10 bed volume) of buffer C. Protein was eluted from the column with a linear gradient of 0-75% 1M NaCl in buffer C over 15 minutes. The rBoNTA(L_{c}) protein eluted from the Mono S column as a single band at 12 mS as shown by Western blot analysis. Fractions were pooled and stored frozen at -20°C in plastic vials. The product was greater than 98% pure as determined by SDS-PAGE.

The LcA+belt and the LcA+Hn were similarly purified, except that sonication was in buffer A (20 mM NaAcetate, 2 mM EDTA buffer, pH 4.8) and dilution was not necessary after centifugation to obtain the soluble fraction. After extensive cleaning of the column, the soluble fractions of either LcA+Belt or LcA+Hn were loaded at 2 ml/min onto a Poros 20 HS column equilibrated with buffer A. After loading, the column was rinsed at 3 ml/min with buffer A for 5 minutes and a 5% step of 1 M NaCl in buffer A was performed to remove interferring cellular products. The LcA+Belt was then eluted with a 9% step and the LcA+Hn eluted with a 10-14% step of 1 M NaCl in buffer A. Fractions were pooled, diluted 1:3 with equilibration buffer A and re-run on the HS column, eluting with a 1 to 75% gradient of 1 M NaCl in buffer A. Verification of the peaks was by Western blot and SDS-PAGE. Each protein was 95% or greater pure. Fractions were pooled and stored frozen at -20°C in plastic vials.

After the first column purification, aliquots of the expressed LcA+Hn were additionally nicked with trypsin at 10 g/ml overnight, at room temperature. This semi-purified protein lysate was then diluted and run on a second Poros HS column as described above. Protein was similarly 95% or greater pure.

Total protein concentrations were determined by using either a Bio-Rad Protein assay at ono-half volume of the standard protocol and bovine serum albumen as the protein standard or the Pierce BCA (bicinchoninic acid) protein assay with the microscale protocol as directed, with bovine serum alubumin as the protein standard.

### Purification of the Lc from the soluble faction of the lowest temperature expressed

Once conditions had been achieved for optimal yield of product, recovery of the Lc by simple cell sonication was deemed sufficient to release the protein. After removal of insoluble cell debris and proteins by centrifugation, this extract was directly loaded onto a cation exchange column and two isoforms of the Lc were observed to elute between 180 and 280 mM NaCl (Figure 16A). Western analysis of collected fractions showed two peaks reactive to antisera, corresponding to a full length Lc, and a Lc truncated by approximately 2.5 kDa. Since both forms were reactive to the amino terminus specific sera, a carboxy terminus truncation was indicated. The calculated pI for a Lc lacking the terminal 21 residues is 6.39, suggesting that it would be eluted at a lower NaCl concentration, as was observed. This difference in elution conditions allowed for a separate purification of each Lc isoform. The products eluted from the cation exchange chromatography column were observed to be approximately 70% pure, with a total protein concentration of 1.1 mg/ml.

The material was reloaded onto the Mono S column for further purification. The larger, non-truacated, LcA eluted as a single peak at 12 mS (Figure 16B). SDS-Page and western blot analysis showed only a single band at 51 k-Da (Figures 17A and 17B). The product was judged to be 98% pure after the final step and a protein determination determined the overall yield was 0.53 mg purified Lc per gram wet cells obtainable from our protocol.

The LcA+Belt eluted from the first column purification was approximately 85% pure, with a protein concentration of 0.454 mg/ml, in a total of 12 ml (Figure 16C). After purification on the second column, a 4 ml pooled peak (Figure 16D) had a concentration of 0.226 mg/ml, with 98% purity, producing a single band as observed by Western analysis (Figure 17A and 17C). The overall yield was 0.347 mg/g wet cells.

The LcA+Hn eluted from the first column purification was approximately 80% pure, with a protein concentration of 0.816 mg/ml, in a total of 12 ml (Figure16E). After purification on the second column, a 4 ml pooled peak (Figure 16F) had a concentration of 0.401 mg/ml, with 98% purity, forming a single band, while the nicked form of the construct produced two bands (Figures 17A through 17D) corresponding to the Hn and Lc. The overall yield was 0.617 mg/gm wet cells. The nicked form of the construct

### EXAMPLE 25

### Assay for cleavage of SNAP-25 peptide

A 17-residue C-terminal peptide of SNAP-25 (acetyl-SNKTRIDEANQRATKML-amide) shown to be the minimum length required for optimal BoNt/A proteolytic activity (Schmidt and Bostian, 1997) was used as the substrate in a cleavage assay as described previously (Ashraf etal, in press). Briefly, a 0.3 ml mixture containing 0.7-1.0 mM of the substrate peptide, 0.25 mM ZnCl₂, 5.0 mM DTT, 50 mM Na-HEPES buffer (pH=7.4) and purified LC (adjusted to produce 10-30% final cleavage) was incubated at 37°C for 15-180 minutes. The reaction was stopped with 0.09 ml of 0.7% trifluoroacetic acid. Quantitation of cleaved and uncleaved peptide was done by reverse-phase HPLC separation and the fraction of the peptide proteolysed was calculated by dividing the combined areas of the two cleaved peaks by the sum of the two product and uncleaved substrate peaks.

### Catalytic activity of the expressed constucts

Incubation of the 17-mer synthetic peptide representing residues 187-203 of SNAP-25 with the purified Lc at 37°C generates only two peptides cleaving between residues 197 (glutamine) and 198 (arginine). No other peptide fragments were generated by prolonged incubation, indicating that any contaminants in the Lc preparation lacked proteolytic activity. FPLC purification run #71, which was the complete Lc, resulted in a specific activity of 2.36 µmol/min/mg ofLc. Native BoNT/A in previous assays with the SNAP-25 synthetic peptide had a specific activity of 0.241µmol/min/mg (Schmidt and Bostian). Thus, the purified Lc produced had a specific activity increased by approximately 10-fold. Run #32 was the Lc+belt, and had an activity of 0.08 µmol/min/mg.

### EXAMPLE 26

### Determination of the length of the purified whole and truncated Lc

HPLC-purified samples were mixed with sinapinic acid and deposited on a stainless steel target. Mass spectra were acquired with a Perseptive Biosystems Voyager DE MALDI-TOF system. Data were obtained in delayed extration mode (750 ns delay) with a 337 nm nitrogen laser (3 ns wide pulse), using an acquisition rate of 2 GHz, 50,000 channels, an accelerating voltage of 25000,93% grid voltage, and a 0.3% guide wire voltage. Typically, 128 scans were averaged. The mass spectrometer was externally calibrated with myoglobin and bovine serum albumin.

The amino-terminal sequence of the expressed Lc was determined by automated Edman degradation performed on an Applied Biosystem Procise Sequencer (Applied Biosystems, Foster City, CA) in the 0-20 picomole detection range.

### Determination of the cleavage point for purified Lc

Purified Lc kept at-20°C in purification buffer with 2mM EDTA had no observable cleavage or truncation products (Figure 18A, lane 1). When the same product was placed at 30°C for I hour, the truncated Lc seen after the first cation exchange column passage was observed (Figure 18A, lane 2). Figure 18 contains the mass spectrum for cleaved BoNT/A Lc. The ion at mlz 49039.0 corresponds to the singly-charged molecule, whereas ions at m/z 24,556.9, and 98,280 correspond to doubly-charged and dimer species, respectively. The verified amino terminus for the Lc was VQFVNKQFNY, with the terminal methione removed, resulting in a peptide of 448 residues. The observed principal mass of 49,039 is approximately 2279 daltons less than the calculated mass for type A Lc, which represents a loss of 21-22 amino acids. Since the amino terminus specific antibody still reacts with the truncated molecule, cleavage occurred near the C terminus of the molecule. Because of mass uncertainty with MALDI-TOFMS (0.05% maximum mass accuracy for this instrument), it was not possible to positively identify the site of cleavage. Nevertheless, it was determined that cleavage occurred at either Y₄₂₆, K₄₂₇, or L₄₂₈. The most probable site of cleavage was between K₄₂₇ and L₄₂₈. Calculated mass for that product was 48,999, a difference of 40 daltons, which represents the best match to the observed ion and a mass accuracy to within 0.08%.

Addition of MgCl₂ to 125 mM and incubation for 1 hour at 30°C resulted in two cleavage products (Figure 18A, lane 3) after the Lc had lost the carboxy terminal residues. Amino terminus sequencing showed the cleavage to be between two tyrosines, Y₂₅₀ and Y₂₅₁.

### EXAMPLE 27

### Phosphorylation of purified Lc

Phosphorylation was at 30°C for 1 to 24 hours in a final reaction volume of 40 µL with 30 units c-src kinase. Non-phosphorylated samples were those in which enzymebwas omitted. The amount of Lc in the reaction was from 6.25 nM to 1.25 nM. The 4X buffer used for the reaction consisted of 100 mM Tris-HCl, pH 7.2,125 mM MgCl₂, 25 mM MnCl₂, 2 mM EGTA, and 2 mM DTT. ATP was at either 500 µM or 1 mM, with [γ-³²P]ATP added to a final concentration of 1 µCi/ul. In some cases, substrate peptide (KVEKIGEGTGVVYK; SEQ ID NO:3) at 93 µM was substituted for the Lc to act as a control. Reactions were stopped by freezing at - 20°C. Phosphorylated samples were run on SDS-PAGE gels, and either blotted and bands visualized with an antibody specific to phosphorylated tyrosine or the amino terminus of the Lc, or they were stained with Coomasie Blue, destained, dried and exposed to Kodak BioMax Light film.

### Phosphorylation of Lc

Purified Lc that was tyrosine phosphorylated resisted cleavage at the Y₂₅₀-Y₂₅₁ site (Figure 18B). During the initial 1 hour period of phosphorylation, the characteristic cleavage products were observed, but did not substantially increase over a 24 hour period of time. A possible explanation would be that phosphorylated Lc molecules were protected from cleavage, but not all of them could be modified prior to concurrent proteolysis. An identical reaction mixture lacking the enzyme showed rapid cleavage of the Lc, with very little remaining by 4 hours, and undetectable by 8 hours. A monoclonal antibody to phosphorylated tyrosine reacted to full length, src phorphorylated Lc, but not to either of the cleavage products in the phosphorylation reaction (Figure 19B), even though cleavage products were clearly visible by SDS-PAGE at all time points. The reaction lacking the enzyme showed no phosphorylated tyrosine bands of any size. Antibody to the amino terminus of the Lc reacted to the full length and larger of the cleavage products, plus three additional bands of between 60 and 75 kDa. These additional bands above the Lc were observed by SDS-PAGE for all the samples and appear to be SDS-resistant complexes of either the Lc or amino terminus fragment with other fragments. Autoradiographs of the phosphorylated and unphosphorylated (lacking enzyme) Lc (Figure 19C) show incorporation of [γ-³²P]ATP in the src phosphorylated full length Lc at 1 hour, with none observed in smaller or larger fragments, nor in samples lacking the enzyme. At 24 hours, very faint bands corresponding to the cleavage products did appear. These could either have arisen from cleaved, phosphorylated, full length Lc, or they may have been phosphorylated after they became fragments.

### EXAMPLE 28

### Immunity

Immunization of mice with the purified forms of the LcA, LcA+belt and LcA+Hn resulted in ELISA titers of between X and X for all construct forms. Protection was observed after challenge with 10² to 10³ MLD₅₀ of purified Type A toxin. See Tables 6-8.

| Table 6: Efficacy of Purified rBoNTA(LC+Belt) Solubly Expressed from *E. coli* to Elicit Protective Immunity in Mice | | | |
|---|---|---|---|
| Dosage^{a,b} | Toxin Challenge | | ELISA Titer |
| (µg) | (Survivors/Total) | | (GMT)^{c} |
| | 10² LD₅₀ | 10³ LD₅₀ | |
| 5 | 10/10 | 10/10 | ND |
| 15 | 10/10 | 10/10 | ND |
| Controls | 0/10 | 0/10 | ND |
| ^{a} Animals were vaccinated at 0, 2, and 4 weeks and challenged on week 6. | | | |
| ^{b} Specific activity (i.e., proteolytic activity) of the rBoNTA(LC+belt) immunogen was not determined. | | | |
| ^{c} Geometric mean of the ELISA titer to BoNTA neurotoxin (ND=not determined). | | | |

| Table 7: Efficacy of Purified rBoNTA(LC+Hn) Solubly Expressed from *E. coli* to Elicit Protective Immunity in Mice | | | |
|---|---|---|---|
| Dosage^{a,b} | Toxin Challenge | | ELISA Titer |
| (µg) | (Survivors/Total) | | (GMT)^{c} |
| | 10² LD₅₀ | 10³ LD₅₀ | |
| 5 | 5/9 | 1/9 | ND |
| 15 | 4/10 | 1/10 | ND |
| Controls | 0/10 | 0/10 | ND |
| ^{a} Animals were vaccinated at 0, 2, and 4 weeks and challenged on week 6. | | | |
| ^{b} Specific activity (i.e., proteolytic activity) of the rBoNTA(LC+Hₙ) immunogen was not determined. | | | |
| ^{c} Geometric mean of the ELISA titer to BoNTA neurotoxin (ND = not determined). | | | |

| Table 8: Efficacy of Purified rBoNTA(LC) Solubly Expressed from *E. coli* to Elicit Protective Immunity in Mice | | | |
|---|---|---|---|
| Dosage ^{a,b} | Toxin Challenge | | ELISA Titer |
| (µg) | (Survivors/Total) | | (GMT)^{c} |
| | 10² LD₅₀ | 10³ LD₅₀ | |
| 5 | 9/10 | 10/10 | ND |
| 15 | 9/10 | 10/10 | ND |
| Controls | 0/10 | 0/10 | ND |
| ^{a} Animals were vaccinated at 0, 2, and 4 weeks and challenged on week 6. | | | |
| ^{b} Specific activity of the rBoNTB(LC) immunogen was 21 mmol/min/mg using 0.8-1.0 mM substrate (VAMP peptide, residues 54-94). | | | |
| ^{c} Geometric mean of the ELISA titer to BoNTB neurotoxin (ND = not determined). | | | |

### EXAMPLE 29

### Discussion

The system of expression of the invention for botulinum neurotoxin Hc (Byrne et al, 1998) and Lc fragments using an optimized synthetic gene, has shown success in achieving high levels of product. In an attempt to produce a molecule that more closely resembles the natural state of the toxin, a cloning and expression scheme that would give a large amount of correctly folded, untagged, Lc was initiated. The two basic strategies employed were to (1) express the Lc at a lower temperature, a classic method for ensuring proper folding, and (2) adding on portions of the rest of the neurotoxin polypeptide, mimicking the natural expression within the clostridial host. As expected, reducing the temperature for induction dramatically increased the solubility of the expressed product from 5.2% at 37°C to 55.5% at 18°C for the Lc. The slower rate of expression at the lower temperatures was compensated for by increasing the length of time for expression. This did not result in increased degradation of the product intracellularly, prior to harvest and purification. Addition of the belt and Hn portions of the toxin had no effect upon solubility of the expressed gene, although each was easily expressed at the lower temperature.

Although cloned and expressed Lc has been available for Lc study, it has been purified with either glutathione or his-tags (Zhou, et al, 1995; Li and Singh, 1999). Previous investigators have used native toxin (Lacy et al, 1998) for x-ray crytallography studies, and it was an object of the invention to produce Lc as close to the native product as possible, e.g., without tags or modifications. For this reason, traditional column chromatography methods were used instead of affinity columns. The calculated pI of the Lc of 8.13 suggested that the Lc would efficiently bind to a cation exchange column. Upon passage over an initial Mono S column, the product appeared relatively clean, although a second immunoreactive band immediately beneath the proper, calculated size for the Lc was noted. After passage over a second cationic exchange column, this band was not observed on Westerns.

Using the above methods of low temperature expression and cation exchange purification, a large quantity of Lc was acquired for assessment of catalytic activity. Activity of the purified Lc was calculated to be approximately 10-fold greater than that of the native toxin. Previous investigators have shown that the Lc must be activated by proteolytic cleavage of the Lc from the Hc (DasGupta and Dekleva, 1990), although the two halves must both be present for efficient intoxication of cells. It is interesting that the Lc with the belt attached lacked the high level of catalytic activity seen with the Lc by itself. Presumably, the belt is wrapped around the Lc, as is observed in x-ray crystallography studies (Lacy et al, 1998). As the entire translocation region is not there to occlude the active site, it may be that the belt in some manner is constricting the Lc, or a conformational change is prevented that is required for full activation. Comparison of the crystallography structure of Lc of the invention with and without the belt would be worth further study.

Two interesting and unexpected pieces of data came from expression ofLc without purification tags. The first was the truncation of the Lc from the carboxy terminus by 20 residues. A recent paper by Kadkhodayan et al, 2000, notes that this portion of the Lc is not required for full catalytic activity. The truncation is intriguing as it removes the Lc/Hc di-sulfide bond at a lysine proximal to the involved cysteine. The two other proteolytic cleavages known to occur at the carboxy teminus of the Lc are also at lysine residues (DasGupta and Dekleva, 1990). Lysine proteolysis is common, with ubiquitin, a lysine specific proteolysis factor found conjugated to cell receptors of eukaryotes being one of the most common routes (Doherty and Mayer, 1992). It has long been hypothesized that the di-sulfide bond holding the Lc and Hc together was reduced as the Lc was transported into the cell, freeing it from the receptor binding portion (de Paiva et al, 1993). Although the ten residue portion flanked by lysine residues seems to be removed during activation "nicking" of the polypeptide, the cysteine residue was assumed to remain as part of the Lc. Work with native toxin and cells has been inititated to determine if the natural state of the toxin inside cells is one lacking the terminal 20 residues and cysteine.

### EXAMPLE 30

### Expression of BoNT LC

Reagents: Terrific Broth (Difco): 48 gm/liter with 4 ml of non-animal glycerol; autoclave 15 minutes. Store refrigerated. Kanamycin: stock solution is 50 mg/ml in distilled water, filter sterilized, store in aliquots at -20°C. Chloramphenicol: stock solution is 50 mg/ml in ethanol, filter sterilized, store in aliquots at -20°C. Add antibiotics to media just prior to use.

Expression of the Lc and Lc with Hₙ (translocation region) was performed for even numbered SEQ ID NOS:20-44 (only SEQ ID NO: 20 relates to BoNT/A LC). Expression was essentially the same for all constructs within the given parameters.

Cultures of BL21 (DE3) cells (See Example 22) were grown in Terrific Broth (TB) plus 50 µg/mL kanamycin. Cultures of BL21(DE3) Codon Plus cells were grown in TB plus 50 µg/mL kanamycin and 50 µg/mL chloramphenicol. Cultures grown overnight at 37° C while shaking at about 200 to about 250 rpm were diluted 1:20 with fresh antibiotic-containing media. Diluted cultures were returned to overnight growth conditions (37° C, shaking at 200-250 rpm) for 1¼ to 2½ hours. An optical density measurement was taken while the cultures were placed on ice for 5 minutes. Preferably, the OD₆₀₀ is between about 0.4 and about 0.6. The incubation time may be extended and/or fresh antibiotic-containing media may be added if the OD₆₀₀ is lower than 0.4 or higher than 0.6.

Next, sufficient IPTG was added to each chilled culture to make the concentration about 1 mM. IPTG-containing cultures were incubated about 24 to about 26 hours at 18°C and shaking at about 200 to about 250 rpm. An optical density measurement was taken at the end of this incubation. Preferably, the OD₆₀₀ is between about 1.7 and about 2.1.

Cultures that satisfied this criteria were centrifuged at about 3000 rpm for about 20 minutes to obtain a cell paste for purification. The cell paste may be stored at -20° C until ready for use.

Aliquots of 1 mL each were pelleted in a microfuge, resuspended in 1 mL of sonication buffer, and sonicated 12 X 30 seconds on ice over 12 minutes. Sonicated cells were microfuged for 10 minutes. The supernatant was aspirated and retained as the soluble fraction. 1 mL of 6M urea was added to each pellet and retained as the insoluble fraction. Appropriate amounts run on by SDS-PAGE should show approximately 50% soluble, 50% insoluble, at about 51 kDa. A western with rabbit anti-Lc sera will be at the same location.

### Purification of BoNT LC

Cell paste was resuspended at 1g/20 mL sonication buffer, sonicated 10X, 30 seconds on, 30 seconds off, on ice. Insoluble material and debris was pelleted by centrifuging for 10 minutes at 12,000 rpm (e.g.in a microfuge), decanting solute, and repeating one time in a fresh tube. The supernatant was decanted into a fresh tube. An equal volume of equilibration buffer may be optionally added to the supernatant to facilitate cation exchange chromatography, e.g. flow. For example, such dilution facilitates column loading and washing when using a Source S resin from Pharmacia whereas such dilution is unnecessary when using a Poros cationic resin. Filter sterilize the supernatant with 0.45 µm filters.

Run #1: A column (100 mm) was equilibrated with equilibration buffer, 2 minutes, 2.5 to 3 ml/min (same rate through out run). Cell paste (20-40 mL per run) was manually loaded. The column was washed for 3 minutes with equilibration buffer. Using gradient buffer, a 0 to 70% gradient was run over 8 minutes. For some cell lysates, a 5% NaCl (5 mS) 5 minutes step was performed. For example, where a Source S resin was used, no salt wash was was performed, but where a Poros resin was used, this salt wash was performed to elute contaminating proteins. Cell protein was collected at between 10 and 22 mS. Fractions (1 mL) were collected through out the gradient. The desired protein will elute at between 10 and 22 mS, depending upon the expression product used.

Run#2: The peak fractions from run #1 were pooled. Equilibration buffer was added to pooled fractions, at a 3:1 ratio. The column was equilibrated with equilibration buffer for 2 minutes, at 2.5 to 3 ml/min (same rate throughout run). The ran#1 pool was loaded onto the column; washed 2 minutes with equilibration buffer. Using gradient buffer, a 0 to 75% gradient was run over 15 minutes. Fractions (1 mL) were collected and peak fractions were pooled. Aliquots of the pooled fractions were stored in plastic vials at -20° C.

A portion of the purified protein was used to measure the A_{260/278}. The ratio may be used as a measure of the presence of DNA and the A₂₈₀ to quantitate the protein by using the calculated molar extinction coefficient and molecular weight

A cleaning procedure must be done on the column between each run. Run 1 M NaCI through column at 3 ml/min for 5 minutes. Run 20 mM NaOH through the column at 1ml/min for 10 minutes. Run 70% ETOH through the column at 1 ml/min for 30 minutes. Run 1 M NaCl through it at 1 ml/min for 15 minutes. Re-equilibrate the column to the proper pH with a low salt buffer.

### Buffers

A combination of sonication buffers, equilibration buffers and gradient buffers is used for each cell lysate. Sonication buffers are always chosen to be 0.4 pH below the equilibration buffer. Gradient buffers are the same as equilibration buffers except for addition of 1 M NaCl.

Gradient buffer **A**: 55 mM Na mono-phosphate, 2 mM EDTA, 1 M NaCl, in milliQ water, pH to 5.8; filter. Gradient buffer **B**: 20 mM NaAcetate, 1 M NaCl, in milliQ water, pH to 5.4, filter. Gradient buffer **C1**: 20 mM NaAcetate, 1 M NaCl, in milliQ water, pH to 4.8, filter. Gradient buffer **C2**: 20 mM NaAcetate, 2 mM EDTA, 1 M NaCl, in milliQ water, pH to 5.4, filter. Gradient buffer **D**: 20 mM NaAcetate, 2 mM EDTA, 1M NaCl, in milliQ water, pH to 4.8, filter.

### Results

Expression and purification of BoNT/A LC according to this method yielded protein with a specific activity (SNAP-25 assay) that was about 10-fold higher than when BoNT/A LC was purified from inclusion bodies (Ahmed and Smith (2000) J. Prot Chem. 19, 475-487).

### REFERENCES

Adler, M., Dinterman, R. E., and Wannemacher, R. W. (1997). Toxicon 35, 1089-1110.
Ahmed, S. A. and Claiborne, A. (1992). J. Biol. Chem. 267,3822-3840.
Ahmed, S. A. and Smith, L. A. (2000). J. Protein Chem. 19, 475-487.
Ahmed, S. A., Byrne, M.P., Jensen, M., Hines, H.B., Brueggemann, E., and Smith, L. A. (2001). J. Protein Chem. 20, 221-231.
Ahmed, S. A., Fairwell, T., Dunn, S., Kirschner, K., and Miles, E. W. (1986). Biochemistry 25, 3118-3124.
Alderton, J. M., Ahmed, S. A., Smith, L. A., and Steinhardt, R. A. (2000). Cell. Calcium 28,161-169.
Andersson, S. G., and Kurland, C. G. (1990). Microbial. Rev. 54, 198-210.
Auld, D. S. (1995). Meth. EnUmol. 248, 228-242.
Bi, G. Q., Alderton, J. M., and Steinhardt, R. A. (1995). J. Cell Biol. 131, 1747-1758.
Bittner, M. A., DasGupta, B. R., and Holz, R. W. (1989). J. Biol. Chem. 264, 10354-10360.
Black, J. D., and Dolly, J. O. (1986). J. Cell Biol.103, 535-544.
Blasi, J., Chapman, B. R., Link, E., Binz, T., Yamasaki, S., De Camilli, P., Sudhof, T. C. Niemann, H., and Jahn, R. (1993). Nature 365, 160-163.
Cai, S., Sarkar, H. K., and Singh, B. R. (1999). Biochemistry 38, 6903-6910.
Cardoso F, Jankivic J (1995). Clinical use of botulinum neurotoxins. In Current Topics in Microbiology and Immunology (Capron A et al., eds.), Springer-Verlag, Germany, pp. 123-141.
Chen, F., Kuziemko, G. M., Amersdorfer, P., Wong, C., Marks, J. D., and Stevens, R. C. (1997). Infect. Immun. 65,1626-1630.
Claiborne, A., Yeh, J. L, Mallett, T. C., Luba, J., Crane, E. J., 3rd, Charrier, V., and Parsonage, D. (1999). Biochemistry 38,15407-15416.
Creighton, T. E. (1984). Proteins, Structures and Molecular Properties, Freeman, New York.
Dalbey, R. E. and Kahn, A. (2000). Annu. Rev. Cell Dev. Biol. 16, 51-87.
DasGupta, B. R., and Dekleva, M. L. (1990). Biochimie 72, 661-664.
DasGupta, B. R., and Foley, J., Jr. (1989). Biochimie 71,1 193-1200.
Dekleva, M. L. and DasGupta, B. R. (1990). J. Bacteriol. 172, 2498-2503.
de Paiva, A., Poulain, B., Lawrence, G. W., Shone, C. C., Tauc, L., and Dolly, J. O. (1993). J. Biol. Chem. 268, 20838-20844.
Dertzbaugh, M. T., and West, M. W. (1996). Vaccine 14, 1538-1544.
Ettinger, R. A., Liu, A. W., Nepom, G. T., and Kwok, W. W. (2000). J. Immunol 165, 3232-3238.
Foran, P., Shone, C. C., and Dolly, J. O. (1994). Biochemistry 33,15365-15374.
Foran, P., Lawrence, G. W., Shone, C. C., Foster, K. A., and Dolly, J. O. (1996). Biochemistry 35,2630-2636.
Fu, F. N., Lomneth, R. B., Cai, S., and Singh, B. R. (1998). Biochemistry 37, 5267-5278.
Kadkhodayan, S., Knapp, M. S., Schmidt, J. J., Fabes, S. E., Rupp, B., and Balhorn, R. (2000). Protein Expr. Purif. 19, 125-130.
Kiyatkin, N., Maksymowych, A. B., and Simpson, L. L. (1997). Infect. Immun. 65,4586-4591.
Klatt, P., Schmidt, K., Lehner, D., Glatter, O., Bachinger, H. P., and Mayer, B. (1995). EMBO J. 14,3687 3695.
Knapp, M., Segelke, B., Balhorn, R., and Rupp. B. (2000). The crystal structure of botulinum toxin A zinc protease domain. Presented at the 37th Annual Meeting of the Interagency Botulinum Research Coordinating Committee, Alisomar, California.
Kreiglstein, K. G., DasGupta, B. R., and Henschen, A. H. (1994). J. Protein Chem. 13, 49-57.
Kurazono, H.. Mochida, S., Binz, T., Bisel, U., Quanz, M., Grebenstein, O., Wetnars, K., Poulain, B., Tauc, L., and Niemann, H. (1992). J. Biol. Chem. 267, 14721-14729.
Lacy, D. B., and Stevens, R. C. (1999). J. Mol. Biol. 291, 1091-1104.
Lacy, D. B., Tepp, W., Cohen, A. C., DasGupta, B. R, and Stevens, R. C. (1998). Nature Struct. Biol. 5, 898-902.
Laemmli, U. K. (1970). Nature 227, 680-685.
Lebeda, F. J., and Olson, M. A. (1994). Proteins 20, 293-300.
Li, L., and Singh, B. R (1999). Protein Expr. Purif. 17, 339-344.
Li, L. and Singh, B. R. (2000). Biochemistry 39, 10581-10586.
Li, Y., Foran. P., Fairweather, N. F., de Paiva, A., Weller, U., Dougan, G., and Dolly, J. O. (1994). Biochemistry 33, 7014-7020.
Maisey, E. A., Wadsworth, J. D., Poulain, B., Shone, C. C., Melling. J.. Gibbs, P., Tauc, L., and Dolly, J. O. (1988). Eur. J. Biochem. 177,683-691.
Makoff, A.J., Oxer, M. D., Romanos, M. A., Fairweather, N. F., and Ballantine, S. (1989). Nucleic Acids Res. 17,10191-10202.
Montal, M. S., Blewitt, R., Tomich, J. M., and Mortal, M. (1992). FEBS Lett. 313,12-18.
Montecucco, C., and Schiavo, G. (1994). Mol. Microbiol 13, 1-8.
Montecucco, C., and Schiavo, G. (1995). Q. Rev. Biophys. 28, 423-472.
Nowakowski, J. L., Courtney, B. C., Bing, Q. A., and Adler, M. (1998). J. Protein Chem. 17, 453-462*.*
Pace, C. N., Vajdos, F., Fee, L., Grimsley, G., and Gray, T. (1995). Protein Sci. 4, 2411-2423.
Rossetto, O., Schiavo, G., Montecucco, C., Poulain, B., Deloye, F.. Lozzi, L., and Shone, C. C. (1994). Nature 372, 415-416.
Schiavo, G., Rossetto, O., Catsicas, S., Polverino de Laureto, P., DasGupta, B. R, Benfenati, F., and Montecucco, C. (1993). J. Biol. Chem 268, 23784-23787.
Schiavo, G., Malizio, C., Trimble, W. S., Polverino de Laureto, P. Milan, G., Sugiyama, H., Johnson, E. A., and Montecucco, C. (1994). J. Biol. Chem. 269, 20213-20216.
Schiavo, G.. Rossetto, Tonello, F., and Montecucco, C. (1995). Intracellular targets and metalloprotease activity of tetanus and botulinum neurotoxins. In Clostridial Neurotoxins: The Molecular Pathogenesis of Tetanus and Botulism (Montecucco, C., ed.), Springer, New York, pp. 257-273.
Schmidt, J. J., and Bostian. K. A. (1995). J. Protein Chem. 14, 703-708.
Schmidt, J. J., and Bostian. K. A. (1997). J. Protein Chem. 16,19-26.
Schmidt, J. J., Stafford RG, Millard CB (2001). Analytical Biochemistry 296, 130-137.
Shone, C. C., and Roberts, A. K. (1994). Eur. J. Biochem. 225, 263-270.
Shone, C. C., and Tranter, H. S. (1995). Curr. Top. Microbiol. Immunol. 195, 143-160.
Shone, C. C., Quinn, C. P., Wait, R, Hallis, B., Fooks, S. G., and Hambleton, P. (1993). Eur. J. Biochem. 217,965-971.
Schagger, H. and von Jagow, G. (1987). Anal. Biochem. 166, 368-379.
Schmidt, J. J., Stafford, R G., and Bostian, K. A. (1998). FEBS Lett. 435, 61-64.
Sheridan, R. E., Deshpande, S. S., Nicholson, J. D., and Adler, M. (1997). Toxicon 35, 1439-1451.
Simpson, L. L., Coffield, J. A., and Bakry, N. (1993). J. Pharmacol. Exp. Ther. 267, 720-727.
Smith, L. A. (1998). Toxicon 36, 1539-1548.
Steinhardt, R. A., Bi, G., and Alderton, J. M. (1994). Science 263, 390-393.
Strasser, A., O'Connor, L., and Dixit, V. M. (2000). Annu. Rev. Biochem 69, 217-245.
Syuto, B., and Kubo, S. (1981). J. Biol. Chem. 256, 3712-3717.
Thompson, D. E., Brehm, J. K., Oultram, J. D., Swinfield, T. J., Shone, C. C.. Atkinson, T., Melling, J., and Minton, N. P. (1990). Eur. J. Biochem. 189, 73-81.
Washbourne, P., PeBizzan, R. Baldini, G., Wilson, M. C., and Montecucco, C. (1997). FEBS Lett. 418,1-5.
Winkler, H. H., and Wood, D. O. (1988). Biochimie 70, 977-986.
Zhou, L., de Paiva, A., Liu, D., Aoki, R., and Dolly, J. O. (1995). Biochemistry 34,15175-15181.

## Claims

1. A nucleic acid molecule comprising the nucleotide sequence 9-1355 of SEQ ID NO: 4 encoding a botulinum neurotoxin serotype A light chain or comprising a nucleotide sequence encoding a truncation thereof which lacks the sequence coding for the last 21 or 22 amino acids at the carboxy terminus of the botulinum neurotoxin serotype A light chain.

2. An expression vector carrying the nucleic acid molecule of claim 1.

3. An *Escherichia coli* host cell containing the expression vector of claim 2.

4. A yeast host cell containing the expression vector of claim 2.

5. A host cell according to claim 3 or 4, which produces a protein comprising a botulinum neurotoxin light chain serotype A or the said truncation thereof.

6. A method for producing a polypeptide comprising a C. botulinum neurotoxin serotype A light chain or a truncation thereof which lacks the last 21 or 22 amino acids at the carboxy terminus thereof, said method comprising culturing a host cell of claim 5 under conditions wherein the nucleic acid molecule carried by the expression vector is expressed.

## Patentansprüche

1. Nukleinsäuremolekül, umfassend die Nukleotidsequenz 9-1355 der SEQ ID NO: 4, die für eine leichte Kette des Botulinum Neurotoxins Serotyp A kodiert, oder umfassend eine Nukleotidsequenz, die für eine Verkürzung davon kodiert, welcher die Sequenz fehlt, welche für die letzten 21 oder 22 Aminosäuren an dem Carboxyterminus der leichten Kette des Botulinum Neurotoxins Serotyp A kodiert.

2. Expressionsvektor, der das Nukleinsäuremolekül nach Anspruch 1 trägt.

3. Wirtszelle von *Escherichia coli,* die den Expressionsvektor nach Anspruch 2 enthält.

4. Hefewirtszelle, die den Expressionsvektor nach Anspruch 2 enthält.

5. Wirtszelle nach Anspruch 3 oder 4, welche in Protein herstellt, umfassend eine leichte Kette des Botulinum Neurotoxins Serotyp A oder die besagte Verkürzung davon.

6. Verfahren zur Herstellung eines Polypeptids umfassend eine leichte Kette des C. *botulinum* Neurotoxins Serotyp A oder eine Verkürzung davon, welcher die letzten 21 oder 22 Aminosäuren am Carboxyterminus davon fehlen, wobei das besagte Verfahren die Kultivierung einer Wirtszelle nach Anspruch 5 und Bedingungen umfasst, worin das Nukleinsäuremolekül, das von dem Expressionsvektor getragen wird, exprimiert wird.

## Revendications

1. Molécule d'acide nucléique comprenant la séquence de nucléotides 9 à 13bb de SEQ ID N° 4 codant une chaîne légère de sérotype A de neurotoxine botulinique ou comprenant une séquence de nucléotides codant une troncature de celle-ci à laquelle manque la séquence codant les derniers 21 ou 22 acides aminés à l'extrémité C-terminale de la chaîne légère de sérotype A de neurotoxine botulinique.

2. Vecteur d'expression portant la molécule d'acide nucléique selon la revendication 1.

3. Cellule hôte d'Escherichia coli contenant le vecteur d'expression de la revendication 2.

4. Cellule hôte de levure contenant le vecteur d'expression de la revendication 2.

5. Cellule hôte selon la revendication 3 ou 4 qui produit une protéine comprenant une chaîne légère de sérotype A de neurotoxine botulinique ou ladite troncature de celle-ci.

6. Procédé de production d'un polypeptide comprenant une chaîne légère de sérotype A de neurotoxine C. botulinique ou une troncature de celle-ci à laquelle manquent les derniers 21 ou 22 acides aminés à l'extrémité C-terminale de celle-ci, ledit procédé comprenant la mise en culture d'une cellule hôte de la revendication 5 dans des conditions dans lesquelles la molécule d'acide nucléique portée par le vecteur d'expression est exprimée.
